# EUROPEAN PATENT APPLICATION

(11) **EP 2 103 215 A1**
(43) Date of publication of application: **23.09.2009**
(21) Application number: 07860373.5
(22) Date of filing: 27.12.2007
(51) Int. Cl.: A01N 43/56, A01N 47/02, A01P 13/02, C07C 315/00, C07C 315/04, C07C 317/14, C07C 317/22, C07C 317/44, C07D 231/20

(54) **HERBICIDE CONTAINING BENZOYLPYRAZOLE COMPOUND**

(30) Priority: 27.12.2006 JP 2006351617; 12.06.2007 JP 2007155359; 27.07.2007 JP 2007195420
(71) Applicant: ISHIHARA SANGYO KAISHA, LTD., Osaka-shi, Osaka 550-0002 (JP)
(72) Inventor: SHIMOHARADA, Hiroshi, Kusatsu-shi Shiga 525-0025 (JP); TSUKAMOTO, Masamitsu, Kusatsu-shi Shiga 525-0025 (JP); IKEGUCHI, Masahiko, Osaka-shi Osaka 550-0002 (JP); KIKUGAWA, Hiroshi, Kusatsu-shi Shiga 525-0025 (JP); NAGAYAMA, Souichiro, Kusatsu-shi Shiga 525-0025 (JP); SANO, Makiko, Kusatsu-shi Shiga 525-0025 (JP); KITAHARA, Yoshinori, Chiba-shi Chiba 260-0833 (JP); OKITA, Tatsuya, Kusatsu-shi Shiga 525-0025 (JP); HATA, Hiroshi, Kusatsu-shi Shiga 525-0025 (JP)
(74) Representative: Hartz, Nikolai
(86) International application number: PCT/JP2007/075151
(87) International publication number: WO 2008/078811

(57) **Abstract**

To provide a novel herbicide which has a wide range of applications to agricultural or non-agricultural fields and various methods for application such as soil treatment and foliage treatment and which exhibits excellent herbicidal effects.

A herbicide containing a benzoylpyrazole compound represented by the formula (I) or its salt, as an active ingredient: 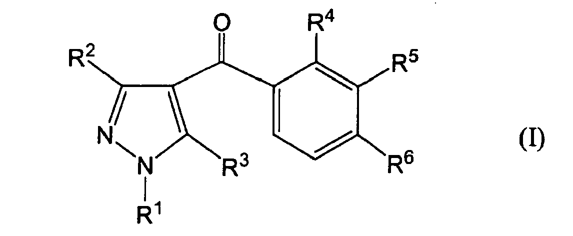 wherein R¹ is alkyl or cycloalkyl, R² is a hydrogen atom or alkyl, R³ is hydroxy, R⁴ is alkyl, haloalkyl or the like, R⁵ is halogen, cyano, cyanoalkyl or the like, and R⁶ is haloalkyl, halogen or the like.

## Description

### TECHNICAL FIELD

The present invention relates to a novel herbicide containing a benzoylpyrazole compound as an active ingredient.

### BACKGROUND ART

Patent Document 1 discloses various benzoylpyrazole compounds. However, a herbicide containing a benzoylpyrazole compound represented by the following formula (I) as an active ingredient, is not disclosed therein.

Patent Document 1: EP0352543

### DISCLOSURE OF THE INVENTION

### OBJECT TO BE ACCOMPLISHED BY THE INVENTION

Heretofore, herbicides which have excellent herbicidal activities against weeds and which are safe to crop plants, have been desired for labor saving in the operation of controlling weeds and for improvement of productivity of agricultural and horticultural plants. However, search for herbicides suitable for such an object depends on trial and error.

### MEANS TO ACCOMPLISH THE OBJECT

The present inventors have conducted extensive studies on benzoylpyrazole compounds in order to find superior herbicides thereby to accomplish the above object and as a result, have accomplished the present invention.

That is, the present invention relates to a herbicide containing a benzoylpyrazole compound represented by the formula (I) or its salt, as an active ingredient: 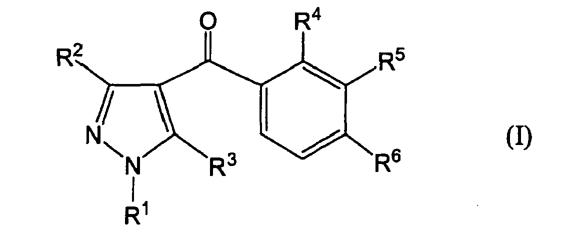 wherein R¹ is alkyl or cycloalkyl, R² is a hydrogen atom or alkyl, R³ is hydroxy, R⁴ is alkyl; haloalkyl; alkoxy; halogen; nitro; cyano; alkylthio; alkylsulfinyl; or alkylsulfonyl, R⁵ is halogen; cyano; cyanoalkyl; haloalkoxyalkyl; amino(thiocarbonyl)alkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷; thiocyanatoalkyl; haloalkoxy; alkoxyalkoxy; alkoxy substituted by at least 2 haloalkoxy; alkoxyhaloalkoxy; haloalkoxyhaloalkoxy; alkoxyalkyl substituted by at least 2 haloalkoxy; alkylthio substituted by at least 2 alkoxy; alkylthio substituted by at least 2 haloalkoxy; alkoxyhaloalkylthio; haloalkoxyhaloalkylthio; alkylthioalkylthio; haloalkylthioalkylthio; alkylthiohaloalkylthio; haloalkylthiohaloalkylthio; alkylthioalkoxy; alkylsulfonyl; alkylsulfonylalkyl; alkoxycarbonylalkyl; alkoxycarbonylalkoxy; heterocyclylalkyl; alkoxy substituted by at least 2 heterocyclyl; alkyl substituted by at least 2 heterocyclylalkoxy; -OC(O)SR⁷; or aminoalkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷, R⁶ is haloalkyl; halogen; nitro; cyano; alkylthio; alkylsulfinyl; or alkylsulfonyl, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, provided that when R⁴ is halogen, R⁵ is not cyanoalkyl; alkyl substituted by one haloalkoxy; or alkoxy substituted by one alkoxy. Further, the present invention relates to a herbicidal composition comprising the above benzoylpyrazole compound or its salt, and an agricultural adjuvant. Further, the present invention provides a method for controlling undesired plants or inhibiting their growth, which comprises applying a herbicidally effective amount of the above benzoylpyrazole compound or its salt to the undesired plants or to a place where they grow.

### EFFECTS OF THE INVENTION

The herbicide containing the compound represented by the above formula (I) exhibits excellent herbicidal effects.

### BEST MODE FOR CARRYING OUT THE INVENTION

In the above formula (I), the alkyl or alkyl moiety may be linear or branched, and specific examples thereof include C₁₋₉ alkyl such as methyl, ethyl, n-propyl, iso-propyl, n-butyl, iso-butyl, sec-butyl, tert-butyl, n-pentyl, iso-pentyl, neopentyl, tert-pentyl, n-hexyl, iso-hexyl, n-heptyl, n-octyl and n-nonyl.

In the above formula (I), the cycloalkyl may, for example, be C₃₋₆ cycloalkyl such as cyclopropyl, cyclobutyl, cyclopentyl or cyclohexyl.

In the above formula (I), the alkenyl or alkenyl moiety may be linear or branched, and specific examples thereof include C₂₋₉ alkenyl such as vinyl, 1-propenyl, 2-propenyl, iso-propenyl, allyl, 1 - butenyl, 2-butenyl, 3-butenyl, 1,3-butadienyl, 2-pentenyl, 4-pentenyl, 2-hexenyl, 4-hexenyl, 2-heptenyl, 4-heptenyl, 2-octenyl, 6-octenyl and 2-nonenyl.

In the above formula, the alkynyl may be linear or branched, and specific examples thereof include C₂₋₉ alkynyl such as ethynyl, propargyl, 1-propynyl, 1-pentynyl, 3-pentynyl, 1-heptynyl and 1-nonynyl.

In the above formula (I), the halogen or halogen as a substituent may be an atom of fluorine, chlorine, bromine or iodine. The number of halogen as substituents may be 1 or more, and in the case of more than 1, they may be the same or different. Further, the positions for substitution of halogen may be any positions.

In the above formula (I), the aryl or aryl moiety as a substituent in R⁷ may, for example, be phenyl or naphthyl. The number of aryl or aryl moieties as substituents may be 1 or more, and in the case of more than 1, they may be the same or different. The positions for their substitution may be any positions.

In the above formula (I), the number of R⁸ for substitution on the arylalkyl as R⁷ may be one or more, and in the case of more than one, they may be the same or different. Further, the positions for the respective substituents may be any positions.

In the above formula (I), the alkoxy substituted by at least 2 haloalkoxy, as R⁵, means that the same or different at least 2 haloalkoxy are bonded to an alkoxy group. The positions for substitution of haloalkoxy may be any positions.

In the above formula (I), the alkoxyhaloalkoxy as R⁵ means that the same or different alkoxy is bonded to a haloalkoxy group. The number of alkoxy for substitution may be one or more, and the positions for their substitution may be any positions. The same applies also to the alkylthioalkylthio, etc.

In the above formula (I), the heterocyclyl moiety in R⁵ may, for example, be a 5- or 6-membered one having from 1 to 4 hetero atoms of one more of types optionally selected from O, S and N, which may be a saturated or unsaturated one. Specifically, oxolanyl, 1,3-dioxolanyl, tetrahydrofuranyl or tetrahydro-2H-pyranyl may, for example, be mentioned. The number of heterocyclyl moieties for substitution may be one or more, and in the case of more than one, they may be the same or different. The positions for substitution of heterocyclyl moieties may be any positions.

The salt of the benzoylpyrazole compound represented by the above formula (I) may be any salt so long as it is agriculturally acceptable, and it may, for example, be an alkali metal salt such as a sodium salt or a potassium salt; an alkaline earth metal salt such as a magnesium salt or a calcium salt; an amine salt such as a dimethylamine salt or a triethylamine salt; an inorganic acid salt such as a hydrochloride, a perchlorate, a sulfate or a nitrate; or an organic acid salt such as an acetate or a methanesulfonate.

A compound represented by the formula (I-a) including the benzoylpyrazole compound represented by the above formula (I), or its salt, may be produced by the following reaction (A) and a common method for producing a salt. 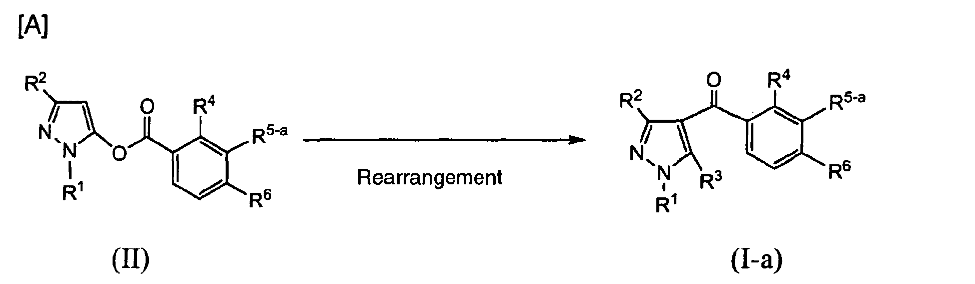 In the above formula, R¹, R², R³, R⁴ and R⁶ are as defined above, and R^{5-a} is a hydrogen atom; alkyl; alkenyl; alkynyl; halogen; cyano; cyanoalkyl; cyanoalkenyl; haloalkyl; alkoxyalkyl; haloalkoxyalkyl; amino(thiocarbonyl)alkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷; thiocyanatoalkyl; alkoxy; alkenyloxy; alkynyloxy; haloalkoxy; alkoxyalkoxy; haloalkoxyalkoxy; alkoxyhaloalkoxy; haloalkoxyhaloalkoxy; alkoxyalkoxyalkyl; alkylthio; alkoxyalkylthio; haloalkoxyalkylthio; alkoxyhaloalkylthio; haloalkoxyhaloalkylthio; alkylthioalkylthio; haloalkylthioalkylthio; alkylthiohaloalkylthio; haloalkylthiohaloalkylthio; alkylthioalkoxy; alkylsulfonyl; alkylsulfonylalkyl; alkoxycarbonylalkyl; alkoxycarbonylalkoxy; heterocyclylalkyl; heterocyclyloxy; heterocyclylalkoxy; heterocyclylalkoxyalkyl; heterocyclyloxyalkyl; cycloalkyloxy; -OC(O)SR⁷; -OC(O)OR⁷; -C(O)OR⁷; -C(O)SR⁷; -C(S)OR⁷; -C(S)SR⁷; aminoalkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷; or 4,5-dihydroisoxazol-3-yl which may be substituted by alkyl, halogen or alkoxy.

The above reaction may be carried out in the presence of a solvent, as the case requires. The solvent may be any solvent so long as it is inert to the reaction, and it may, for example, be a halogenated hydrocarbon such as methylene chloride, chloroform, dichloroethane or trichloroethane; an aromatic hydrocarbon such as benzene, toluene, xylene, nitrobenzene or chlorobenzene; an ester such as methyl acetate, ethyl acetate or propyl acetate; an aprotic polar solvent such as acetonitrile, N,N-dimethylformamide (DMF), dimethylsulfoxide (DMSO), dimethylacetamide (DMA), hexamethylphosphoric acid triamide (HMPA) or sulfolane; or an ether such as diethyl ether, 1,4-dioxane, tetrahydrofuran (THF) or 1,2-dimethoxyethane. As the solvent, one or more of these solvents may suitably be selected for use.

The above reaction may be carried out in the presence of a base, as the case requires. The base may be an organic base or an inorganic base. The organic base may, for example, be a tertiary amine such as triethylamine or diisopropylethylamine; pyridine; 4-(dimethylamino)pyridine; or 2,6-lutidine. The inorganic base may, for example, be an alkali metal carbonate such as sodium carbonate, potassium carbonate or cesium carbonate; an alkali metal hydrogencarbonate such as sodium hydrogencarbonate or potassium hydrogencarbonate; an alkaline earth metal carbonate such as calcium carbonate or barium carbonate; an alkali metal hydroxide such as lithium hydroxide, sodium hydroxide or potassium hydroxide; or an alkaline earth metal hydroxide such as calcium hydroxide or barium hydroxide. As the base, one or more of these bases may suitably be selected for use.

Further, in the above reaction, a catalyst may be added as the case requires. As such a catalyst, acetone cyanohydrin may be used in an amount of from 0.01 to 10 equivalents to the compound of the formula (II).

The reaction temperature for the above reaction is usually from 0°C to 150°C, and the reaction time is usually from 1 minute to 48 hours.

The compound represented by the above formula (II) may be produced in accordance with the following reaction (B). 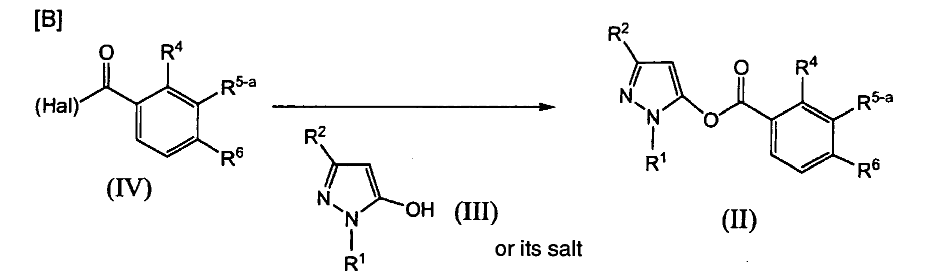 In the above formulae, R¹, R², R⁴, R^{5-a} and R⁶ are as defined above, and Hal is a halogen atom.

The salt of the compound represented by the formula (III) may, for example, be a hydrochloride, a sulfate or a nitrate.

The above reaction may be carried out in the presence of a solvent as the case requires. The solvent may be any solvent so long as it is inert to the reaction, and ones exemplified for the above reaction (A) may be mentioned. As the solvent, one or more of such solvents may be suitably selected for use.

The above reaction may be carried out in the presence of a base as the case requires. The base may be an inorganic base or an organic base, and ones exemplified for the above reaction (A) may be mentioned. As the base, one or more of them may suitably be selected for use.

The reaction temperature for the above reaction is usually from 0°C to 150°C, and the reaction time is usually from 1 minute to 48 hours.

The compound represented by the above formula (IV) may be produced in accordance with the following reaction (C). 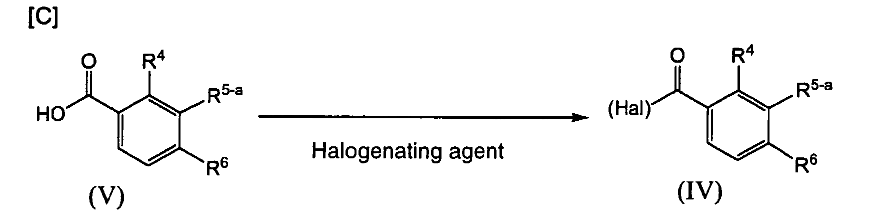 In the above formulae, R⁴, R^{5-a}, R⁶ and Hal are as defined above.

In the above reaction, the halogenating agent may, for example, be thionyl chloride or oxalyl chloride. The halogenating agent may be reacted in an amount of from 1 to 100 equivalents to the compound represented by the formula (V).

The above reaction may be carried out in the presence of a solvent as the case requires. The solvent may be any solvent so long as it is inert to the reaction, and it may, for example, be a halogenated hydrocarbon such as methylene chloride, chloroform, dichloroethane or trichloroethane; an aromatic hydrocarbon such as benzene, toluene, xylene, nitrobenzene or chlorobenzene; an ester such as methyl acetate, ethyl acetate or propyl acetate; or an ether such as diethyl ether, 1,4-dioxane, tetrahydrofuran (THF) or 1,2-dimethoxyethane. As the solvent, one or more of them may be suitably selected for use.

For the above reaction, a catalyst may be used as the case requires. The catalyst may, for example, be DMF.

The reaction temperature for the above reaction is usually from 0°C to 150°C, and the reaction time is usually from 1 minute to 48 hours.

As another method, the compound represented by the formula (II) may be produced in accordance with the following reaction (D). 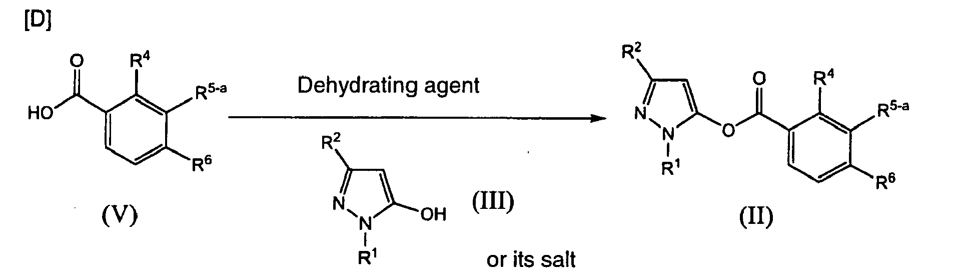 In the above formulae, R¹, R², R⁴, R^{5-a} and R⁶ are as defined above.

The salt of the compound represented by the formula (III) may, for example, be a hydrochloride, a sulfate or a nitrate.

The dehydrating agent which may be used in the above reaction, may, for example, be DCC (dicyclohexylcarbodiimide) or 1-ethyl-3-(3-dimethylaminopropyl)-carbodiimide hydrochloride.

The above reaction may be carried out in the presence of a solvent as the case requires. The solvent may be any solvent so long as it is inert to the reaction, and ones exemplified for the above reaction (A) may be mentioned. As the solvent, one or more of them may suitably be selected for use.

The above reaction may be carried out in the presence of a base as the case requires. The base may, for example, be a tertiary amine such as triethylamine or diisopropylethylamine; pyridine; 4-(dimethylamino)pyridine; or 2,6-lutidine. As the base, one or more of them may suitably be selected for use.

The reaction temperature for the above reaction is usually from 0°C to 150°C, and the reaction time is usually from 1 minute to 48 hours.

The compound represented by the above formula (V) may be produced in accordance with the following reaction (E). 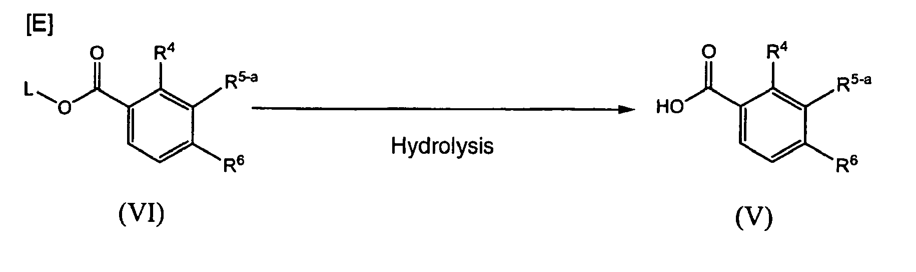 In the above formulae, R⁴, R^{5-a} and R⁶ are as defined above, and L is a protecting group such as alkyl.

The above reaction may be carried out in the presence of water.

The above reaction may be carried out in the presence of a solvent as a case requires. The solvent may, for example, be an aromatic hydrocarbon such as benzene, toluene, xylene, nitrobenzene or chlorobenzene; an aprotic polar solvent such as acetonitrile, DMF, DMSO, DMA, HMPA or sulfolane; an ether such as diethyl ether, 1,4-dioxane, THF or 1,2-dimethoxyethane; an alcohol such as methanol, ethanol or propanol; or water. As the solvent, one or more of them may be suitably selected for use.

The above reaction may be carried out in the presence of a base or an acid as the case requires. The base may be an inorganic base or an organic base. The inorganic base may, for example, be an alkali metal hydroxide such as lithium hydroxide, sodium hydroxide or potassium hydroxide; an alkaline earth metal hydroxide such as calcium hydroxide or barium hydroxide; an alkali metal carbonate such as sodium carbonate, potassium carbonate or cesium carbonate; an alkali metal hydrogencarbonate such as sodium hydrogencarbonate or potassium hydrogencarbonate; or an alkaline earth metal carbonate such as calcium carbonate or barium carbonate. The organic base may, for example, be a tertiary amine such as triethylamine or diisopropylethylamine. The acid may, for example, be hydrochloric acid, sulfuric acid or perchloric acid. As the base or the acid, one or more of the respective ones may suitably be selected for use.

The reaction temperature for the above reaction is usually from 0°C to 150°C, and the reaction time is usually from 1 minute to 48 hours.

Among compounds represented by the above formula (VI), a compound wherein R^{5-a} is R^{5-a-1} may be produced in accordance with the following reaction (F). 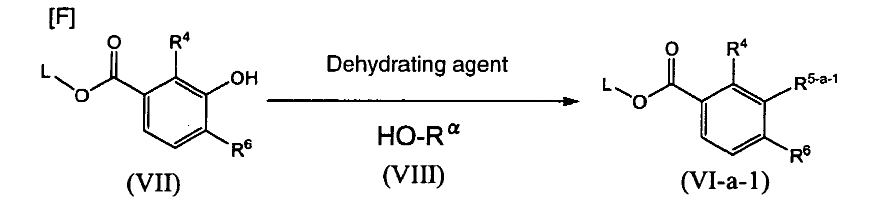 In the formulae, R⁴, R⁶ and L are as defined above, and R^{5-a-1} is alkoxy, haloalkoxy, alkoxyalkoxy, haloalkoxyalkoxy, alkoxyhaloalkoxy, haloalkoxyhaloalkoxy, heterocyclyloxy, heterocyclylalkoxy, cycloalkyloxy, -OC(O)SR⁷, -OC(O)OR⁷, alkylthioalkoxy, alkoxycarbonylalkoxy, alkenyloxy or alkynyloxy, and R^{α} is alkyl, haloalkyl, alkoxyalkyl, haloalkoxyalkyl, alkoxyhaloalkyl, haloalkoxyhaloalkyl, heterocyclyl, heterocyclylalkyl, cycloalkyl, -C(O)SR⁷, -C(O)OR⁷, alkylthioalkyl, alkoxycarbonylalkyl, alkenyl or alkynyl.

The dehydrating agent which may be used in the above reaction, may, for example, be DCC (dicyclohexylcarbodiimide) or 1-ethyl-3-(3-dimethylaminopropyl)-carbodiimide hydrochloride. Otherwise, diethylazodicarboxylate may be used together with triphenylphosphine, tributylphosphine or the like.

The above reaction may be carried out in the presence of a solvent as the case requires. The solvent may be any solvent so long as it is inert to the reaction, and ones exemplified for the above reaction (A) may be mentioned. As the solvent, one or more of them may suitably be selected for use.

The above reaction may be carried out in the presence of a base as the case requires. The base may, for example, be a tertiary amine such as triethylamine or diisopropylethylamine; pyridine; 4-(dimethylamino)pyridine; or 2,6-lutidine. As the base, one or more of them may be suitably selected for use.

The reaction temperature for the above reaction is usually from 0°C to 150°C, and the reaction time is usually from 1 minute to 48 hours.

As another method, the compound represented by the formula (VI-a-1) may be produced also in accordance with the following reaction (G).

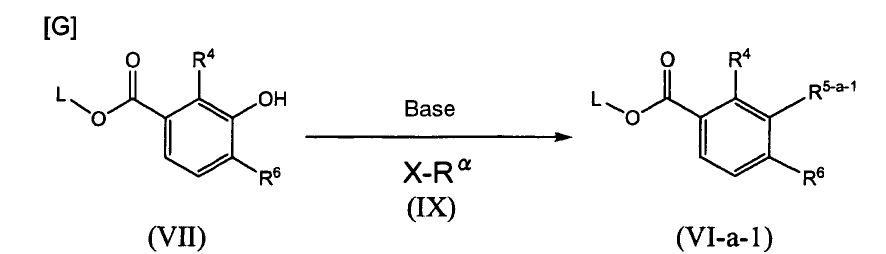

In the formulae, R⁴, R^{5-a-1}, R⁶, R^{α} and L are as defined above. X is a leaving group such as halogen or a methanesulfonyloxy group.

The base which may be used in the above reaction, may be an inorganic base or an organic base. The inorganic base may, for example, be an alkali metal carbonate such as sodium carbonate, potassium carbonate or cesium carbonate; an alkali metal hydrogencarbonate such as sodium hydrogencarbonate or potassium hydrogencarbonate; an alkaline earth metal carbonate such as calcium carbonate or barium carbonate; an alkali metal hydroxide such as lithium hydroxide, sodium hydroxide or potassium hydroxide; an alkaline earth metal hydroxide such as calcium hydroxide or barium hydroxide; or an alkali metal hydride such as sodium hydride or potassium hydride. The organic base may, for example, be triethylamine, diisopropylethylamine, pyridine, 4-(dimethylamino)pyridine or 2,6-lutidine. As the base, one or more of them may suitably be selected for use.

The above reaction may be carried out in the presence of a solvent as the case requires. The solvent may be any solvent so long as it is inert to the reaction, and ones exemplified for the above reaction (A) may be mentioned. As the solvent, one or more of them may suitably be selected for use.

The above reaction may be carried out in the presence of a catalyst as the case requires. The catalyst may, for example, be potassium iodide, tetra-n-butylammonium iodide, copper iodide or copper oxide.

The reaction temperature for the above reaction is usually from 0°C to 200°C, and the reaction time is usually from 1 minute to 48 hours.

The above reaction may be carried out in the presence of an inert gas as the case requires. The inert gas may, for example, be nitrogen gas or argon gas.

The compound represented by the above formula (VII) may be produced in accordance with the following reaction (H). 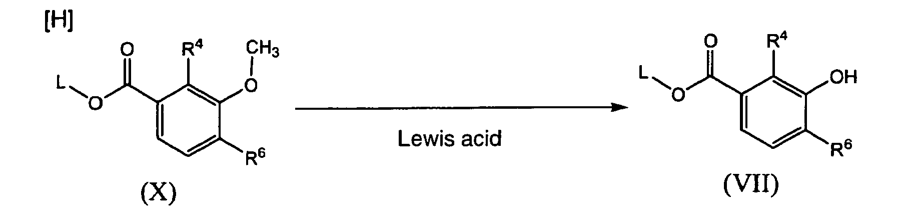 In the formulae, R⁴, R⁶ and L are as defined above.

The Lewis acid which may be used in the above reaction, may, for example, be boron tribromide, aluminum chloride or iron bromide.

The above reaction may be carried out in the presence of a solvent as the case requires. The solvent may be any solvent so long as it is inert to the reaction, and it may, for example, be a halogenated hydrocarbon such as methylene chloride, chloroform, dichloroethane or trichloroethane; an aromatic hydrocarbon such as benzene, toluene, xylene, nitrobenzene or chlorobenzene; carbon disulfide; or nitromethane. As the solvent, one or more of them may suitably be selected for use.

The reaction temperature for the above reaction is usually from 0°C to 150°C, and the reaction time is usually from 1 minute to 48 hours.

The compound represented by the above formula (X) may be produced in accordance with the following reaction (I). 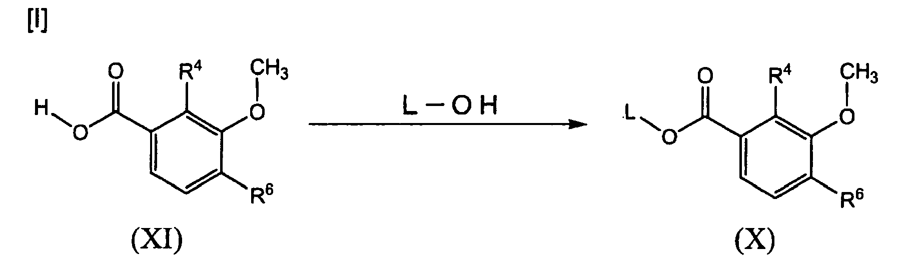 In the formulae, R⁴, R⁶ and L are as defined above.

In the above reaction, L-OH may be used in an excess amount so that it serves also as a solvent. Otherwise, the above reaction may be carried out in the presence of another solvent as the case requires. The solvent may be any solvent so long as it is inert to the reaction, and it may, for example, be an aromatic hydrocarbon such as benzene, toluene, xylene, nitrobenzene or chlorobenzene; an ester such as methyl acetate, ethyl acetate or propyl acetate; a halogenated hydrocarbon such as methylene chloride, chloroform, dichloroethane or trichloroethane; or an aprotic polar solvent such as acetonitrile, DMF, DMSO, DMA, HMPA or sulfolane. As the solvent, one or more of them may suitably be selected for use.

The above reaction may be carried out in the presence of an acid as the case requires. The acid which may be used for the above reaction, may, for example, be hydrochloric acid or sulfuric acid.

The reaction temperature for the above reaction is usually from 0°C to 150°C, and the reaction time is usually from 1 minute to 48 hours.

Among compound represented by the above formula (VI), a compound wherein R^{5-a} is R^{5-a-2} may be produced in accordance with the following reaction (J). 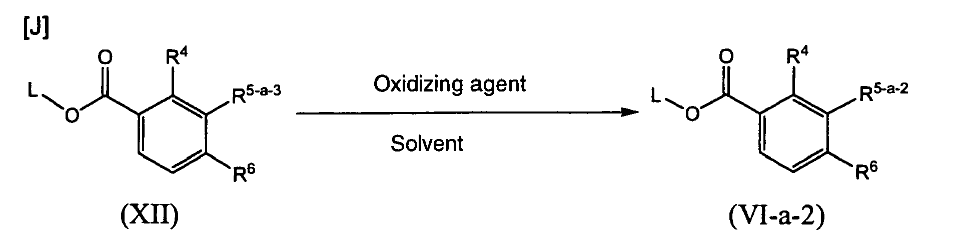 In the formulae, R⁴, R⁶ and L are as defined above, and R^{5-a-2} is alkyl substituted by alkylsulfonyl, and R^{5-a-3} is alkylthioalkyl.

The oxidizing agent which may be used for the above reaction, may, for example, be hydrogen peroxide, peracetic acid or m-chloroperbenzoic acid.

The solvent which may be used for the above reaction, may be any solvent so long as it is inert to the reaction, and it may, for example, be a halogenated hydrocarbon such as methylene chloride, chloroform, dichloroethane or trichloroethane; a ketone such as acetone or dimethyl ethyl ketone; an ether such as diethyl ether, 1,4-dioxane, THF or 1,2-dimethoxyethane; an aromatic hydrocarbon such as benzene, toluene, xylene, nitrobenzene or chlorobenzene; or an organic acid such as acetic acid or propionic acid. As the solvent, one or more of them may be suitably selected for use.

The above reaction may be carried out in the presence of a catalyst as the case requires. The catalyst which may be used for the above reaction, may, for example, be sodium tungstate.

The reaction temperature for the above reaction is usually from 0°C to 150°C, and the reaction time is usually from 1 minute to 48 hours.

The compound represented by the above formula (XII) may be produced in accordance with the following reaction (K). 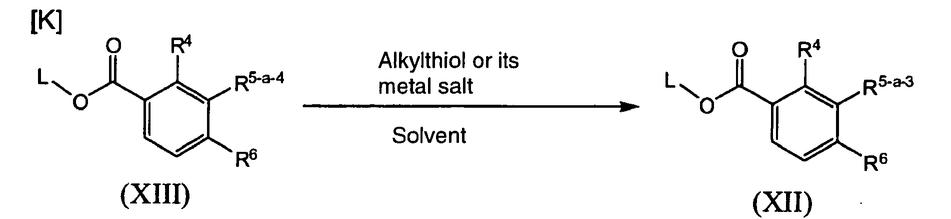 In the formulae, R⁴, R^{5-a-3}, R⁶ and L are as defined above, and R^{5-a-4} is bromoalkyl.

The metal salt of alkylthiol may, for example, be an alkali metal salt such as a sodium salt or a potassium salt.

The solvent which may be used for the above reaction, may be any solvent so long as it is inert to the reaction, and it may, for example, be a halogenated hydrocarbon such as methylene chloride, chloroform, dichloroethane or trichloroethane; a ketone such as acetone or methyl ethyl ketone; an ether such as diethyl ether, 1,4-dioxane, THF or 1,2-dimethoxyethane; water; or an aprotic polar solvent such as DMF, DMSO, DMA, HMPA or sulfolane. As the solvent, one or more of them may suitably be selected for use.

In the above reaction, the reaction may be carried out in the presence of a base as the case requires. The base may be an inorganic base or an organic base. The inorganic base may, for example, be an alkali metal carbonate such as sodium carbonate, potassium carbonate or cesium carbonate; an alkali metal hydrogencarbonate such as sodium hydrogencarbonate or potassium hydrogencarbonate; an alkaline earth metal carbonate such as calcium carbonate or magnesium carbonate; an alkali metal hydroxide such as lithium hydroxides, sodium hydroxide or potassium hydroxide; an alkaline earth metal hydroxide such as calcium hydroxide or barium hydroxide, or an alkali metal hydride such as sodium hydride or potassium hydride. The organic base may, for example, be triethylamine, diisopropylethylamine, pyridine, 4-(dimethylamino)pyridine, or 2,6-lutidine. As the base, one or more of them may suitably be selected for use.

The reaction temperature for the above reaction is usually from 0°C to 150°C, and the reaction time is usually from 1 minute to 48 hours.

Among compounds represented by the above formula (VI), a compound wherein R^{5-a} is R^{5-a-5} may be produced in accordance with the following reaction (L). 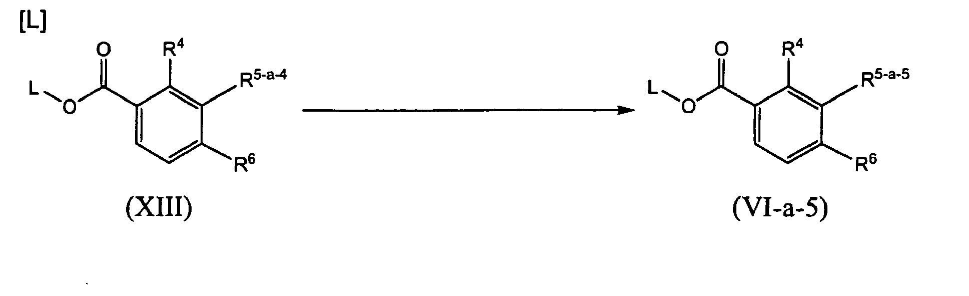 In the formulae, R⁴, R^{5-a-4} , R⁶ and L are as defined above, and R^{5-a-5} is alkoxyalkoxyalkyl or heterocyclyloxyalkyl.

That is, the compound represented by the formula (VI-a-5) may be produced by reacting an alcohol having the alkyl moiety substituted by alkoxy, an alcohol having the alkyl moiety substituted by heterocyclyl, or a metal salt thereof, with the compound represented by the formula (XIII).

The metal salt of an alcohol having the alkyl moiety substituted by alkoxy or an alcohol having the alkyl moiety substituted by heterocyclyl which may be used for the above reaction, may, for example, be an alkali metal salt such as a sodium salt or a potassium salt.

The above reaction may be carried out in the presence of a solvent as the case requires. The solvent may be any solvent so long as it is inert to the reaction, and it may, for example, be an alcohol such as methanol, ethanol or propanol; an ester such as methyl acetate, ethyl acetate or propyl acetate; an ether such as diethyl ether, 1,4-dioxane, THF or 1,2-dimethoxyethane; or an aprotic polar solvent such as acetonitrile, DMF, DMSO, DMA, HMPA or sulfolane. As the solvent, one or more of them may suitably be selected for use.

The above reaction may be carried out in the presence of a base as the case requires. The base may, for example, be an alkali metal hydride such as sodium hydride or potassium hydride; or sodium amide.

The reaction temperature for the above reaction is usually from 0°C to 150°C, and the reaction time is usually from 1 minute to 48 hours.

Among compounds represented by the above formula (VI), a compound wherein R^{5-a} is R^{5-a-6} may be produced in accordance with the following reaction (M). 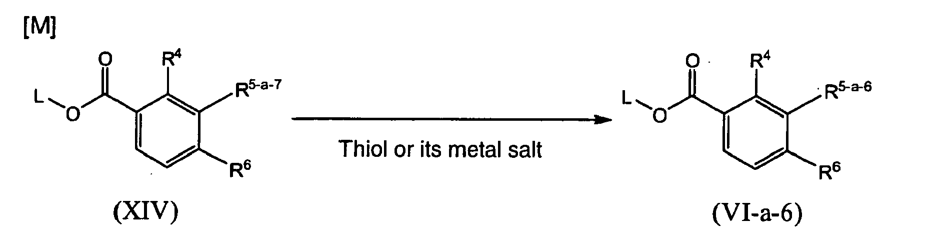 In the formulae, R⁴, R⁶ and L are as defined above, and R^{5-a-6} is alkylthio, alkoxyalkylthio, haloalkoxyalkylthio, alkoxyhaloalkylthio, haloalkoxyhaloalkylthio, alkylthioalkylthio, haloalkylthioalkylthio, alkylthiohaloalkylthio or haloalkylthiohaloalkylthio, and R^{5-a-7} is halogen.

The metal salt of thiol which may be used for the above reaction, may, for example, be an alkali metal salt such as a sodium salt or a potassium salt.

The above reaction may be carried out in the presence of a solvent as the case requires. The solvent may be any solvent so long as it is inert to the reaction, and it may, for example, be an alcohol such as methanol, ethanol or propanol; an ether such as diethyl ether, 1,4-dioxane, THF or 1,2-dimethoxyethane; an aprotic polar solvent such as acetonitrile, DMF, DMSO, DMA, HMPA or sulfolane; or water. As the solvent, one or more of them may suitably be selected for use.

The above reaction may be carried out in the presence of a base as the case requires. The base may, for example, be an alkali metal hydride such as sodium hydride or potassium hydride; an alkali metal hydroxide such as lithium hydroxide, sodium hydroxide or potassium hydroxide; or an alkaline earth metal hydroxide such as calcium hydroxide or barium hydroxide. As the base, one or more of them may suitably be selected for use.

The reaction temperature for the above reaction is usually from 0°C to 250°C, and the reaction time is usually from 1 minute to 48 hours.

Among compounds represented by the above formula (I-a), a compound represented by the formula (I-a-8) wherein R^{5-a} is R^{5-a-8}, may be produced in accordance with the following reaction (N). 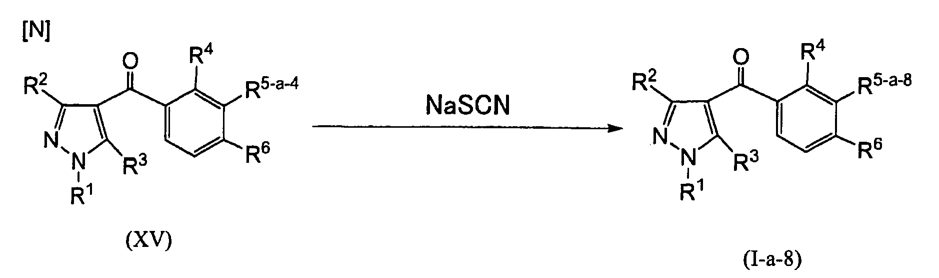 In the formulae, R¹, R², R³, R⁴, R^{5-a-4} and R⁶ are as defined above, and R^{5-a-8} is thiocyanatoalkyl.

The above reaction may be carried out in the presence of a solvent as the case requires. The solvent may be any solvent so long as it is inert to the reaction, and it may, for example, be a halogenated hydrocarbon such as methylene chloride, chloroform, dichloroethane or trichloroethane; an aromatic hydrocarbon such as benzene, toluene, xylene, nitrobenzene or chlorobenzene; an ester such as methyl acetate, ethyl acetate or propyl acetate; an aprotic polar solvent such as acetonitrile, DMF, DMSO, DMA, HMPA or sulfolane; an ether such as diethyl ether, 1,4-dioxane, THF or 1,2-dimethoxyethane; or an alcohol such as methanol, ethanol or propanol. As the solvent, one or more of them may suitably be selected for use.

The reaction temperature for the above reaction is usually from 0°C to 150°C, and the reaction time is usually from 1 minute to 48 hours.

Among compounds represented by the above formula (VI), a compound wherein R^{5-a} is R^{5-a-9}, may be produced in accordance with the following reaction (O). 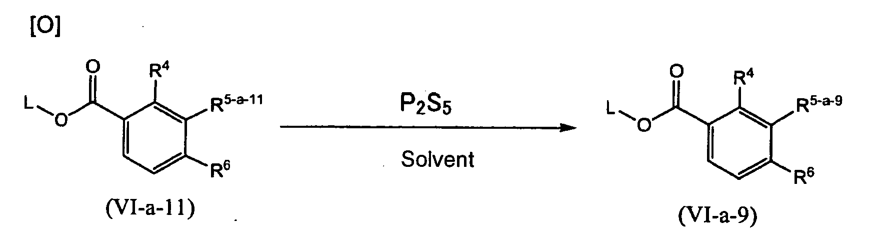 In the formulae, R⁴, R⁶ and L are as defined above, and R^{5-a-9} is amino(thiocarbonyl)alkyl, and R^{5-a-11} is cyanoalkyl.

The solvent which may be used for the above reaction may be any solvent so long as it is inert to the reaction, and ones exemplified for the above reaction (N) may be mentioned. As the solvent, one or more of them may suitably be selected for use.

The reaction temperature for the above reaction is usually from 0°C to 250°C, and the reaction time is usually from 1 minute to 48 hours.

Among compounds represented by the above formula (VI), a compound wherein R^{5-a} is R^{5-a-10}, may be produced in accordance with the following reaction (P). 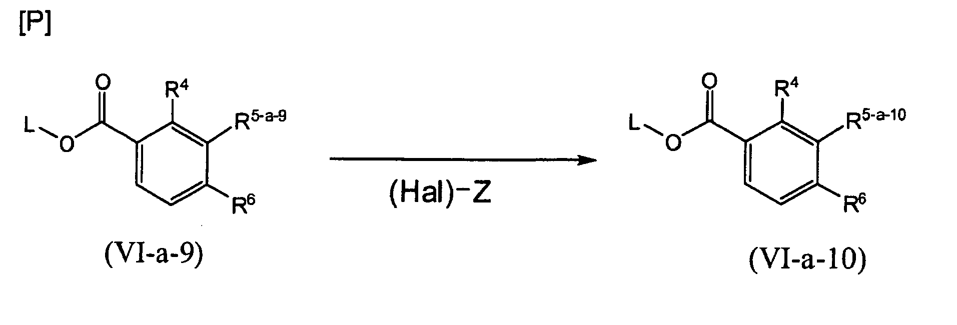 In the formulae, R⁴, R⁶, R^{5-a-9}, L and Hal are as defined above, and R^{5-a-10} is amino(thiocarbonyl)alkyl substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷, and Z is alkyl, cyano, cyanoalkyl, (alkylthio)carbonylalkyl, alkyl(thiocarbonyl)alkyl, -C(O)OR⁷ or -C(O)SR⁷, whereby R⁷ is as defined above.

The above reaction may be carried out in the presence of a solvent as the case requires. The solvent which may be used for the above reaction, may be any solvent so long as it is inert to the reaction, and ones exemplified for the above reaction (N) may be mentioned. As the solvent, one or more of them may be suitably selected for use.

The above reaction may be carried out in the presence of a base as the case requires. The base may be an inorganic base or an organic base. The inorganic base may, for example, be an alkali metal carbonate such as sodium carbonate, potassium carbonate or cesium carbonate; an alkali metal hydrogencarbonate such as sodium hydrogencarbonate or potassium hydrogencarbonate; or an alkaline earth metal carbonate such as calcium carbonate or magnesium carbonate. The organic base may, for example, be an amine such as triethylamine, N,N-dimethylaminopyridine, diisopropylaminopyridine or DBU (diazabicycloundecene).

The reaction temperature for the above reaction is usually from 0°C to 150°C, and the reaction time is usually from 1 minute to 48 hours.

The compound represented by the above formula (VI-a-11) may be produced in accordance with the following reaction (Q). 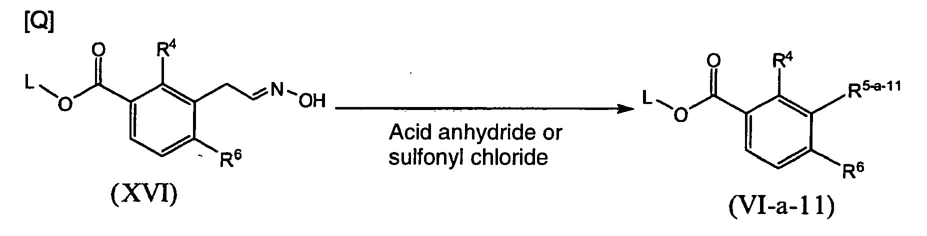 In the formulae, R⁴, R^{5-a-11}, R⁶ and L are as defined above.

The above reaction may be carried out in the presence of a solvent as the case requires. The solvent may be any solvent so long as it is inert to the reaction, and it may, for example, be a halogenated hydrocarbon such as methylene chloride, chloroform, dichloroethane or trichloroethane; a ketone such as acetone or methyl ethyl ketone; an ether such as diethyl ether, 1,4-dioxane, THF or 1,2-dimethoxyethane; an aromatic hydrocarbon such as benzene, toluene, xylene, nitrobenzene or chlorobenzene; or pyridine. As the solvent, one or more of them may be suitably selected for use.

The above reaction may be carried out in the presence of a base as the case requires. The base may, for example, be triethylamine, N,N-dimethylaminopyridine or diisopropylaminopyridine. As the base, one or more of them may be suitably selected for use.

The reaction temperature for the above reaction is usually from 0°C to 150°C, and the reaction time is usually from 1 minute to 48 hours.

The compound represented by the above formula (XVI) may be produced in accordance with the following reaction (R). 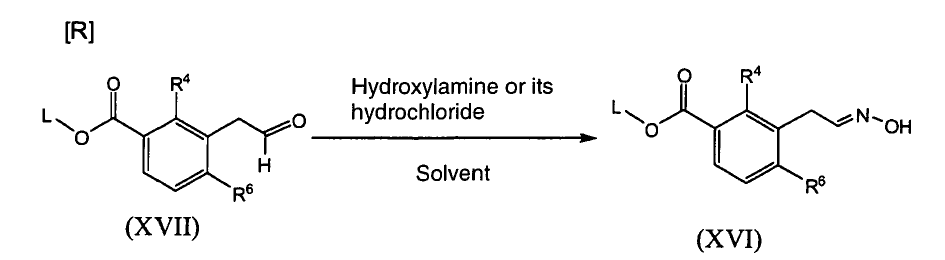 In the formulae, R⁴, R⁶ and L are as defined above.

The solvent which may be used for the above reaction, may be any solvent so long as it is inert to the reaction, and it may, for example, be an alcohol such as methanol, ethanol or propanol; a halogenated hydrocarbon such as methylene chloride, chloroform, dichloroethane or trichloroethane; a ketone such as acetone or methyl ethyl ketone; or an ether such as diethyl ether, 1,4-dioxane, THF or 1,2-dimethoxyethane. As the solvent, one or more of them may be suitably selected for use.

The above reaction may be carried out in the presence of an acid or a base as the case requires. The acid may, for example, be paratoluenesulfonic acid. The base may, for example, be sodium acetate.

The reaction temperature for the above reaction is usually from 0°C to 150°C, and the reaction time is usually from 1 minute to 48 hours.

The compound represented by the above formula (XVII) may be produced in accordance with the following reaction (S). 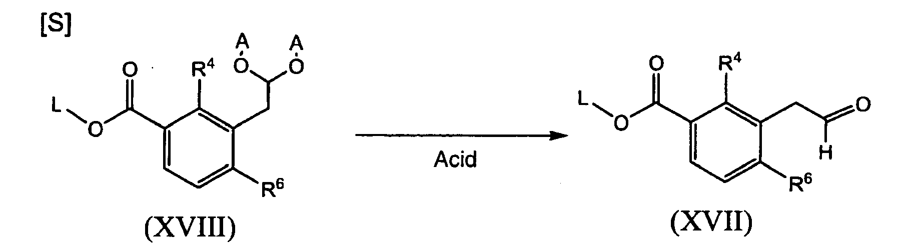 In the formulae, R⁴, R⁶ and L are as defined above, and A is alkyl.

The acid which may be used for the above reaction, may, for example, be hydrochloric acid.

The above reaction may be carried out in the presence of a solvent as the case requires. The solvent may be any solvent so long as it is inert to the reaction, and it may, for example, be water; an alcohol such as methanol, ethanol or propanol; or an ether such as diethyl ether, 1,4-dioxane, THF or 1,2-dimethoxyethane. As the solvent, one or more of them may be suitably selected for use.

The reaction temperature for the above reaction is usually from 0°C to 150°C, and the reaction time is usually from 1 minute to 48 hours.

The compound represented by the above formula (XVIII) may be produced in accordance with the following reaction (T). 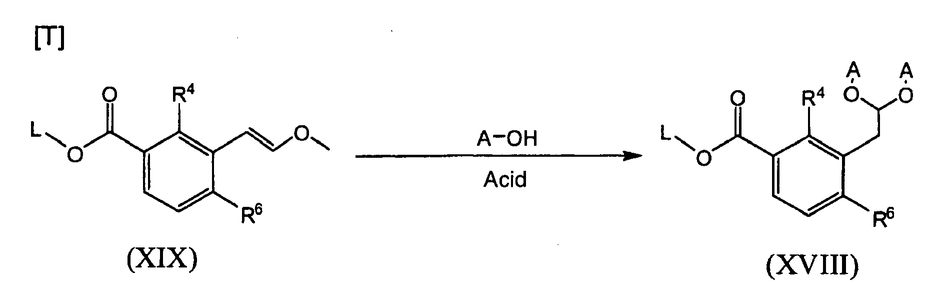 In the formulae, R⁴, R⁶, L and A are as defined above.

The acid which may be used for the above reaction, may, for example, be hydrochloric acid or toluenesulfonic acid.

In the above reaction, A-OH may be used in an excess amount so that it may serve also as a solvent. Otherwise, the above reaction may be carried out in the presence of another solvent as the case requires. The solvent may be any solvent so long as it is inert to the reaction, and it may, for example, be a halogenated hydrocarbon such as methylene chloride, chloroform, dichloroethane or trichloroethane; an aromatic hydrocarbon such as benzene, toluene, xylene, nitrobenzene or chlorobenzene; an ester such as methyl acetate, ethyl acetate or propyl acetate; an aprotic polar solvent such as acetonitrile, DMF, DMSO, DMA, HMPA or sulfolane; an ether such as diethyl ether, 1,4-dioxane, THF or 1,2-dimethoxyethane; or a ketone such as acetone or methyl ethyl ketone. As the solvent, one or more of them may be suitably selected for use.

The reaction temperature for the above reaction is usually from 0°C to 150°C, and the reaction time is usually from 1 minute to 48 hours.

The compound represented by the above formula (XIX) may be produced in accordance with the following reaction (U). 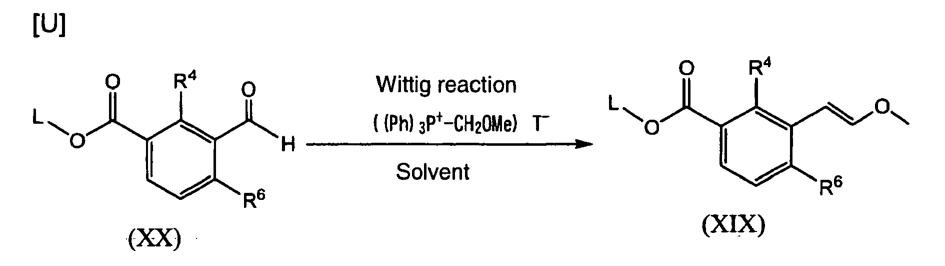 In the formulae, R⁴, R⁶ and L are as defined above, T is halogen, Ph is phenyl, Me is methyl.

The solvent which may be used for the above reaction, may be any solvent so long as it is inert to the reaction, and it may, for example, be an ether such as diethyl ether, 1,4-dioxane, THF or 1,2-dimethoxyethane; an aromatic hydrocarbon such as benzene, toluene, xylene, nitrobenzene or chlorobenzene; or an aprotic polar solvent such as acetonitrile, DMF, DMSO, DMA, HMPA or sulfolane.

The above reaction may be carried out in the presence of a base as the case requires. The base may, for example, be sodium hydride (NaH); an alkali lithium reagent such as n-butyllithium; or a metal amide such as sodium amide (NaNH₂).

The reaction temperature for the above reaction is usually from -80°C to 150°C, and the reaction time is usually from 1 minute to 48 hours.

The compound represented by the above formula (XX) may be produced in accordance with the following reaction (V). 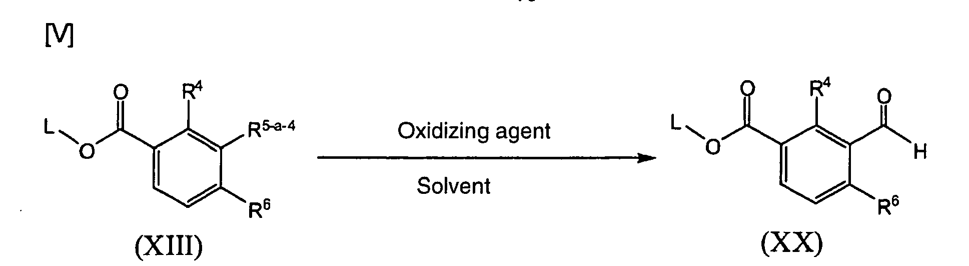 In the formulae, R⁴, R^{5-a-4}, R⁶ and L are as defined above.

The oxidizing agent which may be used for the above reaction may, for example, be an organic oxidizing agent such as N-methylmorpholine oxide; or an inorganic oxidizing agent such as manganese dioxide.

The solvent which may be used for the above reaction, may be any solvent so long as it is inert to the reaction, and it may, for example, be an aprotic polar solvent such as acetonitrile, DMF, DMSO, DMA, HMPA or sulfolane; or an ether such as diethyl ether, 1,4-dioxane, THF or 1,2-dimethoxyethane. As the solvent, one or more of them may be suitably selected for use.

The reaction temperature for the above reaction is usually from 0°C to 150°C, and the reaction time is usually from 1 minute to 48 hours.

Among compounds represented by the above formula (VI), a compound wherein R^{5-a} is R^{5-a-12} may be produced in accordance with the following reaction (W). 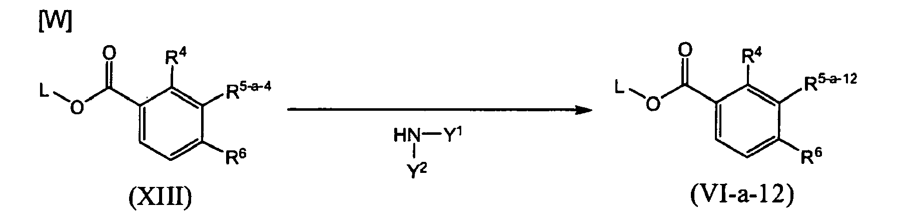 In the formulae, R⁴, R^{5-a-4}, R⁶ and L are as defined above, R^{5-a-12} is aminoalkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷, and each of Y¹ and Y² is a hydrogen atom, alkyl, cyano, cyanoalkyl, (alkylthio)carbonylalkyl, alkyl(thiocarbonyl)alkyl, -C(O)OR⁷ or -C(O)SR⁷, wherein R⁷ is as defined above.

The above reaction may be carried out in the presence of a solvent as the case requires. The solvent may be any solvent so long as it is inert to the reaction, and ones exemplified for the above reaction (A) may be mentioned. As the solvent, one or more of them may be suitably selected for use.

The above reaction may be carried out in the presence of a base as the case requires. The base may be an inorganic base or an organic base. The inorganic base may, for example, be an alkali metal carbonate such as sodium carbonate, potassium carbonate or cesium carbonate; an alkali metal hydrogencarbonate such as sodium hydrogencarbonate or potassium hydrogencarbonate; an alkaline earth metal carbonate such as calcium carbonate or magnesium carbonate; sodium hydride (NaH); an alkali lithium reagent such as n-butyllithium; or a metal amide such as sodium amide (NaNH₂). The organic base may, for example, be an amine such as triethylamine, N,N-dimethylaminopyridine, diisopropylaminopyridine or DBU (diazabicycloundecene).

The above reaction may be carried out in the presence of a catalyst as the case requires. The catalyst may, for example, be TBAI (tertiary butylammonium iodide).

The reaction temperature for the above reaction is usually from 0°C to 150°C, and the reaction time is usually from 1 minute to 48 hours.

Among compounds represented by the above formula (VI), a compound wherein R^{5-a} is R^{5-a-13}, may be produced in accordance with the following reaction (X). 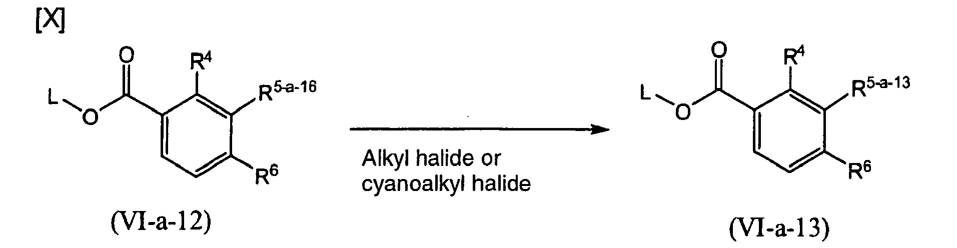 In the formulae, R⁴, R⁶ and L are as defined above, R^{5-a-13} is aminoalkyl substituted by alkyl or cyanoalkyl, and R^{5-a-16} is aminoalkyl.

The above reaction may be carried out in the presence of a solvent as the case requires. The solvent may be any solvent so long as it is inert to the reaction, and ones exemplified for the above reaction (N) may be mentioned. As the solvent, one or more of them may be suitably selected for use.

The above reaction may be carried out in the presence of a base as the case requires. The base may be an inorganic base or an organic base. The inorganic base may, for example, be an alkali metal carbonate such as sodium carbonate, potassium carbonate or cesium carbonate; an alkali metal hydrogencarbonate such as sodium hydrogencarbonate or potassium hydrogencarbonate; or an alkaline earth metal carbonate such as calcium carbonate or magnesium carbonate. The organic base may, for example, be an amine such as triethylamine, N,N-dimethylaminopyridine, diisopropylaminopyridine or DBU (diazabicycloundecene).

The reaction temperature for the above reaction is usually from 0°C to 150°C, and the reaction time is usually from 1 minute to 48 hours.

Among compounds represented by the above formula (VI), a compound wherein R^{5-a} is R^{5-a-14}, may be produced in accordance with the following reaction (Y). 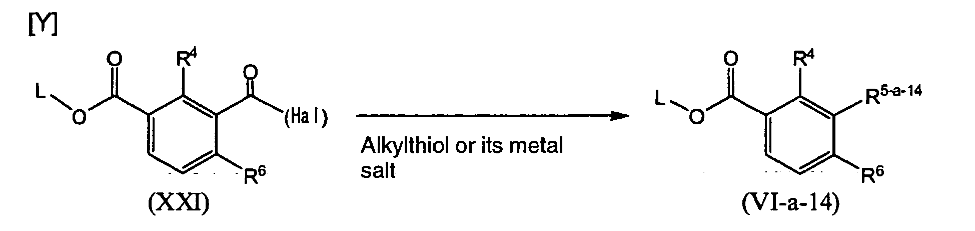 In the formulae, R⁴, R⁶, L and Hal are as defined above, and R^{5-a-14} is alkylthiocarbonyl.

The metal salt of alkylthiol which may be used for the above reaction, may, for example, be an alkali metal salt such as a sodium salt or a potassium salt.

The above reaction may be carried out in the presence of a solvent as the case requires. The solvent may be any solvent so long as it is inert to the reaction, and it may, for example, be an ether such as diethyl ether, 1,4-dioxane, THF or 1,2-dimethoxyethane; an aromatic hydrocarbon such as benzene, toluene, xylene, nitrobenzene or chlorobenzene; an ester such as methyl acetate, ethyl acetate or propyl acetate; a halogenated hydrocarbon such as methylene chloride, chloroform, dichloroethane or trichloroethane; or an aprotic polar solvent such as acetonitrile, DMF, DMSO, DMA, HMPA or sulfolane. As the solvent, one or more of them may be suitably selected for use.

The above reaction may be carried out in the presence of a base as the case requires. The base may be an inorganic base or an organic base. The organic base may, for example, be triethylamine, diisopropylethylamine, pyridine, 4-(dimethylamino)pyridine or 2,6-lutidine. The inorganic base may, for example, be an alkali metal carbonate such as sodium carbonate, potassium carbonate or cesium carbonate; an alkali metal hydrogencarbonate such as sodium hydrogencarbonate or potassium hydrogencarbonate; an alkaline earth metal carbonate such as calcium carbonate or magnesium carbonate; an alkali metal hydroxide such as sodium hydroxide or potassium hydroxide; or an alkali metal hydride such as sodium hydride or potassium hydride. As the base, one or more of them may be suitably selected for use.

The compound represented by the above formula (XXI) may be produced in accordance with the following reaction (Z). 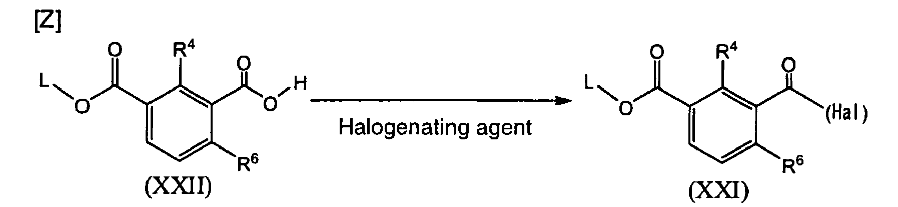 In the formulae, Hal, R⁴, R⁶ and L are as defined above.

This reaction may be carried out in the same manner as the above-described reaction (C).

The compound represented by the above formula (XXII) may be produced in accordance with the following reaction (AA). 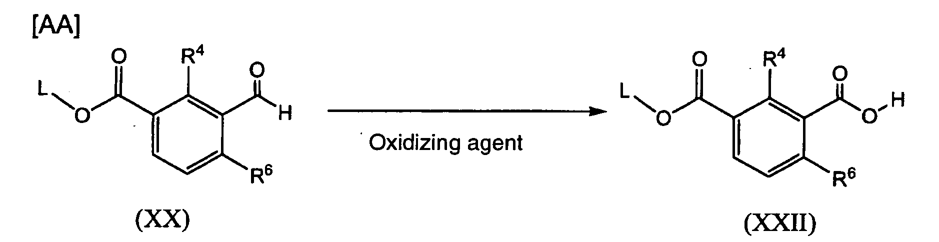 In the formulae, R⁴, R⁶ and L are as defined above.

In the above reaction, the oxidizing agent may, for example, be potassium permanganate, or chromium trioxide.

The above reaction may be carried out in the presence of a solvent as the case requires. The solvent may be any solvent so long as it is inert to the reaction, and it may, for example, be a ketone such as acetone or methyl ethyl ketone; an ester such as methyl acetate, ethyl acetate or propyl acetate; or an ether such as diethyl ether, 1,4-dioxane, THF or 1,2-methoxyethane.

As the solvent, one or more of them may be suitably selected for use.

The reaction temperature for the above reaction is usually from 0°C to 150°C, and the reaction time is usually from 1 minute to 48 hours.

Among compounds represented by the above formula (VI), a compound wherein R^{5-a} is R^{5-a-15}, may be produced in accordance with the following reaction (AB). 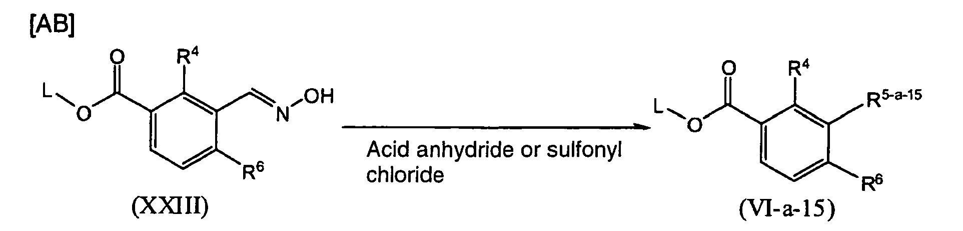 In the formulae, R⁴, R⁶ and L are as defined above, and R^{5-a-15} is cyano.

This reaction may be carried out in the same manner as the above-described reaction (Q).

The compound represented by the above formula (XXIII) may be produced in accordance with the following reaction (AC). 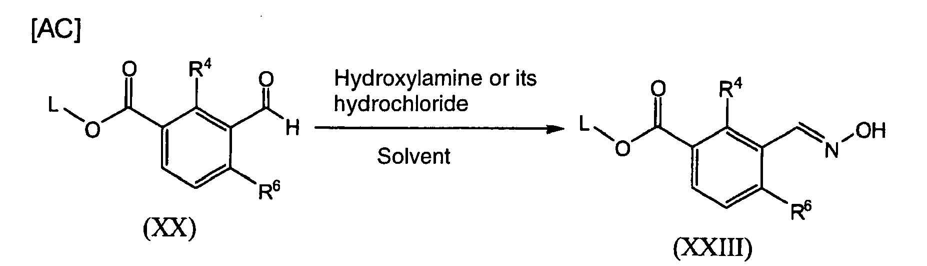 In the formulae, R⁴, R⁶ and L are as defined above.

This reaction may be carried out in the same manner as the above-described reaction (R).

Among compounds represented by the above formula (VI), a compound wherein R^{5-a} is R^{5-a-17} and R⁶ is R^{6-a-1}, may be produced in accordance with the following reaction (AD). 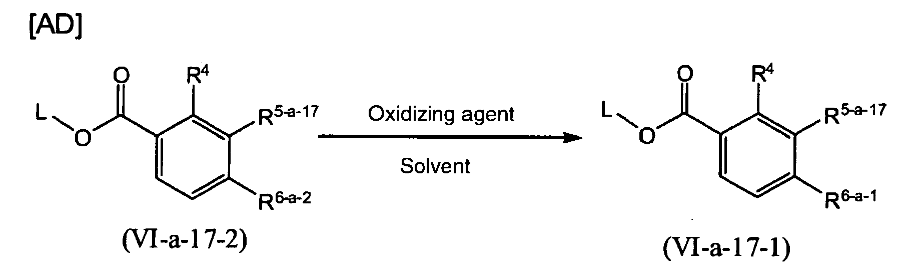 In the formulae, R⁴ and L are as defined above, R^{5-a-17} is alkoxy, alkenyloxy, alkynyloxy, haloalkoxy, alkoxyalkoxy, haloalkoxyalkoxy, alkoxyhaloalkoxy, haloalkoxyhaloalkoxy, alkoxycarbonylalkoxy, heterocyclyloxy, heterocyclylalkoxy, cycloalkyloxy or -OC(O)R⁷, R^{6-a-1} is alkylsulfinyl or alkylsulfonyl, and R^{6-a-2} is alkylthio.

This reaction may be carried out in the same manner as in the above-described reaction (J).

The compound represented by the above formula (VI-a-17-2) may be produced in accordance with the following reaction (AE). 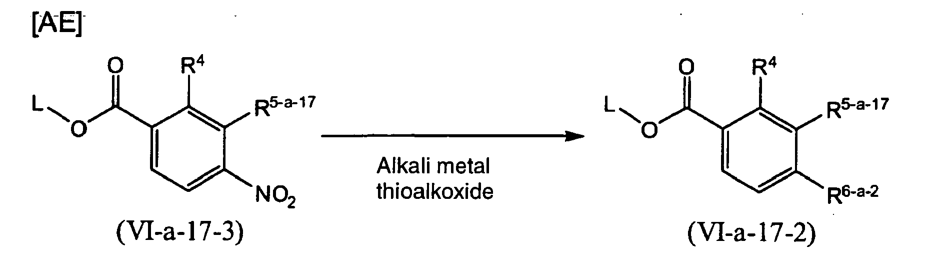 In the formulae, R⁴, R^{5-a-17}, R^{6-a-2} and L are as defined above.

The alkali metal thioalkoxide which may be used for the above reaction, may, for example, be sodium thiomethoxide or sodium thioethoxide.

The above reaction may be carried out in the presence of a solvent as the case requires. The solvent may, for example, be an aprotic polar solvent such as acetonitrile, DMF, DMSO, DMA, HMPA, sulfolane, dioxane or dimethoxyethane.

The reaction temperature for the above reaction is usually from 0°C to 150°C, and the reaction time is usually from 1 minute to 48 hours.

The compound represented by the above formula (VI-a-17-3) may be produced in accordance with the following reaction (AF). 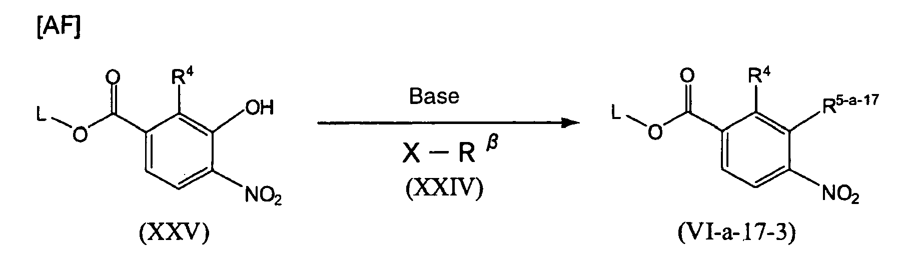 In the formulae, L, X, R⁴ and R^{5-a-17} are as defined above, and R^{β} is alkyl, alkenyl, alkynyl, haloalkyl, alkoxyalkyl, haloalkoxyalkyl, alkoxyhaloalkyl, haloalkoxyhaloalkyl, alkoxycarbonylalkyl, heterocyclyl, heterocyclylalkyl, cycloalkyl or -C(O)R⁷ (wherein R⁷ are as defined above).

The above reaction may be carried out in the presence of a solvent as the case requires. As the solvent, ones exemplified for the above reaction (A) may be used. One or more of them may be suitably selected for use.

The base which may be used for the above reaction may be an inorganic base or an organic base. The organic base may, for example, be triethylamine, diisopropylethylamine, pyridine, 4-(dimethylamino)pyridine or 2,6-lutidine. The inorganic base may, for example, be an alkali metal carbonate such as sodium carbonate, potassium carbonate or cesium carbonate; an alkali metal hydroxide such as sodium hydroxide or potassium hydroxide; or an alkali metal hydride such as sodium hydride or potassium hydride. As the base, one or more of them may be suitably selected for use in an amount of from 0.5 to 100 equivalents to the compound of the formula (XXV).

The above reaction may be carried out in the presence of a catalyst as the case requires. The catalyst may, for example, be potassium iodide or tetra-n-butylammonium iodide.

The reaction temperature for the above reaction is usually from 0°C to 150°C, and the reaction time is usually from 1 minute to 48 hours.

Among compounds represented by the above formula (V), a compound wherein R^{5-a} is R^{5-a-1}, may be produced in accordance with the following reaction (AG). 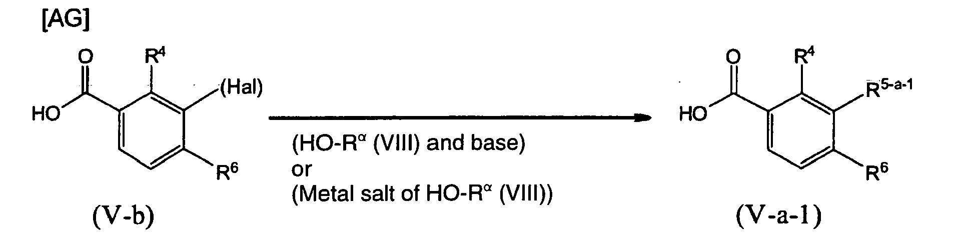 In the formulae, R⁴, R^{5-a-1}, R⁶, R^{α} and Hal are as defined above.

As the base which may be used for the above reaction, the same one as in the above-described reaction (G) may be used.

The metal salt of the compound represented by the formula (VIII) which may be used for the above reaction, may, for example, be an alkali metal salt such as a sodium salt or a potassium salt.

The above reaction may be carried out in the presence of a solvent as the case requires. The solvent may be any solvent so long as it is inert to the reaction, and ones exemplified for the above reaction (A) may be mentioned. As the solvent, one or more of them may be suitably selected for use.

The above reaction may be carried out in the presence of a catalyst as the case requires. As the catalyst, the same one as in the above-described reaction (G) may be used.

The reaction temperature for the above reaction is usually from 0°C to 200°C, and the reaction time is usually from 1 minute to 48 hours.

The above reaction may be carried out in the presence of an inert gas as the case requires. Such an inert gas may, for example, be nitrogen gas or argon gas.

Further, as the solvent which may be used for the reaction, other than ones mentioned for the above reaction (A), an alcohol represented by the formula (VIII) may be used in an excess amount so that it may serve also as a solvent.

Among compounds represented by the above formula (I), a compound wherein R⁵ is R^{5-a-1} may be produced in accordance with the following reaction (AH). 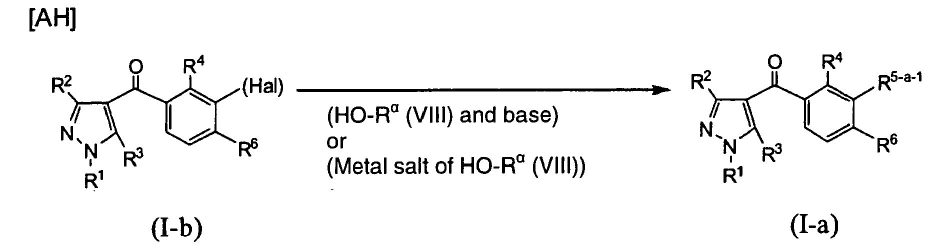 In the formulae, R¹, R², R³, R⁴, R^{5-a-1}, R⁶, R^{α} and Hal are as defined above.

As the base which may be used for the above reaction, the same one as for the above-described reaction (G) may be used.

The metal salt of the compound represented by the formula (VIII) which may be used for the above reaction, may, for example, be an alkali metal salt such as a sodium salt or a potassium salt.

The above reaction may be carried out in the presence of a solvent as the case requires. The solvent may be any solvent so long as it is inert to the reaction, and ones exemplified for the above reaction (A) may be mentioned. As the solvent, one or more of them may be suitably selected for use.

The above reaction may be carried out in the presence of a catalyst as the case requires. As the catalyst, the same one as for the above-described reaction (G) may be used.

The reaction temperature for the above reaction is usually from 0°C to 200°C, and the reaction time is usually from 1 minute to 48 hours.

The above reaction may be carried out in the presence of an inert gas as the case requires. Such an inert gas may, for example, be nitrogen gas or argon gas.

Further, as the solvent which may be used for the reaction, other than ones mentioned for the above reaction (A), an alcohol represented by the formula (VIII) may be used in an excess amount so that it may serve also as a solvent.

Among compounds represented by the above formula (V-b), a compound wherein R⁶ is R^{6-a-1}, may be produced in accordance with the following reaction (AI). 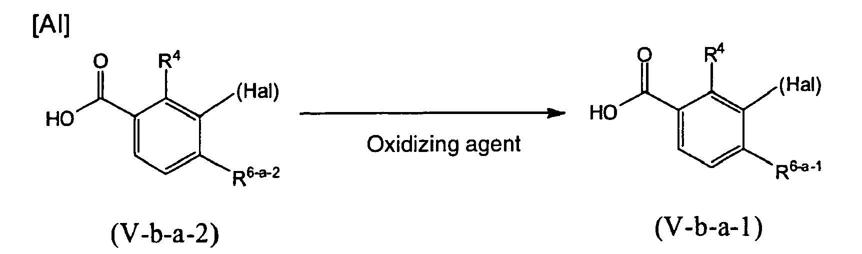 In the formulae, Hal, R⁴, R^{6-a-1} and R^{6-a-2} are as defined above.

This reaction may be carried out in the same manner as the above-described reaction (J).

The compound represented by the above formula (V-b-a-2) may be produced in accordance with the following reaction (AJ). 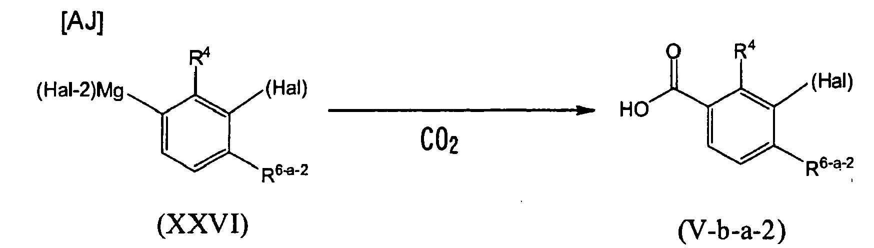 In the formulae, R⁴ and R^{6-a-2} are as defined above, and each of Hal and Hal-2 is halogen. Hal and Hal-2 may be the same or different from each other.

The solvent which may be used for the above reaction, may be any solvent so long as it is inert to the reaction, and it may, for example, be an ether such as diethyl ether, THF, 1,2-dimethoxyethane or 1,4-dioxane; or an aromatic hydrocarbon such as benzene, toluene, xylene, nitrobenzene or chlorobenzene. As the solvent, one or more of them may be suitably selected for use. CO₂ which may be used for the above reaction, may, for example, be CO₂ gas or dry ice.

The reaction temperature for the above reaction is usually from -20°C to 150°C, and the reaction time is usually from 1 minute to 48 hours.

The compound represented by the above formula (XXVI) may be produced in accordance with the following reaction (AK). 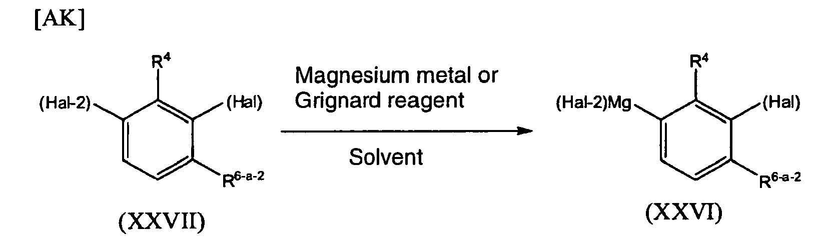 In the formulae, R⁴, R^{6-a-2}, Hal and Hal-2 are as defined above.

The solvent which may be used for the above reaction, may be any solvent so long as it is inert to the reaction, and it may, for example, be an ether such as diethyl ether, THF, 1,2-dimethoxyethane or 1,4-dioxane. As the solvent, one or more of them may be suitably selected for use.

The magnesium metal which may be used for the above reaction, may be used in an amount within a range of from 1 to 5 equivalents to the compound represented by the formula (XXVII). The Grignard reagent which may be used for the above reaction may, for example, be isopropylmagnesium chloride and may be used in an amount within a range of from 1 equivalent to 10 equivalents to the compound represented by the formula (XXVII) as the case requires.

The above reaction may be carried out in the presence of a catalyst as the case requires. The catalyst may, for example, be iodine, or an alkyl halide such as methyl iodide or isopropyl bromide.

The reaction temperature for the above reaction is usually from 0°C to 150°C, and the reaction time is usually from 10 minutes to 48 hours.

The above reaction may be carried out in the presence of an inert gas as the case requires. The inert gas may, for example, be nitrogen gas or argon gas.

The compound represented by the above formula (XXVII) may be produced in accordance with the following reaction (AL). 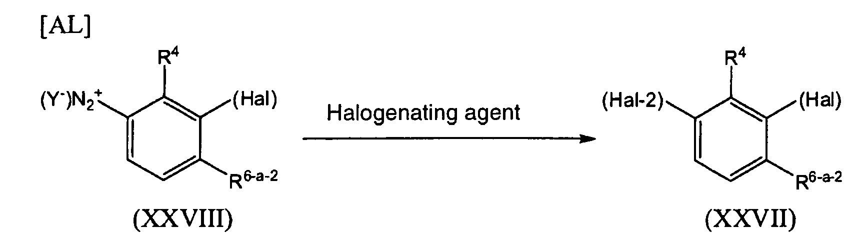 In the formulae, R⁴, R^{6-a-2}, Hal and Hal-2 are as defined above, and Y⁻ is Cl⁻, Br⁻, I⁻, BF₄⁻, HSO₃⁻, NO₃⁻, ClO₄⁻ or OH⁻.

The halogenating agent which may be used for the above reaction, may, for example, be a copper halide such as copper chloride, copper bromide or copper iodide; a hydrogen halide such as hydrogen chloride, hydrogen bromide or hydrogen iodide, or its aqueous solution; an alkali metal salt such as sodium iodide, potassium iodide or potassium bromide; a halogen itself such as chlorine, bromine or iodine; or a polyhalide such as bromoform.

The above reaction may be carried out by letting a phosphine such as triphenylphosphine or trialkylphosphine; or a sulfide such as dimethylsulfide, be present together with the above halogenating agent, or letting a phosphine or a sulfide be generated in the system, as the case requires.

The above reaction may be carried out in the presence of a solvent as the case requires. The solvent may be any solvent so long as it is inert to the reaction, and it may, for example, be an alcohol such as methanol or ethanol; a halogenated hydrocarbon such as chloroform or bromoform; acetonitrile; water; or a polar solvent such as DMSO. As the solvent, one or more of them may be suitably selected for use.

The reaction temperature for the above reaction is usually from -10°C to 150°C, and the reaction time is usually from 1 minute to 48 hours.

The compound represented by the above formula (XXVIII) may be produced in accordance with the following reaction (AM). 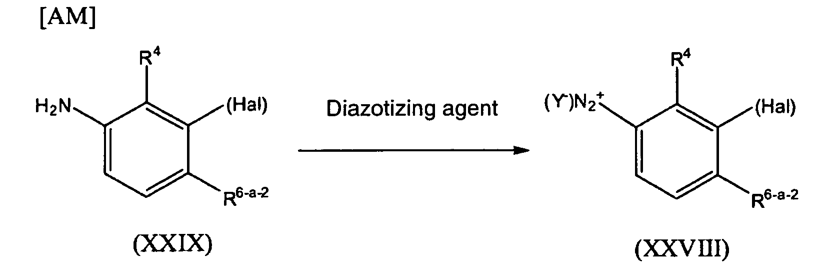 In the formulae, R⁴, R^{6-a-2}, Y⁻ and Hal are as defined above.

The diazotizing agent which may be used for the above reaction, may, for example, be an alkali metal nitrite such as sodium nitrite or potassium nitrite; an alkaline earth metal nitrite such as calcium nitrite or barium nitrite; or a nitrite ester such as methyl nitrite, ethyl nitrite, isopropyl nitrite or butyl nitrite.

The above reaction may be carried out in the presence of an acid as the case requires. The acid may, for example, be a hydrogen halide such as hydrogen chloride, hydrogen bromide or hydrogen iodide, or its aqueous solution; an acid such as sulfuric acid, nitric acid or perchloric acid, or its aqueous solution.

The above reaction may be carried out in the presence of a solvent as the case requires. The solvent which may be used for the above reaction, may be any solvent so long as it is inert to the reaction, and ones exemplified for the above reaction (AL) may be mentioned. As the solvent, one or more of them may be suitably selected for use.

The reaction temperature for the above reaction is usually from -20°C to 150°C, and the reaction time is usually from 1 minute to 48 hours.

The compound represented by the above formula (XXIX) may be produced in accordance with the following reaction (AN). 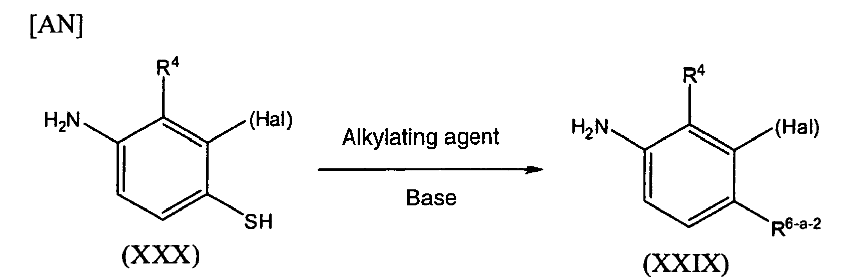 In the formulae, R⁴, R^{6-a-2} and Hal are as defined above.

The above reaction may be carried out in the presence of a solvent as the case requires. The solvent may be any solvent so long as it is inert to the reaction, and water or ones exemplified for the above reaction (A) may be mentioned. As the solvent, one or more of them may be suitably selected for use.

The alkylating agent which may be used for the above reaction, may, for example, be an alkyl halide such as methyl iodide, ethyl iodide, isopropyl iodide, n-propyl iodide, methyl bromide, ethyl bromide, isopropyl bromide or n-propyl bromide; or dimethylsulfuric acid.

The base which may be used for the above reaction, may, for example, be an alkali metal sulfide such as sodium sulfide or potassium sulfide or its hydrate, or the same one for the above-described reaction (G).

The above reaction may be carried out in the presence of a catalyst as the case requires. As the catalyst, ones exemplified for the above reaction (AF) may be used.

The reaction temperature for the above reaction is usually from -10°C to 200°C, and the reaction time is usually from 1 minute to 48 hours.

The compound represented by the above formula (XXX) may be produced in accordance with the following reaction (AO). 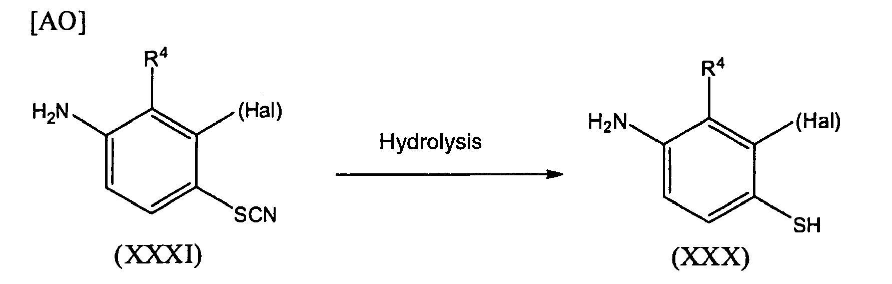 In the formulae, R⁴ and Hal are as defined above.

The above reaction may be carried out in the presence of water.

The above reaction may be carried out in the presence of a solvent as the case requires. The solvent may be any solvent so long as it is inert to the reaction, and ones exemplified for the above reaction (E) may be mentioned. As the solvent, one or more of them may be suitably selected for use.

The above reaction may be carried out in the presence of a base as the case requires. The base may be an inorganic base or an organic base. The organic base may, for example, be triethylamine, diisobutylethylamine, pyridine, 4-(dimethylamino)pyridine or 2,6-lutidine. The inorganic base may, for example, be an alkali metal carbonate such as sodium carbonate, potassium carbonate or cesium carbonate; an alkali metal hydroxide such as sodium hydroxide or potassium hydroxide; an alkali metal sulfide such as sodium sulfide or potassium sulfide; or a hydrate thereof. As the base, one or more of them may be suitably selected for use in an amount of from 0.1 to 100 equivalents to the compound of the formula (XXXI).

The reaction temperature for the above reaction is usually from 0°C to 150°C, and the reaction time is usually from 1 minute to 48 hours.

Among compounds represented by the above formula (V), a compound represented by the formula (V-a-1-3) wherein R⁶ is R^{6-a-3} may be produced in accordance with the following reaction (AP). 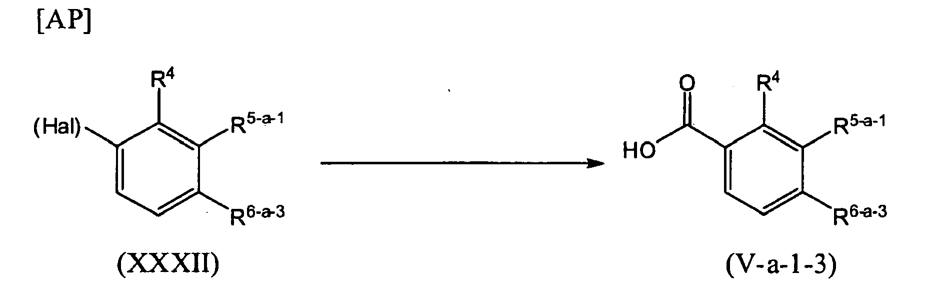 In the formulae, R⁴, R^{5-a-1} and Hal are as defined above, and R^{6-a-3} is alkylsulfonyl.

That is, the compound represented by the formula (V-a-1-3) may be produced by reacting a compound represented by the formula (XXXII) with carbon monoxide or its equivalent, and H₂O, in the presence of a catalyst and a base.

The base which may be used for the above reaction, may be an inorganic base or an organic base. The organic base may, for example, be triethylamine, tributylamine, diisobutylethylamine, pyridine, 4-(dimethylamino)pyridine or 2,6-lutidine. The inorganic base may, for example, be an alkali metal carbonate such as sodium carbonate, potassium carbonate or cesium carbonate; an alkali metal hydroxide such as sodium hydroxide or potassium hydroxide; an alkaline earth metal hydroxide such as calcium hydroxide or barium hydroxide; or an alkali metal acetate such as sodium acetate or potassium acetate. As the base, one or more of them may be suitably selected for use in an amount of from 0.1 to 100 equivalents to the compound represented by the formula (XXXII).

The catalyst to be used for the above reaction may, for example, be a metal catalyst, for example, a palladium catalyst such as palladium chloride, palladium acetate, palladium tetrakis(triphenylphosphine), palladium dichlorobis(triphenylphosphine), trans-di(µ-acetate)bis[o-(di-o-tolylphosphino)benzyl]dipalladium or palladium carbon; a ruthenium catalyst such as triruthenium dodecacarbonyl; or a rhodium catalyst such as rhodium(I) chlorobis(cyclooctene). As the catalyst, one or more of them may be suitably selected for use in an amount of from 10⁻¹⁰ to 1 equivalent to the compound represented by the formula (XXXII).

The equivalent to carbon monoxide which may be used for the above reaction, may, for example, be hexacarbonyl molybdenum, formic acid or chloroform. Carbon monoxide or its equivalent may be reacted under pressure, as the case requires, in an amount of from 1 to 1,000 equivalents to the compound represented by the formula (XXXII). The pressure may suitably be selected with a range of from 1 to 100 MPa.

The above reaction may be carried out in the presence of a solvent as the case requires. The solvent may be any solvent so long as it is inert to the reaction, and water or ones exemplified for the above reaction (A) may be mentioned. As the solvent, one or more of them may be suitably selected for use.

The above reaction may be carried out in the presence of a ligand as the case requires. The ligand may, for example, be tri-t-butylphosphine, tricyclohexylphosphine, triphenylphosphine, tri(o-tolyl)phosphine, 4-bis(diphenylphosphino)butane, 1,3-bis(diphenylphosphino)pentane or 1,1'-bis(diphenylphosphino)ferrocene. As the ligand, one or more of them may be suitably selected for use in an amount of from 10⁻¹⁰ to 1 equivalent to the compound represented by the formula (XXXII).

The above reaction may be carried out in the presence of a cocatalyst as the case requires. The cocatalyst may, for example, be an alkali metal halide such as sodium chloride, potassium chloride, sodium bromide or potassium bromide; or a quaternary ammonium salt such as tetra(n-butyl)ammonium bromide. As the cocatalyst, one or more of them may be suitably selected for use in an amount of from 0.001 to 1 equivalent to the compound represented by the formula (XXXII).

The above reaction may be carried out in the presence of an inert gas as the case requires. The inert gas may, for example, be nitrogen gas or argon gas.

The reaction temperature for the above reaction is usually from 0°C to 300°C, and the reaction time is usually from 1 minute to 72 hours.

The compound represented by the above formula (XXXII) may be produced in accordance with the following reaction (AQ). 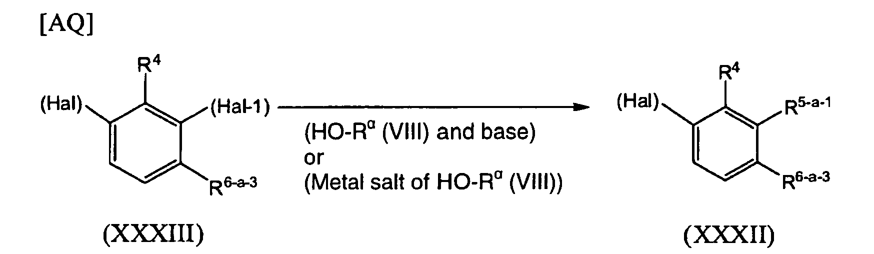 In the formulae, R^{α}, R⁴, R^{5-a-1}, R^{6-a-3}, Hal-1 and Hal are as defined above.

The above reaction may be carried out in the presence of a base as the case requires. The base may, for example, be an alkali metal carbonate such as sodium carbonate, potassium carbonate or cesium carbonate; an alkaline earth metal carbonate such as calcium carbonate or barium carbonate; an alkali metal hydroxide such as lithium hydroxide, sodium hydroxide or potassium hydroxide; an alkaline earth metal hydroxide such as calcium hydroxide or barium hydroxide; an alkali metal hydride such as lithium hydride, sodium hydride or potassium hydride; an alkali lithium reagent such as n-butyllithium; sodium amide (NaNH₂); or a Grignard reagent such as methylmagnesium bromide or isopropylmagnesium chloride.

The metal salt of the compound represented by the formula (VIII) which may be used for the above reaction may be the same one as for the above-described reaction (AH).

The above reaction may be carried out in the presence of a solvent as the case requires. The solvent may be any solvent so long as it is inert to the reaction, and it may, for example, be an alcohol such as methanol, ethanol or methoxyethanol; an ether such as diethyl ether, tetrahydrofuran, 1,4-dioxane, 1,2-dimethoxyethane or cyclohexyl methyl ether; an aprotic polar solvent such as DMF, DMSO, DMA or sulfolane; or a non-polar solvent such as benzene or toluene. Otherwise, the compound represented by the formula (VIII) may serve as the reaction reagent and a solvent. As the solvent, one or more of them may be suitably selected for use.

The above reaction may be carried out in the presence of an inert gas as the case requires. The inert gas may, for example, be nitrogen gas or argon gas.

The reaction temperature for the above reaction is usually from 0°C to 200°C, and the reaction time is usually from 1 minute to 48 hours.

The compound represented by the above formula (XXXIII) may be produced in accordance with the following reaction (AR). 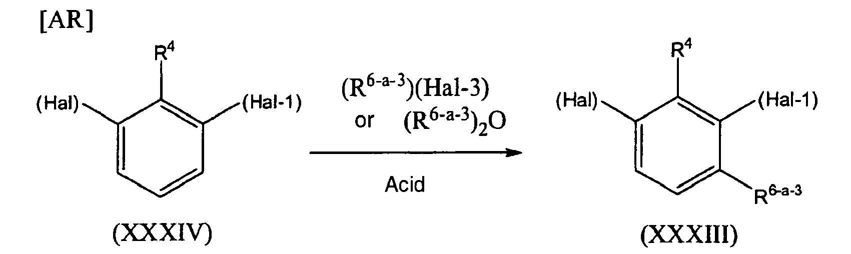 In the formulae, R⁴, R^{6-a-3}, Hal-1 and Hal are as defined above, and Hal-3 is halogen, and Hal-1, Hal and Hal-3 may be the same or different from one another.

The acid which may be used for the above reaction may, for example, be a Lewis acid such as zinc chloride, zinc bromide, aluminum chloride, aluminum bromide, iron chloride, bismuth chloride, indium chloride, antimony chloride, boron tribromide or iron bromide; methanesulfonic acid; sulfuric acid; or trifluoromethanesulfonic acid. It may be used within a range of from 0.01 to 10 equivalents to the compound represented by the formula (XXXIV). As the acid, one or more of them may be suitably selected for use as the case requires.

The alkylsulfonyl halide [(R^{6-a-3})(Hal-3)] or the alkylsulfonic acid anhydride [(R^{6-a-3})₂O] which may be used for the above reaction, may be used in an amount within a range of from 1 to 10 equivalents to the compound represented by the formula (XXXIV). The halogen in the alkylsulfonyl halide may, for example, be fluorine, chlorine, bromine or iodine.

The above reaction may be carried out in the presence of a solvent as the case requires. The solvent may be any solvent so long as it inert to the reaction, and it may, for example, be a halogenated hydrocarbon such as methylene chloride, chloroform, dichloroethane or trichloroethane; carbon disulfide or nitromethane. As the solvent, one or more of them may be suitably selected for use.

The reaction temperature for the above reaction is usually from 0°C to 250°C, and the reaction time is usually from 1 minute to 48 hours.

Among compounds represented by the above formula (I-a), a compound represented by the formula (I-a-3) wherein R⁶ is R^{6-a-3} may be produced in accordance with the following reaction (AS). 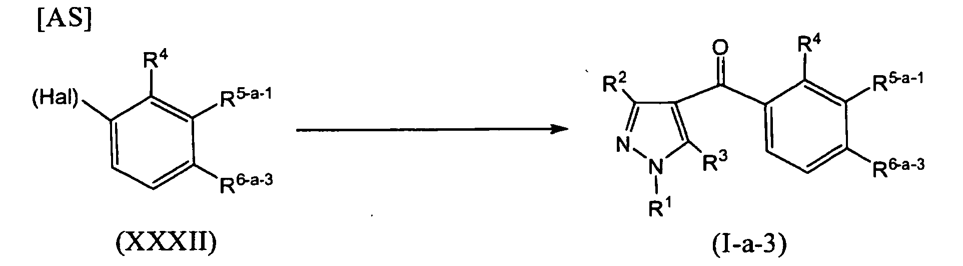 In the formulae, R¹, R², R³, R⁴, R^{5-a-1}, R^{6-a-3} and Hal are as defined above.

That is, the compound represented by the formula (I-a-3) may be produced by reacting a compound represented by the formula (XXXII) with carbon monoxide or its equivalent, and a compound represented by the formula (III): 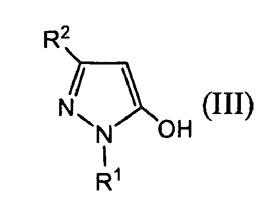 wherein R¹ and R² are as defined above, or its salt, in the presence of a catalyst and a base.

As the salt of the compound represented by the formula (III), ones exemplified for the above reaction (B) may be mentioned.

The base which may be used for the above reaction, may be an inorganic base or an organic base, and ones exemplified for the above reaction (AP) may be mentioned. As the base, one or more of them may be suitably selected for use in an amount of from 0.1 to 100 equivalents to the compound represented by the formula (XXXII).

As the catalyst which may be used for the above reaction, ones exemplified for the above reaction (AP) may be mentioned. As the catalyst, one or more of them may be suitably selected for use in an amount of from 10⁻¹⁰ to 1 equivalent to the compound represented by the formula (XXXII).

The equivalent to carbon monoxide which may be used for the above reaction, may, for example, be hexacarbonyl molybdenum, formic acid or chloroform. Carbon monoxide or its equivalent can be reacted under pressure as the case requires, in an amount of from 1 to 1,000 equivalents to the compound represented by the formula (XXXII). The pressure may suitably be selected within a range of from 1 to 100 MPa.

The above reaction may be carried out in the presence of a solvent as the case requires. The solvent may be any solvent so long as it is inert to the reaction, and ones exemplified for the above reaction (A) may be mentioned. As the solvent, one or more of them may be suitably selected for use.

The above reaction may be carried out in the presence of a ligand as the case requires. As the ligand, ones exemplified for the above reaction (AP) may be mentioned. As the ligand, one or more of them may be suitably selected for use in an amount of from 10⁻¹⁰ to 1 equivalent to the compound represented by the formula (XXXII).

The above reaction may be carried out in the presence of a cocatalyst as the case requires. As the cocatalyst, ones exemplified for the above reaction (AP) may be mentioned. As the cocatalyst, one or more of them may be suitably selected for use in an amount of from 0.001 to 1 equivalent to the compound represented by the formula (XXXII).

The above reaction may be carried out in the presence of an inert gas as the case requires. The inert gas may, for example, be nitrogen gas or argon gas.

The reaction temperature for the above reaction is usually from 0°C to 300°C, and the reaction time is usually from 1 minute to 72 hours.

Among compounds represented by the above formula (I-a-Hal), a compound of the formula (I-a-Hal-3) wherein R⁶ is R^{6-a-3}, may be produced in accordance with the following reaction (AT). 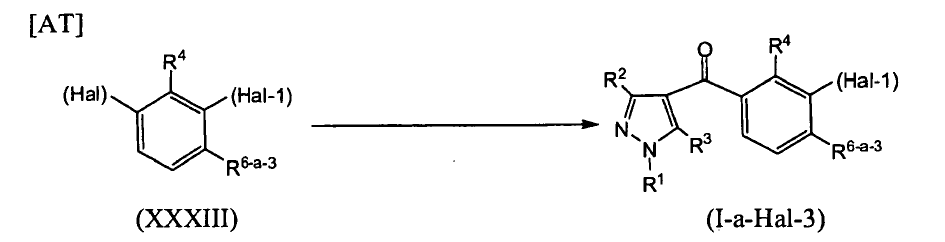 In the formulae, R¹, R², R³, R⁴, R^{6-a-3}, Hal-1 and Hal are as defined above.

That is, the compound represented by the formula (I-a-Hal-3) may be produced by reacting a compound represented by the formula (XXXIII) with carbon monoxide or its equivalent, and a compound represented by the formula (III): 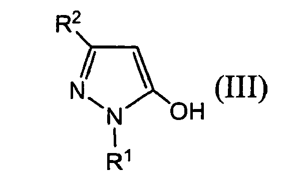 wherein R¹ and R² are as defined above, or its salt, in the presence of a catalyst and a base.

This reaction may be carried out in the same manner as the above-described reaction (AS).

The herbicides of the present invention have excellent herbicidal effects. The application range extends to agricultural fields such as paddy fields, crop plant fields, orchards and mulberry fields and non-agricultural fields such as forest land, farm roads, play grounds and factory sites. The application method may suitably be selected from soil application, foliar application, water application, etc.

The herbicides of the present invention are capable of controlling a wide range of undesired weeds, such as gramineae such as barnyardgrass (Echinochloa crus-galli L., Echinochloa oryzicola vasing.), crabgrass (Digitaria sanguinalis L., Digitaria ischaemum Muhl., Digitaria adscendens Henr., Digitaria microbachne Henr., Digitaria horizontalis Willd.), green foxtail Setaria viridis L.), giant foxtail (Setaria faberi Herrm.), goosegrass (Eleusine indica L.), wild oat (Avena fatua L.), johnsongrass (Sorghum halepense L.), quackgrass (Agropyron repens L.), alexandergrass (Brachiaria plantaginea), guineagrass (Panicum maximum Jacq.), paragrass (Panicum purpurascens), sprangletop (Leptochloa chinensis), red sprangletop (Leptochloa panicea), annual bluegrass (Poa annua L.), black grass (Alopecurus myosuroides Huds.), cholorado bluestem (Agropyron tsukushiense (Honda) Ohwi), cyperaceae such as rice flatsedge (Cyperus iria L.), purple nutsedge (Cyprus rotundus L.), yellow nutsedge (Cyperus esculentus L.), Japanese bulrush (Scirpus juncoides), flatsedge (Cyprus serotinus), small-flower umbrellaplant (Cyprus difformis), slender spikerush (Eleocharis acicularis), and water chestnut (Eleocharis kuroguwai); alismataceae such as Japanese ribbon waparo (Sagittaria pygmaea), arrow-head (Sagittaria trifolia), and narrowleaf waterplantain (Alisma canaliculatum); pontederiaceae such as monochoria (Monochoria vaginalis), and monochoria species (Monochoria korsakowii); scrophulariaceae such as false pimpernel (Lindernia pyxidaria), and abunome (Dopatrium junceum); Iythraceae such as toothcup (Rotala india), and red stem (Ammannia multiflora); elatinaceae such as long stem waterwort (Elatine triandra SCHK.); malvaceae such as velvetleaf (Abutilon theophrasti MEDIC.), and prickly sida (Sida spinosa L.); compositae such as common cocklebur (Xanthium strumarium L.), common ragweed (Ambrosia elatior L.), thistle (Breea setosa (BIEB.) KITAM.), solanaceae such as black nightshade (Solanum nigrum L.), and jimsonweed (Datura stramonium); amaranthaceae such as slender amaranth (Amaranthus viridis L.), and redroot pigweed (Amaranthus retroflexus L.); polygonaceeae such as pale smartweed (Polyaonum lapathifolium L.), ladysthumb (Polygonum persicaria L.), cruciferae such as flexuous bittercress (Cardamine flexuosa WITH.) and shepherd's-purse (Capsella bursa-pastoris Medik.); convolvulaceae such as tall momingglory (Ipomoea purpurea L.), field bindweed (Calystegia arvensis L.), and ivyleaf morningglory (Ipomoea hederacea Jacq.); Chenopodiaceae such as common lambsquarters (Chenopodium album L.); Portulacaceae such as common purslane (Portulaca oleracea L.); leguminosae such as sicklepod (Cassia obtusifolia L.); caryophyllaceae such as common chickweed (Stellaria media L.); labiatae such as henbit (Lamium amplexicaule L.); rubiaceae such as catchweed (Galium spurium L.); euphorbiaceae such as threeseeded copperleaf (Acalypha australis L.); and Commelinaceae such as common dayflower (Commelina communis L.).

Therefore, they can be effectively used for selectively controlling noxious weeds or nonselectively controlling noxious weeds in cultivation of useful crops such as corn (Zea mays L.), soybean (Glycine max Merr.), cotton (Gossypium spp.), wheat (Triticum spp.), rice (Oryza sativa L.), barley (Hordeum vulgare L.), oat (Avena sativa L.), sorgo (Sorghum bicolor Moench), rape (Brassica napus L.), sunflower (Helianthus annuus L.), sugar beet (Beta vulgaris L.), sugar cane (Saccharum officinarum L.), japanese lawngrass (Zoysia japonica stend), peanut (Arachis hypogaea L.), flax (Linum usitatissimum L.), tobacco (Nicotiana tabacum L.), and coffee (Coffea spp.). Particularly, the herbicides of the present invention are effectively used for selectively controlling noxious weeds in cultivation of corn, soybean, cotton, wheat, rice, rape, sunflower, sugar beet, sugar cane, japanese lawngrass, peanut, flax, tobacco, coffee, and the like, and among these, especially corn, wheat, rice, japanese lawngrass and the like.

The herbicides of the present invention may be mixed with the compound of the above formula (I) and various agricultural additives and applied in the form of various formulations such as dusts, granules, water dispersible granules, wettable powders, tablets, pills, capsules (including a formulation packaged by a water soluble film), water-based suspensions, oil-based suspensions, microemulsions, suspoemulsions, water soluble powders, emulsifiable concentrates, soluble concentrates or pastes. It may be formed into any formulation which is commonly used in this field, so long as the object of the present invention is thereby met.

The additives to be used for the formulation include, for example, a solid carrier such as diatomaceous earth, slaked lime, calcium carbonate, talc, white carbon, kaoline, bentonite, kaolinite, sericite, clay, sodium carbonate, sodium bicarbonate, mirabilite, zeolite or starch; a solvent such as water, toluene, xylene, solvent naphtha, dioxane, acetone, isophorone, methyl isobutyl ketone, chlorobenzene, cyclohexane, dimethyl sulfoxide, N,N-dimethylformamide, dimethylacetamide, N-methyl-2-pyrrolidone or an alcohol; an anionic surfactant such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester, a salt of polyoxyethylene aryl ether phosphoric acid ester, a naphthalene sulfonate condensed with formaldehyde or an alkylnaphthalene sulfonate condensed with formaldehyde; a nonionic surfactant such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, polyethylene glycol, a polyoxyethylene fatty acid ester, a polyoxyethylene sorbitan fatty acid ester, a polyoxyethylene glycerin fatty acid ester, a polyoxyethylene hydrogenated castor oil or a polyoxypropylene fatty acid ester; and a vegetable oil or mineral oil such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil or liquid paraffins. These additives may suitably be selected for use alone or in combination as a mixture of two or more of them, so long as the object of the present invention is met. Further, additives other than the above-mentioned may be suitably selected for use among those known in this field. For example, various additives commonly used, such as a filler, a thickener, an antisettling agent, an anti-freezing agent, a dispersion stabilizer, a safener, an anti-mold agent, a bubble agent, a disintegrator and a binder, may be used. The mix ratio by weight of the compound of the above formula(I) to such various additives may be from 0.1:99.9 to 95:5, preferably from 0.2:99.8 to 85:15.

The herbicidal compositions of the present invention have excellent herbicidal effects. The application range extends to agricultural fields such as paddy fields, crop plant fields, orchards and mulberry fields and non-agricultural fields such as forest land, farm roads, play grounds and factory sites. The application method may suitably be selected from soil application, foliar application, water application, etc.

The dose of the herbicide of the present invention can not generally be defined, as it varies depending upon the weather conditions, the soil conditions, the type of the formulation, the type of the weeds to be controlled, the application season, etc. However, it is usually applied in an amount of the compound of the above formula (I) of from 0.1 to 10,000 g/ha, preferably from 1 to 2,000 g/ha, more preferably from 1 to 1,000 g/hr. The present invention includes such a method for controlling undesired weeds, by such applications of the herbicide.

Further, the herbicide of the present invention may be mixed with or may be used in combination with other agricultural chemicals, fertilizers or phytotoxicity-reducing agents, whereby synergistic effects or activities may sometimes be obtained. Such other agricultural chemicals include, for example, a herbicide, a fungicide, an antibiotic, a plant hormone and an insecticide. Especially, with a mixed herbicidal composition having a compound of the above formula (I) mixed with or used in combination with one or more active compounds of other herbicides, the range of weeds to be controlled, the time of application of the composition, the herbicidal activities, etc. may be improved to preferred directions. The compound of the above formula (I) and the active compounds of other herbicides may separately be formulated so that they may be mixed for use at the time of application, or they may be formulated together. The present invention includes such a mixed herbicidal composition.

Another herbicidally active compound includes, for example, the following compounds (common names; including some which are under application for approval by ISO, or test codes), and one or more of them may be suitably selected for use. Even when not specifically mentioned here, in a case where such compounds have salts, alkyl esters, hydrates, different crystal forms, various structural isomers, etc., they are, of course, all included.
(1) Those which are believed to exhibit herbicidal effects by disturbing hormone activities of plants, such as a phenoxy type such as 2,4-D, 2,4-D-butotyl, 2,4-D-butyl, 2,4-D-dimethylammonimum, 2,4-D-diolamine, 2,4-D-ethyl, 2,4-D-2-ethylhexyl, 2,4-D-isobutyl, 2,4-D-isoctyl, 2,4-D-isopropyl, 2,4-D-isopropylammonium, 2,4-D-sodium, 2,4-D-isopropanolammonium, 2,4-D-trolamine, 2,4-DB, 2,4-DB-butyl, 2,4-DB-dimethylammonium, 2,4-DB-isoctyl, 2,4-DB-potassium, 2,4-DB-sodium, dichlorprop, dichlorprop-butotyl, dichlorprop-dimethylammonium, dichlorprop-isoctyl, dichlorprop-potassium, dichlorprop-P, dichlorprop-P-dimethylammonium, dichlorprop-P-potassium, dichlorprop-P-sodium, MCPA, MCPA-butotyl, MCPA-dimethylammonium, MCPA-2-ethylhexyl, MCPA-potassium, MCPA-sodium, MCPA-thioethyl, MCPB, MCPB-ethyl, MCPB-sodium, mecoprop, mecoprop-butotyl, mecoprop-sodium, mecoprop-P, mecoprop-P-butotyl, mecoprop-P-dimethylammonium, mecoprop-P-2-ethylhexyl, mecoprop-P-potassium, naproanilide or clomeprop; an aromatic carboxylic acid type such as 2,3,6-TBA, dicamba, dicamba-butotyl, dicamba-diglycolamine, dicamba-dimethylammonium, dicamba-diolamine, dicamba-isopropylammonium, dicamba-potassium, dicamba-sodium, dichlobenil, picloram, picloram-dimethylammonium, picloram-isoctyl, picloram-potassium, picloram-triisopropanolammonium, picloram-triisopropylammonium, picloram-trolamine, triclopyr, triclopyr-butotyl, triclopyr-triethylammonium, clopyralid, clopyralid-olamine, clopyralid-potassium, clopyralid-triisopropanolammonium or aminopyralid; and others such as naptalam, naptalam-sodium, benazolin, benazolin-ethyl, quinclorac, quinmerac, diflufenzopyr, diflufenzopyr-sodium, fluroxypyr, fluroxypyr-2-butoxy-1-methylethyl, fluroxypyr-meptyl, chlorflurenol or chlorflurenol-methyl.
(2) Those which are believed to exhibit herbicidal effects by inhibiting photosynthesis of plants, such as a urea type such as chlorotoluron, diuron, fluometuron, linuron, isoproturon, metobenzuron, tebuthiuron, dimefuron, isouron, karbutilate, methabenzthiazuron, metoxuron, monolinuron, neburon, siduron, terbumeton or trietazine; a triazine type such as simazine, atrazine, atratone, simetryn, prometryn, dimethametryn, hexazinone, metribuzin, terbuthylazine, cyanazine, ametryn, cybutryne, triaziflam, terbutryn, propazine, metamitron, or prometon; a uracil type such as bromacil, bromacyl-lithium, lenacil or terbacil; an anilide type such as propanil or cypromid; a carbamate type such as swep, desmedipham or phenmedipham; a hydroxybenzonitrile type such as bromoxynil, bromoxynil-octanoate, bromoxynil-heptanoate, ioxynil, ioxynil-octanoate, ioxynil-potassium or ioxynil-sodium; and others such as pyridate, bentazone, bentazone-sodium, amicarbazone, methazole or pentanochlor.
(3) Quaternary ammonium salt type such as paraquat or diquat, which is believed to be converted to free radicals by itself to form active oxygen in the plant body and shows rapid herbicidal efficacy.
(4) Those which are believed to exhibit herbicidal effects by inhibiting chlorophyll biosynthesis of plants and abnormally accumulating a photosensitizing peroxide substance in the plant body, such as a diphenylether type such as nitrofen, chlomethoxyfen, bifenox, acifluorfen, acifluorfen-sodium, fomesafen, fomesafen-sodium, oxyfluorfen, lactofen, aclonifen, ethoxyfen-ethyl(HC-252), fluoroglycofen-ethyl or fluoroglycofen; a cyclic imide type such as chlorphthalim, flumioxazin, flumiclorac, flumiclorac-pentyl, cinidon-ethyl or fluthiacet-methyl; and others such as oxadiargyl, oxadiazon, sulfentrazone, carfentrazone-ethyl, thidiazimin, pentoxazone, azafenidin, isopropazole, pyraflufen-ethyl, benzfendizone, butafenacil, saflufenacil, flupoxam, fluazolate, profluazol, pyraclonil, flufenpyr-ethyl or bencarbazone.
(5) Those which are believed to exhibit herbicidal effects characterized by bleaching activities by inhibiting chromogenesis of plants such as carotenoids, such as a pyridazinone type such as norflurazon, chloridazon or metflurazon; a pyrazole type such as pyrazolynate, pyrazoxyfen, benzofenap, topramezone(BAS-670H) or pyrasulfotole; and others such as amitrole, fluridone, flurtamone, diflufenican, methoxyphenone, clomazone, sulcotrione, mesotrione, tembotrione, tefuryltrione (AVH-301), isoxaflutole, difenzoquat, difenzoquat-metilsulfate, isoxachlortole, benzobicyclon, picolinafen or beflubutamid.
(6) Those which exhibit strong herbicidal effects specifically to gramineous plants, such as an aryloxyphenoxypropionic acid type such as diclofop-methyl, diclofop, pyriphenop-sodium, fluazifop-butyl, fluazifop, fluazifop-P, fluazifop-P-butyl, haloxyfop-methyl, haloxyfop, haloxyfop-etotyl, haloxyfop-P, haloxyfop-P-methyl, quizalofop-ethyl, quizalofop-P, quizalofop-P-ethyl, quizalofop-P-tefuryl, cyhalofop-butyl, fenoxaprop-ethyl, fenoxaprop-P, fenoxaprop-P-ethyl, metamifop-propyl, metamifop, clodinafop-propargyl, clodinafop or propaquizafop; a cyclohexanedione type such as alloxydim-sodium, alloxydim, clethodim, sethoxydim, tralkoxydim, butroxydim, tepraloxydim, profoxydim or cycloxydim; or others such as flamprop-M-methyl, flamprop-M or flamprop-M-isopropyl.
(7) Those which are believed to exhibit herbicidal effects by inhibiting an amino acid biosynthesis of plants, such as a sulfonylurea type such as chlorimuron-ethyl, chlorimuron, sulfometuron-methyl, sulfometuron, primisulfuron-methyl, primisulfuron, bensulfuron-methyl, bensulfuron, chlorsulfuron, metsulfuron-methyl, metsulfuron, cinosulfuron, pyrazosulfuron-ethyl, pyrazosulfuron, flazasulfuron, rimsulfuron, nicosulfuron, imazosulfuron, cyclosulfamuron, prosulfuron, azimsulfuron, flupyrsulfuron-methyl-sodium, flupyrsulfuron, triflusulfuron-methyl, triflusulfuron, halosulfuron-methyl, halosulfuron, thifensulfuron-methyl, thifensulfuron, ethoxysulfuron, oxasulfuron, ethametsulfuron, ethametsulfuron-methyl, iodosulfuron, iodosulfuron-methyl-sodium, sulfosulfuron, triasulfuron, tribenuron-methyl, tribenuron, tritosulfuron, foramsulfuron, trifloxysulfuron, trifloxysulfuron-sodium, mesosulfuron-methyl, mesosulfuron, orthosulfamuron, flucetosulfuron, amidosulfuron, TH-547, a compound disclosed in W02005092104; a triazolopyrimidinesulfonamide type such as flumetsulam, metosulam, diclosulam, cloransulam-methyl, florasulam or penoxsulam; an imidazolinone type such as imazapyr, imazapyr-isopropylammonium, imazethapyr, imazethapyr-ammonium, imazaquin, imazaquin-ammonium, imazamox, imazamox-ammonium, imazamethabenz, imazamethabenz-methyl or imazapic; a pyrimidinylsalicylic acid type such as pyrithiobac-sodium, bispyribac-sodium, pyrirriinobac-methyl, pyribenzoxim, pyriftalid, pyrimisulfan (KUH-021); a sulfonylaminocarbonyltriazolinone type such as flucarbazone, flucarbazone-sodium, propoxycarbazone-sodium or propoxycarbazone; and others such as glyphosate, glyphosate-sodium, glyphosate-potassium, glyphosate-ammonium, glyphosate-diammonium, glyphosate-isopropylammonium, glyphosate-trimesium, glyphosate-sesquisodium, glufosinate, glufosinate-ammonium, bilanafos, bilanafos-sodium or cinmethylin.
(8) Those which are believed to exhibit herbicidal effects by inhibiting cell mitoses of plants, such as a dinitroaniline type such as trifluralin, oryzalin, nitralin, pendimethalin, ethalfluralin, benfluralin, prodiamine, butralin or dinitramine; an amide type such as bensulide, napropamide, propyzamide or pronamide; an organic phosphorus type such as amiprofos-methyl, butamifos, anilofos or piperophos; a phenyl carbamate type such as propham, chlorpropham, barban or carbetamide; a cumylamine type such as daimuron, cumyluron, bromobutide or methyldymron; and others such as asulam, asulam-sodium, dithiopyr, thiazopyr, chlorthal-dimethyl, chlorthal or diphenamid.
(9) Those which are believed to exhibit herbicidal effects by inhibiting protein biosynthesis or lipid biosynthesis of plants, such as a chloroacetamide type such as alachlor, metazachlor, butachlor, pretilachlor, metolachlor, S-metolachlor, thenylchlor, pethoxamid, acetochlor, propachlor, dimethenamid, dimethenamid-P, propisochlor or dimethachlor; a thiocarbamate type such as molinate, dimepiperate, pyributicarb, EPTC, butylate, vernolate, pebulate, cycloate, prosulfocarb, esprocarb, thiobencarb, diallate, tri-allate or orbencarb; and others such as etobenzanid, mefenacet, flufenacet, tridiphane, cafenstrole, fentrazamide, oxaziclomefone, indanofan, benfuresate, pyroxasulfone (KIH-485), dalapon, dalapon-sodium, TCA-sodium or trichloroacetic acid.
(10) MSMA, DSMA, CMA, endothall, endothall-dipotassium, endothall-sodium, endothall-mono(N,N-dimethylalkylammonium), ethofumesate, sodium chlorate, pelargonic acid, nonanoic acid, fosamine, fosamine-ammonium, pinoxaden, HOK-201, aclolein, ammonium sulfamate, borax, chloroacetic acid, sodium chloroacete, cyanamide, methylarsonic acid, dimethylarsinic acid, sodium dimethylarsinate, dinoterb, dinoterb-ammonium, dinoterb-diolamine, dinoterb-acetate, DNOC, ferrous sulfate, flupropanate, flupropanate-sodium, isoxaben, mefluidide, mefluidide-diolamine, metam, metam-ammonium, metam-potassium, metam-sodium, methyl isothiocyanate, pentachlorophenol, sodium pentachlorophenoxide, pentachlorophenol laurate, quinoclamine, sulfuric acid, urea sulfate, etc.
(11) Those which are believed to exhibit herbicidal effects by being parasitic on plants, such as Xanthomonas campestris, Epicoccosirus nematosorus, Epicoccosirus nematosperus, Exserohilum monoseras or Drechsrela monoceras.

The mixing ratio of the compound represented by the formula (I) of the present invention to the above-mentioned active ingredient of another herbicide can not generally be defined, since it varies depending upon the weather conditions, the soil conditions, the type of the formulation, the types of weeds to be controlled, the application time, etc., but it is usually from 1:1,000 to 1,000:1, preferably from 1:800 to 100:1, more preferably from 1:600 to 20:1.

Now, examples of the preferred embodiments of the present invention will be given below, but it should be understood that the present invention is by no means restricted thereto.
(1) The herbicide containing a benzoylpyrazole compound represented by the formula (I) or its salt, as an active ingredient, wherein R⁴ is alkyl, and R⁵ is cyano; cyanoalkyl; haloalkoxyalkyl; amino(thiocarbonyl)alkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷; thiocyanatoalkyl; haloalkoxy; alkoxyalkoxy; alkylsulfonyl; alkylsulfonylalkyl; alkoxycarbonylalkyl; alkoxycarbonylalkoxy; heterocyclylalkyl; or aminoalkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷.
(2) The herbicide containing a benzoylpyrazole compound represented by the formula (I) or its salt, as an active ingredient, according to the above (1), wherein R¹ is alkyl, R² is a hydrogen atom, R³ is hydroxy, R⁴ is alkyl, R⁵ is alkoxyalkoxy, and R⁶ is alkylsulfonyl.
(3) The herbicide containing a benzoylpyrazole compound represented by the formula (I) or its salt, as an active ingredient, according to the above (2), wherein R¹ is methyl, ethyl or isopropyl, R² is a hydrogen atom, R³ is hydroxy, R⁴ is methyl, R⁵ is 2-methoxyethoxy, and R⁶ is methylsulfonyl.
(4) The herbicide containing a benzoylpyrazole compound represented by the formula (I) or its salt, as an active ingredient, according to the above (3), wherein R¹ is methyl.
(5) The herbicide containing a benzoylpyrazole compound represented by the formula (I) or its salt, as an active ingredient, according to the above (3), wherein R¹ is ethyl.
(6) The herbicide containing a benzoylpyrazole compound represented by the formula (I) or its salt, as an active ingredient, according to the above (3), wherein R¹ is isopropyl.
(7) A herbicidal composition comprising the benzoylpyrazole compound of the above formula (I) or its salt, and an agricultural adjuvant.
(8) A method for controlling undesired plants or inhibiting their growth, which comprises applying a herbicidally effective amount of the benzoylpyrazole compound of the formula (I) or its salt, to the undesired plants or to a place where they grow.
(9) The method according to the above (8), wherein undesired plants are controlled or their growth is inhibited in a cornfield.
(10) The method according to the above (9), wherein the corn is transformed one.
(11) The method according to the above (8), wherein undesired plants are controlled or their growth is inhibited in a wheat field.
(12) The method according to the above (8), wherein undesired plants are controlled or their growth is inhibited in a rice field.
(13) The method according to the above (8), wherein undesired plants are controlled or their growth is inhibited in a non-agricultural field.
(14) Use of a benzoylpyrazole compound of the above formula (I) or its salt, as an active ingredient of a selective herbicide in a cornfield.
(15) A process for producing a compound represented by the formula (I-a) which is included in the compound represented by the above formula (I): 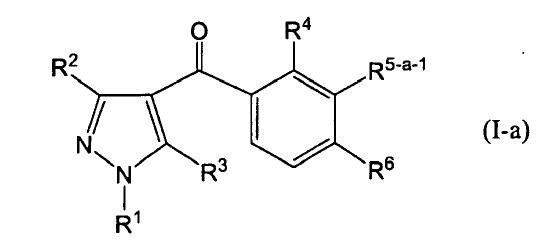 wherein R¹ is alkyl or cycloalkyl, R² is a hydrogen atom or alkyl, R³ is hydroxy, R⁴ is alkyl; haloalkyl; alkoxy; halogen; nitro; cyano; alkylthio; alkylsulfinyl; or alkylsulfonyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R⁶ is haloalkyl; halogen; nitro; cyano; alkylthio; alkylsulfinyl; or alkylsulfonyl, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, which comprises reacting a compound represented by the formula (I-b): 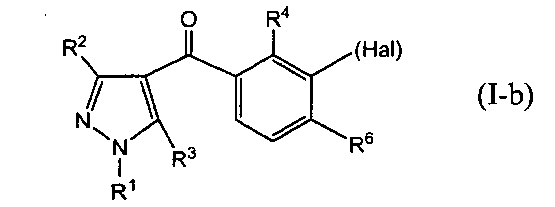 wherein R¹, R², R³, R⁴ and R⁶ are as defined above, and Hal is halogen, with a compound represented by the formula (VIII):

   HO-R^{α} (VIII)

   wherein R^{α} is alkyl; haloalkyl; alkoxyalkyl; haloalkoxyalkyl; alkoxyhaloalkyl; haloalkoxyhaloalkyl; heterocyclyl; heterocyclylalkyl; cycloalkyl; -C(O)SR⁷; -C(O)OR⁷; alkylthioalkyl; alkoxycarbonylalkyl; alkenyl; or alkynyl, and R⁷ is as defined above, and a base, or reacting the compound represented by the formula (I-b) with a metal salt of the compound represented by the formula (VIII).
(16) The process according to the above (15), wherein R¹ is alkyl, R² is a hydrogen atom, R⁴ is alkyl, R^{5-a-1} is alkoxyalkoxy, and R^{α} is alkoxyalkyl.
(17) The process according to the above (15), wherein R¹ is alkyl, R² is a hydrogen atom, R⁴ is alkyl, R^{5-a-1} is alkoxy, and R^{α} is alkyl.
(18) A process for producing a compound represented by the formula (I-a-Hal-3): 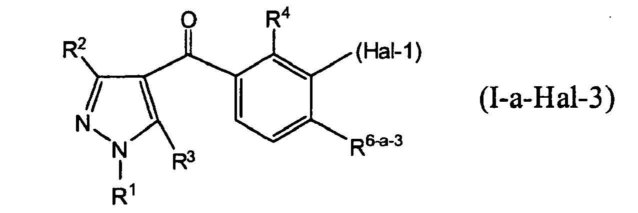 wherein R¹ is alkyl or cycloalkyl, R² is a hydrogen atom or alkyl, R³ is hydroxy, R⁴ is alkyl; haloalkyl; alkoxy; halogen; nitro; cyano; alkylthio; alkylsulfinyl; or alkylsulfonyl, R^{6-a-3} is alkylsulfonyl, and Hal-1 is halogen, which comprises reacting a compound represented by the formula (XXXIII): 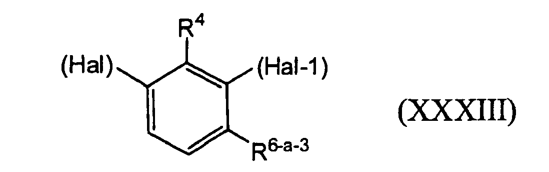 wherein R^{6-a-3} is as defined above, and each of Hal-1 and Hal, which may be the same or different from each other, is halogen, with a compound represented by the formula (III): 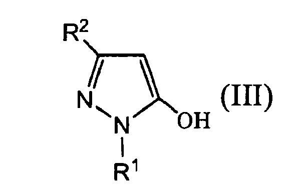 wherein R¹ and R² are as defined above, or its salt, and carbon monoxide or its equivalent, in the presence of a catalyst and a base.
(19) The process according to the above (18), wherein R¹ is alkyl, R² is a hydrogen atom, and R⁴ is alkyl.
(20) The process according to the above (18), wherein the catalyst is a metal catalyst.
(21) The process according to the above (20), wherein the metal catalyst is a palladium catalyst.
(22) A process for producing a compound represented by the formula (XXXIII): 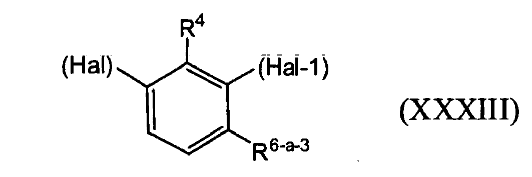 wherein R⁴ is alkyl; haloalkyl; alkoxy; halogen; nitro; cyano; alkylthio; alkylsulfinyl; or alkylsulfonyl, R^{6-a-3} is alkylsulfonyl, and each of Hal-1 and Hal which may be the same or different from each other, is halogen, which comprises reacting a compound represented by the formula (XXXIV): 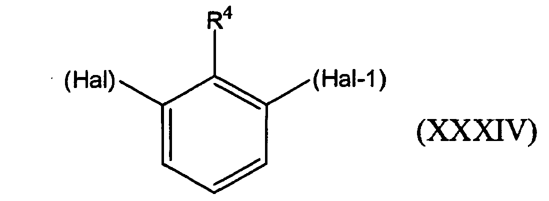 wherein R⁴, Hal-1 and Hal are as defined above, with a compound represented by the formula:

   (R^{6-a-3})(Hal-3) or (R^{6-a-3})₂O

   wherein R^{6-a-3} is as defined above, and Hal-3 is halogen, provided that Hal-1, Hal and Hal-3 may be the same or different from one another, in the presence of an acid.
(23) The process according to the above (22), wherein R⁴ is alkyl.
(24) A process for producing a compound represented by the formula (V-a-1-3): 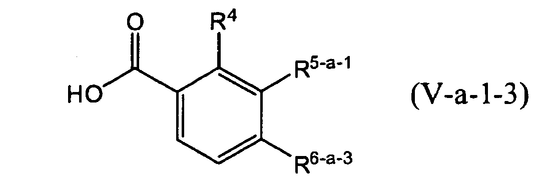 wherein R⁴ is alkyl; haloalkyl; alkoxy; halogen; nitro; cyano; alkylthio; alkylsulfinyl; or alkylsulfonyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R^{6-a-3} is alkylsulfonyl, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, which comprises reacting a compound represented by the formula (XXXII): 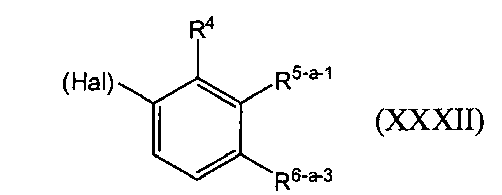 wherein R⁴, R^{5-a-1} and R^{6-a-3} are as defined above, and Hal is halogen, with carbon monoxide or its equivalent, and H₂O, in the presence of a catalyst and a base.
(25) The process according to the above (24), wherein R⁴ is alkyl, and R^{5-a-1} is alkoxyalkoxy.
(26) The process according to the above (24), wherein the catalyst is a metal catalyst.
(27) The process according to the above (26), wherein the metal catalyst is a palladium catalyst.
(28) A process for producing a compound represented by the formula (XXXII): 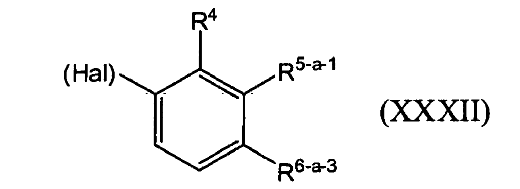 wherein R⁴ is alkyl; haloalkyl; alkoxy; halogen; nitro; cyano; alkylthio; alkylsulfinyl; or alkylsulfonyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R^{6-a-3} is alkylsulfonyl, Hal is halogen, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, which-comprises reacting a compound represented by the formula (XXXIII): 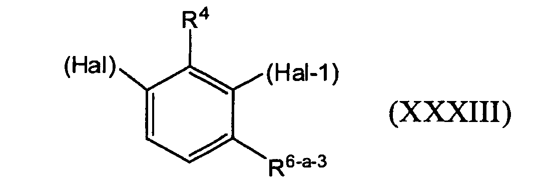 wherein R⁴ and R^{6-a-3} are as defined above, and each of Hal-1 and Hal, which may be the same or different from each other, is halogen, with a compound represented by the formula (VIII):

   HO-R^{α} (VIII)

   wherein R^{α} is alkyl; haloalkyl; alkoxyalkyl; haloalkoxyalkyl; alkoxyhaloalkyl; haloalkoxyhaloalkyl; heterocyclyl; heterocyclylalkyl; cycloalkyl; -C(O)SR⁷; -C(O)OR⁷; alkylthioalkyl; alkoxycarbonylalkyl; alkenyl; or alkynyl, and R⁷ is as defined above, in the presence of a base, or reacting the compound represented by the formula (XXXIII) with a metal salt of the compound represented by the formula (VIII).
(29) The process according to the above (28), wherein R⁴ is alkyl, R^{5-a-1} is alkoxyalkoxy, and R^{α} is alkoxyalkyl.
(30) The process according to the above (28), wherein R⁴ is alkyl, R^{5-a-1} is alkoxy, and R^{α} is alkyl.
(31) A compound represented by the formula (XXXII-a): 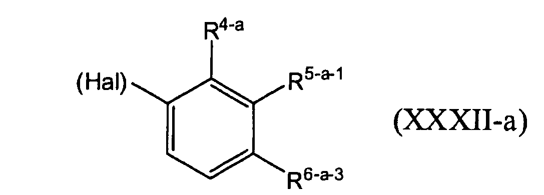 wherein R^{4-a} is alkyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R^{6-a-3} is alkylsulfonyl, Hal is halogen, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy.
(32) The compound according to the above (31), wherein R^{5-a-1} is alkoxyalkoxy.
(33) A process for producing a compound of the formula (XXVI): 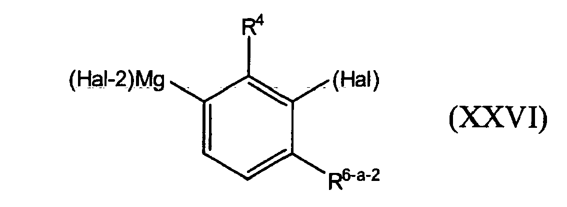 wherein R⁴ is alkyl; haloalkyl; alkoxy; halogen; nitro; cyano; alkylthio; alkylsulfinyl; or alkylsulfonyl, R^{6-a-2} is alkylthio, each of Hal and Hal-2 which may be the same or different from each other, is halogen, which comprises reacting a compound represented by the formula (XXVII): 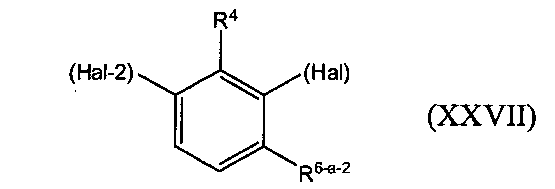 wherein R⁴, R^{6-a-2}, Hal and Hal-2 are as defined above, with magnesium metal or a Grignard reagent, in the presence of a solvent.
(34) The process according to the above (33), wherein R⁴ is alkyl.
(35) A process for producing a compound represented by the formula (XXVII): 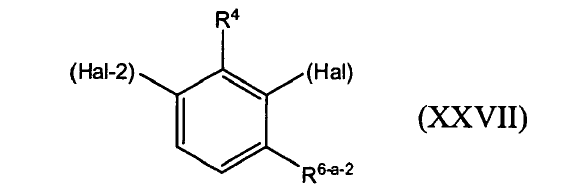 wherein R⁴ is alkyl; haloalkyl; alkoxy; halogen: nitro; cyano; alkylthio; alkylsulfinyl; or alkylsulfonyl, R^{6-a-2} is alkylthio, and Hal and Hal-2 which may be the same or different from each other, is halogen, which comprises reacting a compound represented by the formula (XXVIII): 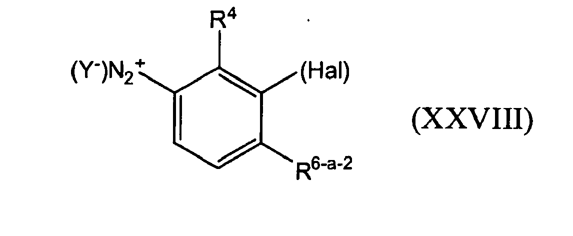 wherein R⁴, R^{6-a-2} and Hal are as defined above, and Y⁻ is Cl⁻, Br⁻, I⁻, BF₄⁻, HSO₃⁻, NO₃⁻, ClO₄⁻ or OH⁻, with a halogenating agent.
(36) The process according to the above (35), wherein R⁴ is alkyl.
(37) A process for producing a compound represented by the formula (XXVIII): 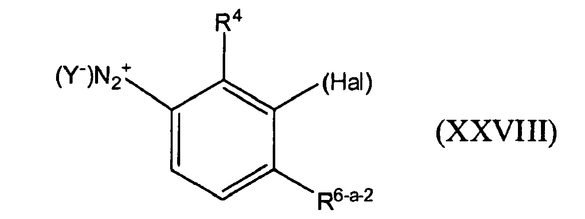 wherein R⁴ is alkyl; haloalkyl; alkoxy; halogen, nitro; cyano; alkylthio; alkylsulfinyl; or alkylsulfonyl, R^{6-a-2} is alkylthio, Hal is halogen, and Y is Cl⁻, Br⁻, I⁻, BF₄⁻, HSO₃⁻, NO₃⁻, ClO₄⁻ or OH⁻, which comprises reacting a compound represented by the formula (XXIX): 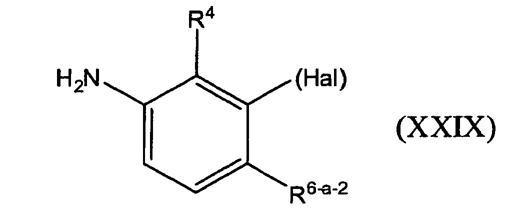 wherein R⁴, R^{6-a-2} and Hal are as defined above, with a diazotizing agent.
(38) The process according to the above (37), wherein R⁴ is alkyl.
(39) A process for producing a compound represented by the formula (XXIX): 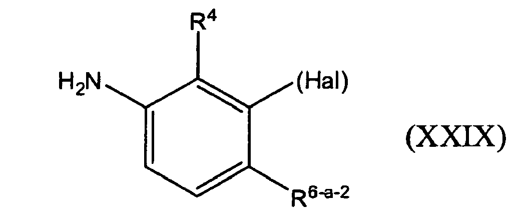 wherein R⁴ is alkyl; haloalkyl; alkoxy; halogen, nitro; cyano; alkylthio; alkylsulfinyl; or alkylsulfonyl, R^{6-a-2} is alkylthio, Hal is halogen, which comprises reacting a compound represented by the formula (XXX): 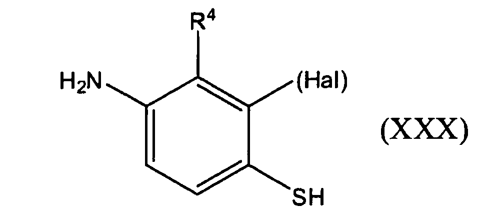 wherein R⁴ is as defined above, with an alkylating agent in the presence of a base.
(40) The process according to the above (39), wherein R⁴ is alkyl.
(41) A compound represented by the formula (XXVII): 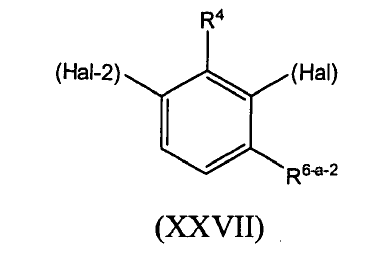 wherein R⁴ is alkyl; haloalkyl; alkoxy; halogen, nitro; cyano; alkylthio; alkylsulfinyl; or alkylsulfonyl, R^{6-a-2} is alkylthio, and each of Hal and Hal-2 which are the same or different, is halogen.
(42) The compound according to the above (41), wherein R⁴ is alkyl.
(43) A compound represented by the above formula (XXIX): 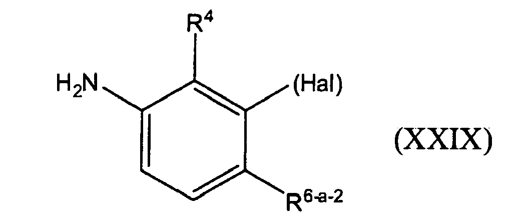 wherein R⁴ is alkyl; haloalkyl; alkoxy; halogen, nitro; cyano; alkylthio; alkylsulfinyl; or alkylsulfonyl, R^{6-a-2} is alkylthio, and Hal is halogen.
(44) The compound according to the above (43), wherein R⁴ is alkyl.

### EXAMPLES

Now, the present invention will be described with reference to Examples, but it should be understood that the present invention is by no means restricted thereto. Preparation Examples for the compounds of the above formula (I) will be described below.

### PREPARATION EXAMPLE 1

### Preparation of 5-hydroxy-1-methylpyrazol-4-yl 3-(2,2-dimethoxyethoxy)-2-methyl-4-(methylsulfonyl)phenyl ketone (after-mentioned Compound No. 1)

(1) 3-Hydroxy-2-methyl-4-(methylsulfonyl) benzoic acid (300 mg) was dissolved in N,N-dimethylformamide (10 mL), and potassium carbonate (360 mg) and bromoacetoaldehyde dimethylacetal (660 mg) were added at room temperature. The reaction mixture was heated to 80°C and stirred for 32 hours. The reaction mixture was cooled to room temperature, and 100 mL of water and a 0.5N sodium hydroxide aqueous solution (10 mL) were added. Then, the mixture was extracted with ethyl acetate (200 mL), and the organic layer was dried over anhydrous sodium sulfate. The solvent was distilled off under reduced pressure to obtain oily 2,2-dimethoxyethyl 3-(2,2-dimethoxyethoxy)-2-methyl-4-(methylsulfonyl)benzoate.
   ¹H-NMR 400MHz(CDCl₃ δppm): 2.54(s,3H), 3.31 (s,1H), 3.39(s,6H), 3.44(s,6H), 4.06(d,2H,J=5.4Hz), 4.31 (d,2H,J=5.4Hz), 4.73(t,1H,J=5.4Hz), 4.87(t,1H,J=5.4Hz), 7.76(d,1H,J=8.4Hz), 7.82(d,1H,J=8.4Hz).
(2) 2,2-Dimethoxyethyl 3-(2,2-dimethoxyethoxy)-2-methyl-4-(methylsulfonyl)benzoate obtained in the above step (1) was dissolved in methanol (20 mL), and a 20 wt% sodium hydroxide aqueous solution (2 mL) was added at room temperature. After stirring for 30 minutes, 100 mL of 1 N hydrochloric acid was added to the reaction solution, and the mixture was extracted with ethyl acetate (200 mL). The organic layer was washed twice with a saturated sodium chloride aqueous solution and dried over anhydrous sodium sulfate. The solvent was distilled off under reduced pressure to obtain 3-(2,2-dimethoxyethoxy)-2-methyl-4-(methylsulfonyl)benzoic acid (390 mg) as white solid.
   ¹H-NMR 400MHz(acetone-d₆ δppm): 2.56(s,3H), 3.31 (s,3H), 3.44(s,6H), 4.06(d,2H,J=5.2Hz), 4.88(t,1H,J=5.2Hz), 7.82(br s,2H).
(3) 3-(2,2-Dimethoxyethoxy)-2-methyl-4-(methylsulfonyl)benzoic acid (390 mg) was dissolved in chloroform (15 mL), and oxalyl chloride (0.5 mL) and a catalytic amount of DMF were added. The reaction mixture was stirred at room temperature for 30 minutes, and the solvent was distilled off under reduced pressure. The residue was dissolved in anhydrous THF (20 mL), and 5-hydroxy-1-methylpyrazole (132 mg), triethylamine (250 mg) and N,N-dimethylaminopyridine (300 mg) were added, followed by refluxing under heating for 1 hour. The reaction mixture was cooled to room temperature, and ethyl acetate (200 mL) was added, and the mixture was washed twice with a saturated sodium chloride aqueous solution. The organic layer was dried over anhydrous sodium sulfate, and then, the solvent was distilled off under reduced pressure. The residue was dissolved in anhydrous acetonitrile (10 mL), and triethylamine (250 mg) and a catalytic amount of acetone cyanohydrin were added, followed by stirring at room temperature for 12 hours. The solvent was distilled off under reduced pressure to obtain crude 5-hydroxy-1-methylpyrazol-4-yl 3-(2,2-dimethoxyethoxy)-2-methyl-4-(methylsulfonyl)phenyl ketone.
   ¹H-NMR (400MHz CDCl₃ δppm): 2.38(s,3H), 3.29(s,3H), 3.47(s,6H), 3.70(s,3H), 4.09(d,2H,J=5.2Hz), 4.1 (br s,1H), 4.83(t,1H,J=5.2Hz), 7.32(s,1H), 7.35(d,1H,J=8.4Hz), 7.91(d,1H,J=8.4Hz).
(4) The crude 5-hydroxy-1-methylpyrazol-4-yl 3-(2,2-dimethoxyethoxy)-2-methyl-4-(methylsulfonyl)phenyl ketone obtained in the above step (3), was dissolved in anhydrous THF (10 mL), and triethylamine (250 mg) and chlorothiolformic acid S-ethyl having a purity of 96% (200 mg) was added, followed by stirring at room temperature for 1 hour. Ethyl acetate (200 mL) was added to the obtained reaction mixture, and the mixture was washed twice with a saturated sodium chloride aqueous solution. The organic layer was dried over anhydrous sodium sulfate, and the solvent was distilled off under reduced pressure. The residue was purified by silica gel column chromatography (n-hexane:ethyl acetate=1:1) to obtain 5-(ethylthio)carbonyloxy-1-methylpyrazol-4-yl 3-(2,2-dimethoxyethoxy)-2-methyl-4-(methylsulfonyl)phenyl ketone (150 mg).
(5) 5-(Ethylthio)carbonyloxy-1-methylpyrazol-4-yl 3-(2,2-dimethoxyethoxy)-2-methyl-4-(methylsulfonyl)phenyl ketone (91 mg) obtained in the above step (4) was dissolved in methanol (10 ml), and a 0.5N sodium hydroxide aqueous solution (3 ml) was added at room temperature. The reaction solution was stirred at room temperature for 1 hour, and ethyl acetate (200 ml) was added, followed by washing once with a saturated sodium chloride aqueous solution. The organic layer was dried over anhydrous sodium sulfate, and the solvent was distilled off under reduced pressure to obtain the desired product (80 mg).

### PREPARATION EXAMPLE 2

### Preparation of 5-hydroxy-1-methylpyrazol-4-yl 3-(2-methoxyethoxy)-2-methyl-4-(methylsulfonyl)phenyl ketone (after-mentioned Compound No. 151)

(1) Methyl 3-hydroxy-2-methyl-4-nitrobenzoate (63.3 g) was dissolved in DMF (400 mL), and potassium carbonate (41.4 g) and potassium iodide (3 g) were added, followed by stirring. To this mixed liquid, chloroethyl methyl ether (56.7 g) was added, followed by stirring for 20 hours under heating at 90°C. The reaction mixture was added to an aqueous sodium chloride solution, and the mixture was extracted with a mixed solvent of ethyl acetate/n-hexane. The organic layer was washed with a saturated sodium chloride aqueous solution, and the organic layer was dried over anhydrous sodium sulfate. The solvent was distilled off under reduced pressure to obtain methyl 3-(2-methoxyethoxy)-2-methyl-4-nitrobenzoate (80.0 g).
   ¹H-NMR (400MHz,CDCl₃,δppm)2.54(3H,s), 3.39(3H,s), 3.70(2H,m), 3.91(3H,m),4.11(2H,m). 7.59(1H,d,J=8.0Hz), 7.64(1H,d,J=8.0Hz).
(2) Methyl 3-(2-methoxyethoxy)-2-methyl-4-nitrobenzoate (50 g) was dissolved in DMF (320 mL), and sodium thiomethoxide (95 wt% product, 14.4 g) was added under cooling with water. After stirring for 30 minutes, to the reaction liquid, a mixed solvent of ethyl acetate/n-hexane (300 mL/100 mL) and then 1 N hydrochloric acid (300 mL) were added, followed by stirring. The organic layer was washed with an aqueous sodium chloride solution and then dried over anhydrous sodium sulfate. The solvent was distilled off under reduced pressure to obtain methyl 3-(2-methoxyethoxy)-2-methyl-4-(methylthio)benzoate (47.7 g) as solid.
   ¹H-NMR (400MHz,CDCl₃,δppm); 2.24(3H,s), 2.53(3H,s), 3.48(3H,s), 3.78(2H,m), 3.86(3H,s), 4.01 (2H,m), 6.96(1H,d,J=8.4Hz), 7.68(1H,d,J=8.4Hz).
(3) Methyl 3-(2-methoxyethoxy)-2-methyl-4-(methylthio)benzoate (47.7 g) was dissolved in acetic acid (350 mL), and sodium tungstate(IV) dihydrate (4.6 g) and then a 30% hydrogen peroxide aqueous solution (60.0 g) were gradually dropwise added at room temperature. After stirring for 1 hour, the mixture was heated to 60°C and stirred for 40 minutes. An aqueous sodium chloride solution was added to the reaction mixture, and the mixture was extracted with a mixed solvent of a ethyl acetate/n-hexane (1:1). The organic layer was washed with a saturated sodium chloride aqueous solution and dried over anhydrous sodium sulfate. The solvent was distilled off under reduced pressure, followed by recrystallization in methanol to obtain methyl 3-(2-methoxyethoxy)-2-methyl-4-(methylsulfonyl)benzoate (46.8 g).
   ¹H-NMR (400MHz,CDCl₃,δppm); 2.54(3H,s), 3.26(3H,s), 3.46(3H,s), 3.79(2H,m), 3.91(3H,s), 4.19(2H,m), 7.71 (1H,d,J=8.4Hz), 7.84(1H,d,J=8.4Hz).
(4) Methyl 3-(2-methoxyethoxy)-2-methyl-4-(methylsulfonyl)benzoate (46.79 g) was dissolved in a mixed solvent of methanol/DMSO (280 mL/100 mL), and a 20 wt% sodium hydroxide aqueous solution (77.5 g) was added at room temperature. After stirring overnight, an aqueous sodium chloride solution was added to the reaction solution, and the solution was alkalized with an aqueous potassium carbonate solution, followed by washing with ether. The aqueous layer was acidified with dilute hydrochloric acid and extracted with ethyl acetate. The organic layer was washed with a saturated sodium chloride aqueous solution and dried over anhydrous sodium sulfate. The solvent was distilled off under reduced pressure to obtain 3-(2-methoxyethoxy)-2-methyl-4-(methylsulfonyl)benzoic acid (30.4 g).
   ¹H-NMR (400MHz,CDCl₃,δppm); 2.63(3H,s), 3.31(3H,s), 3.49(3H,s), 3.83(2H,m), 4.23(2H,m), 7.91 (2H,s).
(5) 3-(2-Methoxyethoxy)-2-methyl-4-(methylsulfonyl)benzoic acid (16.98 g) was dissolved in chloroform (200 mL), and oxalyl chloride (15 g) and a catalytic amount of DMF were added. The reaction mixture was stirred at room temperature for 1 hour, and the solvent was distilled off under reduced pressure. The residue was dissolved in anhydrous tetrahydrofuran (150 mL), and 5-hydroxy-1-methylpyrazole (6.35 g), triethylamine (8.92 g) and 4-(N,N-dimethylamino)pyridine (10.8 g) were added, followed by stirring at room temperature for 17 hours. The reaction mixture was put into water and acidified with hydrochloric acid, and then, the product was extracted with ethyl acetate. The ethyl acetate solution was washed twice with a saturated sodium chloride aqueous solution. The organic layer was dried over anhydrous sodium sulfate, and then, the solvent was distilled off under reduced pressure. The residue was dissolved in anhydrous acetonitrile (200 mL), and triethylamine (8.1 g) and a catalytic amount (0.75 g) of acetone cyanohydrin were added, followed by stirring at room temperature for 3 hours. The solvent was distilled off under reduced pressure, and the residue was put in water and acidified with dilute hydrochloric acid, whereupon the product was extracted with ethyl acetate and dried over anhydrous sodium sulfate. Then, the solvent was distilled off under reduced pressure to obtain the desired product (16.8 g) with a melting point of 112-114°C.
   ¹H-NMR (40OMHz,CDCl₃,δppm); 2.41 (3H,s), 3.31 (3H,s), 3.48(3H,s), 3.73(3H,s), 3.82(2H,m), 4.26(2H,m), 7.34(1H,s), 7.36(1H,d,J=8.4Hz), 7.94(1H,d,J=8.4Hz).

### PREPARATION EXAMPLE 3

### Preparation of 5-hydroxy-1-methylpyrazol-4-yl 3-(2-methoxyethoxy)-2-methyl-4-(methylsulfonyl)phenyl ketone

(1) Thionyl chloride (120 g) and methanesulfonic acid (240 g) were mixed at room temperature and refluxed at 120°C for 1 hour with stirring. After confirming termination of the generation of gas, the reaction mixture was cooled to 50°C. 1,3-Dichloro-2-methylbenzene (81 g) and trifluoromethanesulfonic acid (5 g) were added thereto, and the mixture was refluxed at 120°C for 5 hours. The reaction mixture was cooled to 50°C and put into 1 L of ice water. Precipitated crude crystals were dissolved in chloroform (1 L), and washed once with each of water and a 2M sodium hydroxide aqueous solution. The organic layer was dried over anhydrous sodium sulfate, and then, the solvent was distilled off under reduced pressure. Obtained crude crystals were recrystallized from ethanol to obtain 1,3-dichloro-2-methyl-4-(methylsulfonyl)benzene (88 g) as white crystals.
   ¹H-NMR 400MHz(CDCl₃ δppm): 2.58(s,3H), 3.28(s,3H), 7.50(d,1H,J=8.2Hz), 7.97(d,1H,J=8.2Hz).
(2) 1,3-Dichloro-2-methyl-4-(methylsulfonyl)benzene (31 g), sodium bicarbonate (33 g), palladium acetate (0.875 g) and 1,4-bis(diphenylphosphino)butane (3.25 g) were charged into a stainless steel autoclave having a capacity of 500 mL, and a mixed solvent (t-butanol:water=10:1, 250 mL) deaerated with nitrogen gas, was added. The container was closed, and the interior was replaced twice with carbon monoxide, and then, the pressure of carbon monoxide was set to be 2 MPa, followed by stirring at 125°C for 8 hours, whereupon the mixture was cooled to room temperature. Next day, the mixture was likewise stirred at 125°C for 8 hours and then cooled to room temperature, and one day after, the mixture was likewise stirred at 125°C for 4 hours. The reaction container was cooled, and a 2M sodium hydroxide solution (200 mL) was added. After stirring for 15 minutes, insolubles were filtered off by celite, and the residue was washed with a 2M sodium hydroxide solution (200 mL). The filtrate was washed three times with toluene (200 mL), and concentrated hydrochloric acid was added to the aqueous layer until the pH became 1. Precipitated crystals were collected by filtration and dried to obtain 3-chloro-2-methyl-4-(methylsulfonyl)benzoic acid (30 g) as white solid.
   ¹H-NMR 400MHz(CDCl₃ δppm): 2.72(s,3H), 3.30(s,3H), 7.97(d,1H,J=8.4Hz),8.11(d,1H,J=8.4Hz).
(3) In a nitrogen atmosphere, sodium hydroxide (0.8 g) was added to 2-methoxyethanol (30 mL) at room temperature, followed by stirring at 120°C for 15 minutes until the solid was completely dissolved. The reaction mixture was cooled to room temperature, and about 15 mL of the solvent was distilled off under reduced pressure. 3-Chloro-2-methyl-4-(methylsulfonyl)benzoic acid (1 g) was added thereto, followed by stirring at 120°C for 12 hours. Then, the reaction solution was cooled to room temperature, and concentrated hydrochloric acid was added until the pH became 1. Extraction with ethyl acetate (100 mL) was carried out twice, and the combined organic layer was dried over anhydrous sodium sulfate, and then, the solvent was distilled off under reduced pressure. The residue was dissolved in ethyl acetate (20 mL), and hexane (5 mL) was added thereto. This solution was left to stand still for 12 hours in a refrigerator (about 5°C) to obtain 3-(2-methoxyethoxy)-2-methyl-4-methylsulfonyl)benzoic acid (0.92 g) as slightly brown solid.
   ¹H-NMR 400MHz(CDCl₃, δppm): 2.63(3H,s), 3.31 (3H,s), 3.49(3H,s), 3.83(2H,m), 4.23(2H,m), 7.91 (2H,s).
(4) Using 3-(2-methoxyethoxy)-2-methyl-4-(methylsulfonyl) benzoic acid, the desired product is obtained in the same manner as in the above Preparation Example 2(5).

### PREPARATION EXAMPLE 4

### Preparation of 5-hydroxy-1-methylpyrazol-4-yl 3-(2-methoxyethoxy)-2-methyl-4-(methylsulfonyl)phenyl ketone

(1) 3-Chloro-2-methyl-4-(methylsulfonyl)benzoic acid (40 g) and chloroform (400 mL) were mixed at room temperature, and further, oxalyl chloride (28.1 mL) and DMF (three drops) were added. After stirring for about 4 hours until generation of gas terminated, stirring was continued at 40°C for 30 minutes. The solvent and excess reagents were distilled off under reduced pressure, and to the residue, chloroform (400 mL), 5-hydroxy-1-methyl-1H-pyrazole (16.6 g) and triethylamine (17.9 g) were added, followed by stirring at room temperature for 5 hours. The reaction solution was washed with dilute hydrochloric acid (100 mL), and the aqueous layer was extracted with chloroform (100 mL). The organic layers were put together and dried over anhydrous sodium sulfate, and then, the solvent was distilled off under reduced pressure. The residue was dissolved in anhydrous acetonitrile (400 mL), and then, 5-hydroxy-1-methyl-1H-pyrazole (1.58 g), acetone cyanohydrin (1.37 g) and triethylamine (16.3 g) were added, followed by stirring at 40°C for 12 hours. The reaction solution was concentrated under reduced pressure, and toluene (200 mL) was added, followed by extraction with a 1N sodium hydroxide aqueous solution (200 mL). To the aqueous layer, concentrated hydrochloric acid was added until the pH became 1, and formed solid was collected by filtration. The solid obtained by filtration was washed with toluene and ethyl acetate and then dried to obtain 5-hydroxy-1-methylpyrazol-4-yl 3-chloro-2-methyl-4-(methylsulfonyl)phenyl ketone (42 g) as slightly yellow solid.
   ¹H-NMR 400MHz(DMSO-d₆ δppm): 2.32(s,3H), 3.40(s,3H), 3.53(s,3H), 7.35(s,1H), 7.51 (d,1H,J=8.OHz), 7.97(d,1H,J=8.0Hz).
(2) In a nitrogen atmosphere, sodium hydroxide (0.6 g) was added to 2-methoxyethanol (30 mL) and stirred at 100°C for 15 minutes until the solid was completely dissolved. The reaction solution was cooled to room temperature, and then, about 15 mL of the solvent was distilled off under reduced pressure. 5-Hydroxy-1-methylpyrazol-4-yl 3-chloro-2-methyl-4-(methylsulfonyl)phenyl ketone (1 g) was added thereto, followed by stirring at 85°C for 2 hours and further by stirring at 120°C for 3 hours. A 1M sodium hydroxide aqueous solution (150 mL) was added, and the mixture was washed with diethyl ether (150 mL). To the aqueous layer, concentrated hydrochloric acid was added until the pH became 1, followed by extraction twice with ethyl acetate (100 mL). The combined organic layer was dried over anhydrous sodium sulfate, and then the solvent was distilled off under reduced pressure to obtain an oily crude product. This product was dissolved in ethyl acetate (20 mL), and then, hexane (5 mL) was added at room temperature, and the mixture was left to stand for 12 hours to obtain the desired product (0.84 g) as slightly brown crystals.

### PREPARATION EXAMPLE 5

### Preparation of 5-hydroxy-1-methylpyrazol-4-yl (3-(2-methoxyethoxy)-2-methyl-4-(methylsulfonyl)phenyl ketone

(1) Sodium sulfide nonahydrate (18 g) was dissolved in water (50 mL), and a mixture having 3-chloro-2-methyl-4-thioisocyanate aniline (13 g) suspended in ethanol (250 mL), was dropwise added thereto at room temperature, followed by stirring for 2 hours. The reactor was put in an ice bath, and methyl iodide (13 g) was slowly added, followed by stirring for 10 minutes. Then, the mixture was further stirred at room temperature for 3 hours. The solvent was distilled off under reduced pressure, and a saturated sodium chloride aqueous solution (150 mL) was added, followed by extraction twice with chloroform (100 mL). The combined organic layer was dried over anhydrous sodium sulfate, and then, the solvent was distilled off under reduced pressure to obtain 3-chloro-2-methyl-4-(methylthio)aniline (13 g).
   ¹H-NMR 400MHz(CDCl₃ δppm): 2.25(s,3H), 2.40(s,3H), 6.60(d,1H,J=8.4Hz), 7.01 (d,1H,J=8.4Hz).
(2) 3-Chloro-2-methyl-4-(methylthio)aniline (0.94 g) and sodium nitrite (0.69 g) were dissolved in DMSO (25 mL), followed by heating to 35°C, and then a mixture comprising a 47 wt% hydrobromic acid (2.35 mL) and DMSO (25 mL) was dropwise added. After stirring at the same temperature for 20 hours, the mixture was put into a solution having potassium carbonate (5 g) dissolved in ice water (100 mL), followed by extraction twice with ether (50 mL). The combined organic layer was washed with water and dried over anhydrous sodium sulfate. The concentrated reaction mixture was isolated by silica gel column chromatography to obtain (4-bromo-2-chloro-3-methylphenyl)(methyl)sulfide (0.61 g).
   ¹H-NMR 400MHz(CDCl₃ δppm): 2.45(s,3H), 2.53(s,3H), 6.85(d,1H,J=8.4Hz), 7.43(d,1H,J=8.4Hz).
(3) Into a container equipped with a reflux condenser and kept in a nitrogen atmosphere, magnesium metal (0.58 g) and anhydrous THF (10 mL) were introduced, and isopropyl bromide (0.70 mL) was slowly dropwise added so that the temperature would not rise too much. Then, the mixture was stirred at room temperature for 25 minutes, and an anhydrous THF solution (15 mL) of (4-bromo-2-chloro-3-methylphenyl)(methyl)sulfide (3.7 g) was dropwise added over a period of 20 minutes, followed by further stirring at room temperature for 3 hours. The reaction container was placed in an ice bath and stirred while carbon dioxide gas was blown thereinto for 1 hour. Then, after returning the temperature to room temperature, 20 wt% hydrochloric acid (12 mL) was added, followed by stirring for further 30 minutes. To the reaction mixture, chloroform (40 mL) was added, followed by extraction three times. The combined organic layer was washed with water, and subjected to alkali extraction with a 20 wt% sodium hydroxide aqueous solution (50 mL), and then the pH was adjusted to 1 with 35 wt% hydrochloric acid, followed by extraction three times with ethyl acetate (50 mL). The combined organic layer was washed with water and a saturated sodium chloride aqueous solution and dried over anhydrous sodium sulfate, and then, the solvent was distilled off to obtain 3-chloro-2-methyl-4-(methylthio)benzoic acid (2.1 g).
   ¹H-NMR 400MHz(CDCl₃ δppm): 2.51 (s,3H), 2.72(s,3H), 7.03(d,1H,J=8.4Hz), 7.93(d,1H,J=8.4Hz).
(4) Using 3-chloro-2-methyl-4-(methylthio)-benzoic acid, 3-chloro-2-methyl-4-(methylsulfonyl)-benzoic acid is obtained in the same manner as in the above reaction (AI).
(5) Using 3-chloro-2-methyl-4-(methylsulfonyl)-benzoic acid, the desired product is obtained in the same manner as in the above Preparation Example 4(1) and (2).

### PREPARATION EXAMPLE 6

### Preparation of 5-hydroxy-1-methylpyrazol-4-yl 3-(2-methoxyethoxy)-2-methyl-4-(methylsulfonyl)phenyl ketone

(1) In a 1L flask, 2,6-dichlorotoluene (100 g), methanesulfonyl chloride (71 g) and anhydrous aluminum chloride (83 g) were put and stirred for 12 hours while heating at 120°C by an oil bath. While cooling in an ice bath, 2N hydrochloric acid (400 mL) was slowly added. The mixture was stirred for 1 hour at room temperature, and then, hexane (300 mL) was added, followed by stirring for further 2 hours at room temperature. Obtained white crystals were collected by filtration, washed with water (100 mL) and hexane (100 mL) and dried to obtain 1,3-dichloro-2-methyl-4-(methylsulfonyl)benzene (90 g) as white crystals (melting point: 125-127°C).
   ¹H-NMR 400MHz(CDCl₃, δppm): 2.55(3H,s), 3.25(3H,s), 7.47(1H,d,J=8.4Hz), 7.94(1H,d,d=8.4Hz).
(2) 2-Methoxyethanol (1.15 g) was slowly added at room temperature to a suspension having a 60 wt% sodium hydride paraffin dispersion (550 mg) added to anhydrous THF (50 mL). After stirring for 30 minutes, 1,3-dichloro-2-methyl-4-(methylsulfonyl)benzene (3 g) was added, and the mixture was stirred at 50°C for 2 hours. The reaction mixture was cooled to room temperature, and ethyl acetate (250 mL) was added. The mixture was transferred to a separating funnel and washed twice with a saturated sodium chloride aqueous solution. Then, the organic layer was dried over anhydrous sodium sulfate, and the solvent was distilled off under reduced pressure to obtain 1-chloro-3-(2-methoxyethoxy)-2-methyl-4-(methylsulfonyl)benzene (3.4 g) as white solid (melting point: 83-85°C).
   ¹H-NMR 400MHz(CDCl₃, δppm): 2.39(3H,s), 3.23(3H,s), 3.46(3H,s), 3.77(2H,m), 4.18(2H,m), 7.30(1H,d,J=8.8Hz), 7.73(1H,d,J=8.8Hz).
(3) 1-Chloro-3-(2-methoxyethoxy)-2-methyl-4-(methylsulfonyl)benzene (2 g), a 5 wt% palladium carbon (20 mg), 1,4-bis(diphenylphosphino)butane (16 mg) and sodium carbonate (1.1 g) were introduced into an autoclave, and isopropanol (8.5 mL) and water (1.5 mL) were added. The interior was substituted with carbon monoxide, followed by stirring at 190°C under a pressure of 2 MPa for 18 hours. An excess gas was removed, and to the reaction mixture, a 1N sodium hydroxide aqueous solution (10 mL) and t-butyl methyl ether (10 mL) were added. After stirring vigorously for 5 minutes, insolubles were filtered off by celite. The filtrate was transferred to a separating funnel, and t-butyl methyl ether (200 mL) was added, followed by extraction with a 1N sodium hydroxide aqueous solution (100 mL × 2). The aqueous layer was acidified with 2N hydrochloric acid (150 mL), followed by extraction with ethyl acetate (150 mL × 2). The organic layer was dried over anhydrous sodium sulfate, and the solvent was distilled off under reduced pressure to obtain 3-(2-methoxyethoxy)-2-methyl-4-(methylsulfonyl)benzoic acid (1.93 g) as white crystals (melting point: 104-106°C).
(4) Using 3-(2-methoxyethoxy)-2-methyl-4-(methylsulfonyl) benzoic acid, the desired product is obtained in the same manner as in the above Preparation Example 2(5).

### PREPARATION EXAMPLE 7

### Preparation of 5-hydroxy-1-methylpyrazol-4-yl 3-(2-methoxyethoxy)-2-methyl-4-(methylsulfonyl)phenyl ketone

(1) 1,3-Dichloro-2-methyl-4-(methylsulfonyl)benzene (1 g) and 5-hydroxy-1-methylpyrazole (820 mg) were dissolved in 1,4-dioxane (20 mL), and palladium acetate (50 mg), triethylamine (630 mg), potassium carbonate (1.15 g) and 1,1'-bis(diphenylphosphino)ferrocene (230 mg) were added thereto. This mixture was put into an autoclave, and the interior was substituted by carbon monoxide, followed by stirring at 130°C under a pressure of 2 MPa for 12 hours. After cooling to room temperature, an excessive gas was removed, and to the reaction solution, a 1N sodium hydroxide aqueous solution (10 mL) and diethyl ether (10 mL) were added. After stirring vigorously for 5 minutes, insolubles were filtered off by celite, and the filtrate was transferred to a separating funnel. Diethyl ether (200 mL) was added, followed by extraction with a 1 N sodium hydroxide aqueous solution (100 mL × 2). The combined aqueous layer was acidified with 2N hydrochloric acid (150 mL) and extracted with ethyl acetate (150 mL x 2). The organic layer was dried over anhydrous sodium sulfate, and then the solvent was distilled off under reduced pressure to obtain (3-chloro-2-methyl-4-(methylsulfonyl)phenyl)(5-hydroxy-1-methyl-1H-pyrazol-4-yl)ketone (620 mg).
(2) Using (3-chloro-2-methyl-4-(methylsulfonyl)phenyl)(5-hydroxy-1-methyl-1H-pyrazol-4-yl)ketone, the desired product is obtained in the same manner as in the above Preparation Example 4(2).

### PREPARATION EXAMPLE 8

### Preparation of 5-hydroxy-1-methylpyrazol-4-yl 3-(2-methoxyethoxy)-2-methyl-4-(methylsulfonyl)phenyl ketone

(1) Into an autoclave having an internal capacity of 50 mL, anhydrous THF (18 mL) and a 60 wt% sodium hydride/paraffin dispersion (316 mg) were charged, and 5-hydroxy-1-methylpyrazole (739 mg) was gradually added in a nitrogen atmosphere with stirring under cooling with ice. After stirring for 1 hour under cooling, 1-chloro-3-(2-methoxyethoxy)-2-methyl-4-(methylsulfonyl)benzene (2.0 g), 1,4-bis(diphenylphosphino)butane (100 mg) and a 53 wt% water-containing product (100 mg) of 10 wt% palladium carbon were added and pressurized to 2 MPa with carbon monoxide, then heated to 190°C and stirred for 20 hours. After completion of the reaction, the mixture was cooled to room temperature and put into a mixture comprising water (200 mL) and an organic solvent (diethyl ether 30 mL and ethyl acetate 70 mL), followed by stirring. The aqueous layer was acidified to about pH 3 with dilute hydrochloric acid, and the product was extracted with ethyl acetate and dried over sodium sulfate, and the solvent was distilled off under reduced pressure to obtain the desired product (1.6 g).

Now, typical examples of the compounds of the above formula (I) and their peripheral compounds are shown in Table 1, and their ¹H-NMR spectrum data are shown in Table 2. These compounds may be prepared in accordance with the above Preparation Examples or the above-described various production processes. In Tables 1 and 2, No. represents Compound No. Further, in Table 1, Me represents a methyl group, Et an ethyl group, n-Pr a n-propyl group, i-Pr an isopropyl group, t-Bu a tertiary butyl group.

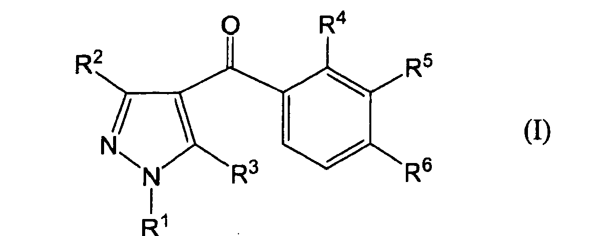

**TABLE 1**

| No. | R¹ | R² | R³ | R⁴ | R⁵ | R⁶ |
|---|---|---|---|---|---|---|
| 1 | Me | H | OH | Me | OCH₂CH(OCH₃) ₂ | SO₂Me |
| 2 | Me | H | OH | Me | CH₂SCN | SO₂Me |
| 3 | Me | H | OH | Me | CH₂C(S)NH₂ | SO₂Me |
| 4 | Me | H | OH | Me | OCH₂CH₂SMe | SO₂Me |
| 5 | Me | H | OH | Me | OC(O)SMe | SO₂Me |
| 6 | Me | H | OH | Me | OC(O)SEt | SO₂Me |
| 7 | Me | H | OH | Cl | OCH₂CHFOCF₃ | SO₂Me |
| 8 | Et | H | OH | Cl | OCH₂CHFOCF₃ | SO₂Me |
| 9 | Me | H | OH | Me | OCH₂CHFOCF₃ | SO₂Me |
| 10 | Me | H | OH | Cl | OCH₂CHFOMe | SO₂Me |
| 11 | Et | H | OH | Cl | OCH₂CHFOMe | SO₂Me |
| 12 | Me | H | OH | Me | OCH₂CHFOMe | SO₂Me |
| 13 | Et | H | OH | Me | OCH₂CHFOMe | SO₂Me |
| 14 | Me | H | OH | CF₃ | OCH₂CHFOMe | SO₂Me |
| 15 | Et | H | OH | CF₃ | OCH₂CHFOMe | SO₂Me |
| 16 | Me | H | OH | Br | OCH₂CHFOMe | SO₂Me |
| 17 | Et | H | OH | Br | OCH₂CHFOMe | SO₂Me |
| 18 | Me | H | OH | SO₂Me | OCH₂CHFOMe | CF₃ |
| 19 | Et | H | OH | SO₂Me | OCH₂CHFOMe | CF₃ |
| 20 | Me | H | OH | Cl | OCHFCH₂OCF₃ | SO₂Me |
| 21 | Et | H | OH | Cl | OCHFCH₂OCF₃ | SO₂Me |
| 22 | Me | H | OH | Cl | SCH₂CH₂SCH₃ | SO₂Me |
| 23 | Et | H | OH | Cl | SCH₂CH₂SCH₃ | SO₂Me |
| 24 | Me | H | OH | Me | SCH₂CH₂SCH₃ | SO₂Me |
| 25 | Et | H | OH | Me | SCH₂CH₂SCH₃ | SO₂Me |
| 26 | Me | H | OH | CF₃ | SCH₂CH₂SCH₃ | SO₂Me |
| 27 | Et | H | OH | CF₃ | SCH₂CH₂SCH₃ | SO₂Me |
| 28 | Me | H | OH | Br | SCH₂CH₂SCH₃ | SO₂Me |
| 29 | Et | H | OH | Br | SCH₂CH₂SCH₃ | SO₂Me |
| 30 | Me | H | OH | SO₂Me | SCH₂CH₂SCH₃ | CF₃ |
| 31 | Et | H | OH | SO₂Me | SCH₂CH₂SCH₃ | CF₃ |
| 32 | Me | H | OH | Cl | SCH₂CH₂SCF₃ | SO₂Me |
| 33 | Et | H | OH | Cl | SCH₂CH₂SCF₃ | SO₂Me |
| 34 | Me | H | OH | Me | SCH₂CH₂SCF₃ | SO₂Me |
| 35 | Et | H | OH | Me | SCH₂CH₂SCF₃ | SO₂Me |
| 36 | Me | H | OH | CF₃ | SCH₂CH₂SCF₃ | SO₂Me |
| 37 | E t | H | OH | CF₃ | SCH₂CH₂SCF₃ | SO₂Me |
| 38 | Me | H | OH | Br | SCH₂CH₂SCF₃ | SO₂Me |
| 39 | Et | H | OH | Br | SCH₂CH₂SCF₃ | SO₂Me |
| 40 | Me | H | OH | SO₂Me | SCH₂CH₂SCF₃ | CF₃ |
| 41 | Et | H | OH | SO₂Me | SCH₂CH₂SCF₃ | CF₃ |
| 42 | Me | H | OH | Cl | OCH₂CF₂OCH₃ | SO₂Me |
| 43 | Et | H | OH | Cl | OCH₂CF₂OCH₃ | SO₂Me |
| 44 | Me | H | OH | Me | OCH₂CF₂OCH₃ | SO₂Me |
| 45 | Et | H | OH | Me | OCH₂CF₂OCH₃ | SO₂Me |
| 46 | Me | H | OH | CF₃ | OCH₂CF₂OCH₃ | SO₂Me |
| 47 | Et | H | OH | CF₃ | OCH₂CF₂OCH₃ | SO₂Me |
| 48 | Me | H | OH | Br | OCH₂CF₂OCH₃ | SO₂Me |
| 49 | Et | H | OH | Br | OCH₂CF₂OCH₃ | SO₂Me |
| 50 | Me | H | OH | SO₂Me | OCH₂CF₂OCH₃ | CF₃ |
| 51 | Et | H | OH | SO₂Me | OCH₂CF₂OCH₃ | CF₃ |
| 52 | Me | H | OH | Me | OCH₂CH(OMe)CH₂OEt | SO₂Me |
| 53 | Me | H | OH | Me | OCH₂CH(CF₃)OMe | SO₂Me |
| 54 | Et | H | OH | Me | OCH₂CH(CF₃)OMe | SO₂Me |
| 55 | t-Bu | H | OH | Me | OCH₂CH(OMe)₂ | SO₂Me |
| 56 | Me | H | OH | Me | CH₂N(CO₂Me)₂ | SO₂Me |
| 57 | Et | H | OH | Me | CH₂N(CO₂Me)₂ | SO₂Me |
| 58 | Me | H | OH | Me | CH₂N(CH₂CN)₂ | SO₂Me |
| 59 | E t | H | OH | Me | CH₂N(CH₂CN)₂ | SO₂Me |
| 60 | Me | H | OH | Me | OCH₂CH(CF₃)OCF₃ | SO₂Me |
| 61 | Et | H | OH | Me | OCH₂CH(CF₃)OCF₃ | SO₂Me |
| 62 | Me | H | OH | Me | CH₂OCH₂CH(OMe)₂ | SO₂Me |
| 63 | Et | H | OH | Me | CH₂OCH₂CH(OMe)₂ | SO₂Me |
| 64 | Me | Me | OH | Me | OCH₂CH(OCH₃)₂ | SO₂Me |
| 65 | Me | Me | OH | Me | OCH₂CH(OMe)CH₂OEt | SO₂Me |
| 66 | Me | Me | OH | Me | CH₂SCN | SO₂Me |
| 67 | Me | Me | OH | Me | CH₂C(S)NH₂ | SO₂Me |
| 68 | Me | Me | OH | Me | OC(O)SMe | SO₂Me |
| 69 | Me | Me | OH | Me | OC(O)SEt | SO₂Me |
| 70 | Me | Me | OH | Cl | OCH₂CHFOCF₃ | SO₂Me |
| 71 | Et | Me | OH | Cl | OCH₂CHFOCF₃ | SO₂Me |
| 72 | Me | Me | OH | Me | OCH₂CHFOCF₃ | SO₂Me |
| 73 | Me | Me | OH | Cl | OCH₂CHFOMe | SO₂Me |
| 74 | Et | Me | OH | Cl | OCH₂CHFOMe | SO₂Me |
| 75 | Me | Me | OH | Me | OCH₂CHFOMe | SO₂Me |
| 76 | Et | Me | OH | Me | OCH₂CHFOMe | SO₂Me |
| 77 | Me | Me | OH | CF₃ | OCH₂CHFOMe | SO₂Me |
| 78 | Et | Me | OH | CF₃ | OCH₂CHFOMe | SO₂Me |
| 79 | Me | Me | OH | Br | OCH₂CHFOMe | SO₂Me |
| 80 | Et | Me | OH | Br | OCH₂CHFOMe | SO₂Me |
| 81 | Me | Me | OH | SO₂Me | OCH₂CHFOMe | CF₃ |
| 82 | Et | Me | OH | SO₂Me | OCH₂CHFOMe | CF₃ |
| 83 | Me | Me | OH | Cl | OCHFCH₂OCF₃ | SO₂Me |
| 84 | Et | Me | OH | Cl | OCHFCH₂OCF₃ | SO₂Me |
| 85 | Me | Me | OH | Cl | SCH₂CH₂SCH₃ | SO₂Me |
| 86 | Et | Me | OH | Cl | SCH₂CH₂SCH₃ | SO₂Me |
| 87 | Me | Me | OH | Me | SCH₂CH₂SCH₃ | SO₂Me |
| 88 | Et | Me | OH | Me | SCH₂CH₂SCH₃ | SO₂Me |
| 89 | Me | Me | OH | CF₃ | SCH₂CH₂SCH₃ | SO₂Me |
| 90 | Et | Me | OH | CF₃ | SCH₂CH₂SCH₃ | SO₂Me |
| 91 | Me | Me | OH | Br | SCH₂CH₂SCH₃ | SO₂Me |
| 92 | Et | Me | OH | Br | SCH₂CH₂SCH₃ | SO₂Me |
| 93 | Me | Me | OH | SO₂Me | SCH₂CH₂SCH₃ | CF₃ |
| 94 | Et | Me | OH | SO₂Me | SCH₂CH₂SCH₃ | CF₃ |
| 95 | Me | Me | OH | Cl | SCH₂CH₂SCF₃ | SO₂Me |
| 96 | Et | Me | OH | Cl | SCH₂CH₂SCF₃ | SO₂Me |
| 97 | Me | Me | OH | Me | SCH₂CH₂SCF₃ | SO₂Me |
| 98 | Et | Me | OH | Me | SCH₂CH₂SCF₃ | SO₂Me |
| 99 | Me | Me | OH | CF₃ | SCH₂CH₂SCF₃ | SO₂Me |
| 100 | Et | Me | OH | CF₃ | SCH₂CH₂SCF₃ | SO₂Me |
| 101 | Me | Me | OH | Br | SCH₂CH₂SCF₃ | SO₂Me |
| 102 | Et | Me | OH | Br | SCH₂CH₂SCF₃ | SO₂Me |
| 103 | Me | Me | OH | SO₂Me | SCH₂CH₂SCF₃ | CF₃ |
| 104 | Et | Me | OH | SO₂Me | SCH₂CH₂SCF₃ | CF₃ |
| 105 | Me | Me | OH | Cl | OCH₂CF₂OCH₃ | SO₂Me |
| 106 | Et | Me | OH | Cl | OCH₂CF₂OCH₃ | SO₂Me |
| 107 | Me | Me | OH | Me | OCH₂CF₂0CH₃ | SO₂Me |
| 108 | Et | Me | OH | Me | OCH₂CF₂OCH₃ | SO₂Me |
| 109 | Me | Me | OH | CF₃ | OCH₂CF₂OCH₃ | SO₂Me |
| 110 | Et | Me | OH | CF₃ | OCH₂CF₂OCH₃ | SO₂Me |
| 111 | Me | Me | OH | Br | OCH₂CF₂OCH₃ | SO₂Me |
| 112 | Et | Me | OH | Br | OCH₂CF₂OCH₃ | SO₂Me |
| 113 | Me | Me | OH | SO₂Me | OCH₂CF₂OCH₃ | CF₃ |
| 114 | Et | Me | OH | SO₂Me | OCH₂CF₂OCH₃ | CF₃ |
| 115 | Me | Me | OH | Me | OCH₂CH(CF₃)OMe | SO₂Me |
| 116 | E t | Me | OH | Me | OCH₂CH(CF₃)OMe | SO₂Me |
| 117 | t-Bu | Me | OH | Me | OCH₂CH(OMe)₂ | SO₂Me |
| 118 | Me | Me | OH | Me | CH₂N(CO₂Me)₂ | SO₂Me |
| 119 | Et | Me | OH | Me | CH₂N(CO₂Me)₂ | SO₂Me |
| 120 | Me | Me | OH | Me | CH₂N(CH₂CN)₂ | SO₂Me |
| 121 | E t | Me | OH | Me | CH₂N(CH₂CN)₂ | SO₂Me |
| 122 | Me | Me | OH | Me | OCH₂CH(CF₃)OCF₃ | SO₂Me |
| 123 | Et | Me | OH | Me | OCH₂CH(CF₃)OCF₃ | SO₂Me |
| 124 | Me | Me | OH | Me | CH₂OCH₂CH(OMe)₂ | SO₂Me |
| 125 | Et | Me | OH | Me | CH₂OCH₂CH(OMe)₂ | SO₂Me |
| 126 | Me | H | OH | Me | CO₂Me | SO₂Me |
| 127 | Et | H | OH | Me | CO₂Me | SO₂Me |
| 128 | Et | H | OH | Me | CO₂ (i-Pr) | SO₂Me |
| 129 | Me | H | OH | Cl | CO₂Et | SO₂Me |
| 130 | Et | H | OH | Me | CO₂Me | CF₃ |
| 131 | Et | H | OH | Me | OCH₂CH₂OMe | SO₂Me |
| 132 | Et | H | OH | SO₂Me | CO₂Me | CN |
| 133 | Me | H | OH | Me | C(O)SMe | SO₂Me |
| 134 | Me | H | OH | Me | C(O)SEt | SO₂Me |
| 135 | Me | H | OH | Me | 2-(2-Oxolanyl)ethoxy | SO₂Me |
| 136 | Me | H | OH | Me | 2-2-1,3-Dioxolanyl)ethoxy | SO₂Me |
| 137 | Et | H | OH | Me | CH₂OMe | SO₂Me |
| 138 | Et | H | OH | Me | 2-Oxolanylmethoxymethyl | SO₂Me |
| 139 | Me | H | OH | Cl | CO₂Me | SO₂Me |
| 140 | E t | H | OH | Cl | CO₂Me | SO₂Et |
| 141 | Me | H | OH | Cl | C(O)SMe | SO₂Me |
| 142 | Me | H | OH | Cl | C(O)SEt | SO₂Me |
| 143 | Me | H | OH | Me | OMe | SO₂Me |
| 144 | Me | H | OH | Me | OEt | SO₂Me |
| 145 | Me | H | OH | Me | O(i-Pr) | SO₂Me |
| 146 | Me | H | OH | Me | OCHF₂ | SO₂Me |
| 147 | Me | H | OH | Me | (4,5-Dihydroisoxazol-3-yl) | SO₂Me |
| 148 | Me | H | OH | Me | O(n-Pr) | SO₂Et |
| 149 | Me | H | OH | Cl | CH₂OMe | SO₂Me |
| 150 | Me | H | OH | Me | OCO₂Me | SO₂Me |
| 151 | Me | H | OH | Me | OCH₂CH₂OMe | SO₂Me |
| 152 | Et | H | OH | Me | OEt | SO₂Me |
| 153 | Et | H | OH | Cl | CO₂Et | SO₂Me |
| 154 | Et | H | OH | Cl | CO₂(n-Pr) | SO₂Me |
| 155 | Et | H | OH | Me | CO₂Et | SO₂Me |
| 156 | Me | H | OH | Me | CH₂CO₂Me | SO₂Me |
| 157 | Me | H | OH | Me | OCH₂CO₂Et | SO₂Me |
| 158 | Me | H | OH | Me | O(n-Pr) | SO₂Me |
| 159 | Et | H | OH | SO₂Me | H | CF₃ |
| 160 | Me | H | OH | Me | CH₂OCH₂CF₃ | SO₂Me |
| 161 | Me | H | OH | Cl | CH₂OCH₂CF₃ | SO₂Me |
| 162 | Et | H | OH | Me | Cl | SO₂Me |
| 163 | Me | H | OH | Me | CH₂SO₂Me | SO₂Me |
| 164 | Me | H | OH | Me | CH₂OEt | SO₂Me |
| 165 | Me | H | OH | Me | CH₂CH₂OMe | SO₂Me |
| 166 | Me | H | OH | Me | CH₂OCH₂CH₂OMe | SO₂Me |
| 167 | Me | H | OH | Me | OCH₂CH₂OEt | SO₂Me |
| 168 | Me | H | OH | Me | OCH₂CH₂Cl | SO₂Me |
| 169 | Me | H | OH | Me | OCH₂CF₃ | SO₂Me |
| 170 | Me | H | OH | Me | CH₂OCH₂OMe | SO₂Me |
| 171 | Me | H | OH | Me | CN | SO₂Me |
| 172 | Me | H | OH | Me | CH₂CN | SO₂Me |
| 173 | Me | H | OH | Br | CO₂Me | SO₂Me |
| 174 | Et | H | OH | Cl | CO₂Me | SO₂Me |
| 175 | Me | H | OH | Cl | OCH₂CH₂OCF₃ | SO₂Me |
| 176 | Et | H | OH | Cl | OCH₂CH₂OCF₃ | SO₂Me |
| 177 | Me | H | OH | Me | OCH₂CH₂OCF₃ | SO₂Me |
| 178 | Et | H | OH | Me | OCH₂CH₂OCF₃ | SO₂Me |
| 179 | Me | H | OH | CF₃ | OCH₂CH₂OCF₃ | SO₂Me |
| 180 | Et | H | OH | CF₃ | OCH₂CH₂OCF₃ | SO₂Me |
| 181 | Me | H | OH | Br | OCH₂CH₂OCF₃ | SO₂Me |
| 182 | Et | H | OH | Br | OCH₂CH₂OCF₃ | SO₂Me |
| 183 | Me | H | OH | SO₂Me | OCH₂CH₂OCF₃ | CF₃ |
| 184 | E t | H | OH | SO₂Me | OCH₂CH₂OCF₃ | CF₃ |
| 185 | Me | H | OH | Cl | OCH₂CH₂OCHClF | SO₂Me |
| 186 | Et | H | OH | Cl | OCH₂CH₂OCHClF | SO₂Me |
| 187 | Me | H | OH | Me | OCH₂CH₂OCHClF | SO₂Me |
| 188 | Et | H | OH | Me | OCH₂CH₂OCHClF | SO₂Me |
| 189 | Me | H | OH | CF₃ | OCH₂CH₂OCHClF | SO₂Me |
| 190 | Et | H | OH | CF₃ | OCH₂CH₂OCHClF | SO₂Me |
| 191 | Me | H | OH | Br | OCH₂CH₂OCHClF | SO₂Me |
| 192 | Et | H | OH | Br | OCH₂CH₂OCHClF | SO₂Me |
| 193 | Me | H | OH | SO₂Me | OCH₂CH₂OCHClF | CF₃ |
| 194 | Et | H | OH | SO₂Me | OCH₂CH₂OCHClF | CF₃ |
| 195 | Me | H | OH | Cl | OCH₂CH₂OCF₂Cl | SO₂Me |
| 196 | Et | H | OH | Cl | OCH₂CH₂OCF₂Cl | SO₂Me |
| 197 | Me | H | OH | Me | OCH₂CH₂OCF₂Cl | SO₂Me |
| 198 | Et | H | OH | Me | OCH₂CH₂OCF₂Cl | SO₂Me |
| 199 | Me | H | OH | CF₃ | OCH₂CH₂OCF₂Cl | SO₂Me |
| 200 | Et | H | OH | CF₃ | OCH₂CH₂OCF₂Cl | SO₂Me |
| 201 | Me | H | OH | Br | OCH₂CH₂OCF₂Cl | SO₂Me |
| 202 | Et | H | OH | Br | OCH₂CH₂OCF₂Cl | SO₂Me |
| 203 | Me | H | OH | SO₂Me | OCH₂CH₂OCF₂Cl | CF₃ |
| 204 | Et | H | OH | SO₂Me | OCH₂CH₂OCF₂Cl | CF₃ |
| 205 | Me | H | OH | Cl | SCH₂CH₂OCH₃ | SO₂Me |
| 206 | Et | H | OH | Cl | SCH₂CH₂OCH₃ | SO₂Me |
| 207 | Me | H | OH | Me | SCH₂CH₂OCH₃ | SO₂Me |
| 208 | Et | H | OH | Me | SCH₂CH₂OCH₃ | SO₂Me |
| 209 | Me | H | OH | CF₃ | SCH₂CH₂OCH₃ | SO₂Me |
| 210 | Et | H | OH | CF₃ | SCH₂CH₂OCH₃ | SO₂Me |
| 211 | Me | H | OH | Br | SCH₂CH₂OCH₃ | SO₂Me |
| 212 | Et | H | OH | Br | SCH₂CH₂OCH₃ | SO₂Me |
| 213 | Me | H | OH | SO₂Me | SCH₂CH₂OCH₃ | CF₃ |
| 214 | Et | H | OH | SO₂Me | SCH₂CH₂OCH₃ | CF₃ |
| 215 | Me | H | 0H | Cl | SCH₂CH₂OCF₃ | SO₂Me |
| 216 | Et | H | OH | Cl | SCH₂CH₂OCF₃ | SO₂Me |
| 217 | Me | H | OH | Me | SCH₂CH₂OCF₃ | SO₂Me |
| 218 | Et | H | OH | Me | SCH₂CH₂OCF₃ | SO₂Me |
| 219 | Me | H | OH | CF₃ | SCH₂CH₂OCF₃ | SO₂Me |
| 220 | Et | H | OH | CF₃ | SCH₂CH₂OCF₃ | SO₂Me |
| 221 | Me | H | OH | Br | SCH₂CH₂OCF₃ | SO₂Me |
| 222 | Et | H | OH | Br | SCH₂CH₂OCF₃ | SO₂Me |
| 223 | Me | H | OH | SO₂Me | SCH₂CH₂OCF₃ | CF₃ |
| 224 | Et | H | OH | SO₂Me | SCH₂CH₂OCF₃ | CF₃ |
| 225 | Me | H | OH | Cl | OCH₂CH (CH₃) OCH₃ | SO₂Me |
| 226 | Et | H | OH | Cl | OCH₂CH (CH₃) OCH₃ | SO₂Me |
| 227 | Me | H | OH | Me | OCH₂CH (CH₃) OCH₃ | SO₂Me |
| 228 | Et | H | OH | Me | OCH₂CH (CH₃) OCH₃ | SO₂Me |
| 229 | Me | H | OH | CF₃ | OCH₂CH (CH₃) OCH₃ | SO₂Me |
| 230 | Et | H | OH | CF₃ | OCH₂CH (CH₃) OCH₃ | SO₂Me |
| 231 | Me | H | OH | Br | OCH₂CH (CH₃) OCH₃ | SO₂Me |
| 232 | Et | H | OH | Br | OCH₂CH (CH₃) OCH₃ | SO₂Me |
| 233 | Me | H | OH | SO₂Me | OCH₂CH (CH₃) OCH₃ | CF₃ |
| 234 | Et | H | OH | SO₂Me | OCH₂CH (CH₃) OCH₃ | CF₃ |
| 235 | Me | H | OH | Me | CH₂N (Me) CH₂CN | SO₂Me |
| 236 | Me | H | OH | Me | (Tetrahydrofuran-2-yl)methoxy | SO₂Me |
| 237 | Me | H | OH | Cl | SMe | SO₂Me |
| 238 | Me | H | OH | Cl | Cl | SO₂Me |
| 239 | Me | H | OH | Cl | OMe | SO₂Me |
| 240 | Me | H | OH | Me | (Tetrahydro-2H-pyran-2-yl)methoxy | SO₂Me |
| 241 | Me | H | OH | Cl | OCH₂CH₂OMe | SO₂Me |
| 242 | Me | H | OH | Me | Tetrahydrofuran-3-yloxy | SO₂Me |
| 243 | Me | H | OH | Me | OCH₂CH₂CH₂OMe | SO₂Me |
| 244 | Et | H | OH | Cl | (1,3-Dioxolan-2-yl)ethoxy | SO₂Me |
| 245 | Me | H | OH | Me | Propargyloxy | SO₂Me |
| 246 | Me | H | OH | Me | (Tetrahydrofuran-3-yloxy)methyl | SO₂Me |
| 247 | Me | H | OH | Cl | SO₂Me | SO₂Me |
| 248 | Me | H | OH | Me | (CH₂)₆Me | SO₂Me |
| 249 | Me | H | OH | Me | CH₂CH₂CH₂OMe | SO₂Me |
| 250 | Et | H | OH | Cl | (1,3-Dioxolan-2-yl)methoxy | SO₂Me |
| 251 | Me | H | OH | Me | CH₂N[C(O)SEt]CH₂CN | SO₂Me |
| 252 | Me | H | OH | Me | CH=CHCN | SO₂Me |
| 253 | Me | H | OH | Me | CH₂CH₂CN | SO₂Me |
| 254 | Me | H | OH | Me | OCH(CH₃)CH₂OMe | SO₂Me |
| 255 | Me | H | OH | Me | OCH₂CH(Et)OMe | SO₂Me |
| 256 | Me | H | OH | Me | (1,3-Dioxolan-2-yl)methyl | SO₂Me |
| 257 | Me | H | OH | Me | CH₂O(i-Pr) | SO₂Me |
| 258 | i-Pr | H | OH | Me | OCH₂CH₂OMe | SO₂Me |
| 259 | Me | Me | OH | Me | OCH₂CH₂OMe | SO₂Me |
| 260 | i-Pr | Me | OH | Me | OCH₂CH₂OMe | SO₂Me |
| 261 | Me | Et | OH | Me | OCH₂CH₂OMe | SO₂Me |

**TABLE 2**

| No. | ¹H-NMR δppm (solvent: CDCl₃ unless otherwise specified, measuring apparatus: JEOL-GSX (400 MHz) or VARIAN MERCURY plus (300 MHz)/the same applies hereinafter) |
|---|---|
| 1 | 2.38(s, 3H), 3.29(s, 3H), 3.47(s, 6H), 3.70(s, 3H), 4.09(d, 2 H, J=5. 2Hz), 4. 1(br s, 1H), 4. 83(t, 1H, J=5. 2Hz), 7. 32(s, 1H) 7. 35(d, 1H, J=8. 4Hz), 7. 91(d, 1H, J=8. 4Hz). |
| 2 | 2.58(s, 3H), 2. 80-3. 20(br s, 1H), 3. 25(s, 3H), 3.73(s, 3H), 4 . 89(s, 2H), 7.33(s, 1H), 7. 58(d, 1H. J=8. 1Hz), 8.07(d, 1H, J= 8. 1Hz). |
| 3 | 2. 50(s, 3H), 3.20(s, 3H), 3.72(s, 3H), 4. 66(s, 2H), 7.32(s, 1 H), 7. 40-7. 50(br s, 1H), 7.52(d, 1H, J=8. 1Hz), 8.11(d, 1H, J= 8. 1Hz). |
| 126 | 2. 32(s, 3H), 3. 13 (s, 3H), 3. 61(s, 3H), 3. 93(s, 3H), 7. 28(s, 1H), 7. 56(d, 1H. J=7. 8Hz), 7. 93(d, 1H, J=7. 8Hz), 8. 44(br. s, 1 H). |
| 127 | 1. 46 (t, 3H), 2. 38(s, 3H), 3. 18 (s, 3H), 3.98 (s, 3H), 4.07 ( q. 2H) , 7. 32 (d, 1H, J=7. 8Hz), 7. 61 (s, 1H). 7. 98(d, 1H, J=7 . 8Hz). |
| 131 | 1.40(t, 3H, J=7. 0Hz), 2. 39(s, 3H), 3. 25(s, 3H), 3. 42 (s, 3H), 3. 76(m, 2H), 4. 02(q, 2H, J=7.0Hz). 4. 20(m, 2H), 7.28(s. 1H), 7. 31(d, 1H. J=7. 6Hz), 7. 87(d, 1H. J=7.6Hz). |
| 143 | 2. 31(s, 3H), 3. 20(s, 3H), 3. 66(s, 3H), 3. 92(s, 3H), 7. 1( br s 1H). 7. 29(d, 1H J=7. 6Hz), 7. 30(s, 1H), 7. 85(d, 1H, J=7. 6Hz). |
| 144 | 1. 48(t, 3H, J=7. 2Hz), 2. 36(s, 3H), 3. 34(s, 3H), 3. 71(s, 3H), 4.10(q. 2H, J=7. 2Hz), 6. 98(br s, 1H), 7.37(m. 2H), 7.90(d. 1H J=7.2Hz). |
| 145 | 1. 34(d, 6H, J=6. 4Hz), 2. 33(s, 3H), 3. 22(s, 3H), 3. 70(s, 3H), 4. 82(qq, 1H, J=6. 4, 6. 4Hz), 6. 90(br s, 1H), 7. 29(d, 1H, J=7. 6 Hz), 7. 33(s, 1H), 7. 94(d, 1H, J=7.6Hz). |
| 146 | 2. 43(s, 3H), 2. 23(s, 3H), 3. 71(s, 3H), 5. 30(br s, 1H), 6. 75 (t 1H, J=74. 8Hz), 7. 33(s, 1H), 7. 50(d, 1H, J=8. 0Hz), 8. 00 (d, 1 H, J=8.0Hz). |
| 151 | 2.39(s, 3H), 3.29(s, 3H), 3.46 (s, 3H), 3.71(s. 3H), 3.81(m, 2 H), 4.24(m. 2H). 7.34(s. 1H), 7.35(d, 1H, J=7.6Hz), 7.92(d, 1 H, J=7.6Hz). |
| 152 | 1.44(t, 3H, J=7.2Hz), 1.48(t, 3H, J=7.2Hz), 2.36(s, 3H), 3.27 (s, 3H), 4.06(q, 2H, J=7.2Hz), 4.13(q, 2H, J=7.2Hz), 5.2(br s , 1H), 7.33(d, 1H, J=8.0Hz), 7.34(s, 1H), 7.90(d, 1H, J=8.0Hz ). |
| 153 | (Acetone-d₆) 1.30(br s, 3H), 1.37(t, 3H, J=7.0Hz), 3.25(s, 3H ), 3.95 (br s, 2H), 4.43(q, 2H, J=7.0Hz), 7.27(br s, 1H), 7.75 (br s, 1H), 8.07(br s, 1H). |
| 154 | (Acetone-d₆) 1.18(t. 3H, J=7.4Hz). 1.34(br s. 3H), 1.80(m, 2H ), 3.25(s, 3H), 3.98(br s, 2H), 4.33(t, 2H, J=5.6Hz), 7.32(br s, 1H), 7.81(br s, 1H), 8.08(br s, 1H). |
| 156 | 2.33(s, 3H), 3.16(s, 3H), 3.73(s, 2H), 3.76(s, 3H), 4.42(s, 2 H), 7.20-7.60 (br s, 1H), 7.34(s, 1H), 7.52(d, 1H, J=8.1Hz), 8.10(d, 1H, J=8.1Hz). |
| 157 | 1.27(t, 3H, J=7.6Hz), 2.32(s, 3H), 3.32 (s, 3H), 3.66(s, 3H), 4.25(q, 2H, J=7.6Hz), 4.61(s, 2H), 7.30(s. 1H), 7.35(d, 1H, J =8.0Hz), 7.88(d, 1H, J=8.0Hz). |
| 160 | 2.48(s, 3H), 3.16(s, 3H), 3.71(s, 3H), 3.98-4.04(m, 2H), 5.23 (s, 2H), 7.30(s, 1H), 7.55(d, 1H. J=8.0Hz), 8.04(br s, 1H), 8 .09(d, 1H, J=8.0Hz) |
| 163 | (Acetone-d₆) 2.51(s. 3H), 3.12(s, 3H), 3.23(s. 3H), 3.29(s. 3 H), 5.4 (br s, 2H), 6.8 (br s, 1H), 7.42 (d, 1H, J=8.0Hz), 8.00( d, 1H, J=8.0Hz). |
| 171 | 2.72(s. 3H), 3.34(s. 3H), 3.74(s, 3H), 5.10-5.60(br s, 1H), 7 .32(s, 1H). 7.81(d, 1H, J=8.1Hz). 8.16(d. 1H, J=8.1Hz). |
| 172 | 2.53(s, 3H), 3.24(s, 3H), 3.74(s, 3H), 4.47(s. 2H), 6.70-7.20 (br s, 1H), 7.33(s, 1H), 7.60(d. 1H, J=8.1Hz), 8.14(d, 1H, J= 8. 1Hz). |
| 174 | 1. 42 (t, 3H, J=7.3Hz), 3. 20 (s, 3H), 4. 04 (s, 3H), 4.09 (q, 2 H, J=7.3Hz), 7.34(s, 1H), 7.64(d, 1H, J=7.8Hz). 8.07(d, 1H, J=7.8Hz). |
| 227 | 1.23(d. 3H, J=6.4Hz). 2.34(s. 3H), 3.24(s, 3H), 3.41(s. 3H), 3.65(s. 3H), 3.77 (m, 2H), 3.99(dd. 1H, J=9.2. 4.0Hz), 4.05(dd , 1H, J=9.2, 6.4Hz). 7.28 (s, 1H), 7.29(d. 1H, J=8.4Hz), 7.86 ( d, 1H, J=8.4Hz). |
| 235 | 2.41(s, 3H), 2.48(s, 3H), 3.27(s, 3H), 3.63(s, 2H), 3.72(s, 3 H), 4.23(s, 2H), 7.29(s, 1H), 7.51(d, 1H. J=8.1Hz). 8.12(d. 1 H, J=8.1Hz). |
| 238 | 2.55(s. 3H), 3.45(s, 3H), 7.29-7.33(m, 2H), 7.35(d, 1H, J=8. 4 Hz). |
| 244 | 1.43(t, 3H, 1=7.3Hz), 2.28 (m, 2H), 3.29 (s, 3H), 3.86 (m, 2 H), 3.96 (m, 2H), 4.08 (m, 2H), 4.39 (m, 2H), 5.13 (t, 1H. J= 5.5Hz), 7. 32 (s, 1H), 7.33 (d, 1H, J=7.8Hz), 7.96(d, 1H J=7. 8Hz). |
| 247 | 2.49(s. 3H), 3.54(s. 3H), 3.57(s. 3H), 7.40(s. 1H), 7.63(d, 1 H, J=7.6Hz), 8.06(d, 1H. J=7.6Hz). |
| 250 | 1. 42 (t, 3H, J=7.3Hz), 3.35 (s, 3H). 3.95 (m, 2H), 4.04-4.12 (m, 4H), 4.29 (m, 2H), 5.46 (t, 1H. J=5.5Hz), 7.32 (s, 1H), 7 .36 (d, 1H, J=7.8Hz). 7.98(d, 1H, J=7.8Hz). |
| 254 | 1.25 (t, 3H, J=7.3Hz). 2.35 (s, 3H), 3.25 (s, 3H), 3.34 (s, 3 H), 3. 53 (m, 1H), 3.70 (s, 3H), 3.74 (m, 1H), 4.88 (m, 1H), 7 .24 (s, 1H), 7.31 (d, 1H, J-7.8Hz), 7.85 (d, 1H, J=7.8Hz). |
| 256 | 2.41(s, 3H), 3.14(s. 3H), 3.61(d. 2H, J=5.2Hz), 3.65(s. 3H), 3.77-3.82 (m, 2H). 3. 88-3. 96 (m, 2H), 5.12(t, 1H, J=4.8Hz), 5. 4 5(br s, 1H), 7.24(s. 1H), 7.37(d. 1H, J=8.0Hz), 8.01(d, 1H, J =8.0Hz). |
| 257 | 1.25(d, 6H. J=6.4Hz), 1.44(t. 3H, J=7.4Hz), 2.47(s. 3H), 3. 25 (s, 3H), 3. 83 (m, 1H), 4. 06 (q, 2H, J=7. 4Hz), 4. 7 (br s, 1H), 5. 00 (s. 2H), 7.31(s, 1H). 7.50(d, 1H. J=8. 4Hz), 8.07 (d, 1H, J=8 .4Hz). |
| 258 | 1.48(d, 6H. J=6.8Hz), 2.40(s, 3H), 3.30(s, 3H), 3.46(s, 3H), 3.80(t, 2H J=8.4Hz) 4.24(t, 2H, J=8.4Hz) 5.89(m, 1H) 7.35(d. 1H, J=8.0Hz) 7.37(s. 1H) 7.91(d. 1H, J=8.0Hz). |
| 259 | 1.62(s, 3H), 2.26(s. 3H), 3.25(s. 3H), 3.42(s, 3H), 3.58(s, 3 H), 3.76(t, 2H, J=4.4Hz), 4.19(t, 2H, J=4.4Hz), 7.11(d, 1H. J =8.0Hz), 7.87(d, 1H, J=8.0Hz). |
| 260 | 1.46(d, 6H, J=6.8Hz), 1.67 (s, 3H), 2.32(s, 3H), 3.27(s, 3H), 3.56(s, 3H), 3.80(t, 2H J=8.4Hz) 4.23(t, 2H, J=8.4Hz) 4.55(m, 1H) 7.15(d. 1H, J=8.0Hz). 7.91(d, 1H, J=8.0Hz). |
| 261 | 0.85(t, 3H. J=7.2Hz), 2.00(q, 2H, J=7.2Hz). 2.30(s. 3H), 3.28 (s, 3H), 3.45(s, 3H), 3.62(s, 3H), 3.79(t. 2H, J=4.4Hz). 4.22 (t, 2H, J=4.4Hz), 7.16(d. 1H, J=8.0Hz), 7.89 (d, 1H, J=8.0Hz). |

### TEST EXAMPLE 1

Upland field soil was put into a 1/170,000 hectare pot, and seeds of various plants were sown. When the respective plants reached predetermined leaf stage ((1) barnyardgrass (Echinochloa crus-galli L.): 1.0 to 3.0 leaf stage, (2) crabgrass (Digitaria sanguinalis L.): 1.0 to 3.0 leaf stage, (3) green foxtail Setaria viridis L.): 1.0 to 3.0 leaf stage, (4) redroot pigweed (Amaranthus retroflexus L.): cotyledon stage to 2.0 leaf stage, (5) prickly sida (Sida spinosa L.): cotyledon stage to 1.0 leaf stage, (6) velvetleaf Abutilon theophrasti MEDIC.): 0.1 to 1.5 leaf stage, (7) rice (Oryza sativa L.): 1.0 to 3.0 leaf stage, (8) wheat (Triticum spp.): 1.7 to 3.4 leaf stage, (9) corn (Zea mays L.): 2.0 to 3.5 leaf stage, (10) soybean (Glycine max Merr.): primary leaf stage to 0.4 leaf stage), wettable powders or emulsifiable concentrates of the compounds of the above formula (I) prepared in accordance with a conventional preparation method, were weighed so that the active ingredients became the prescribed amounts, and diluted with water in an amount corresponding to 500 liter per 1 hectare (containing 0.1 vol% of "KUSARINOH", manufactured by NIHON NOHYAKU CO., LTD. as an agricultural spreader). The spray solutions thus prepared were applied for foliar treatment by a small sprayer.

On the 17t to 25th day after application, the state of growth of the respective plants was visually observed, and the herbicidal effect was evaluated by a growth inhibition rate (%) of 0 (equivalent to the non-treated area) to 100% (complete kill). The results are shown in Table 3.

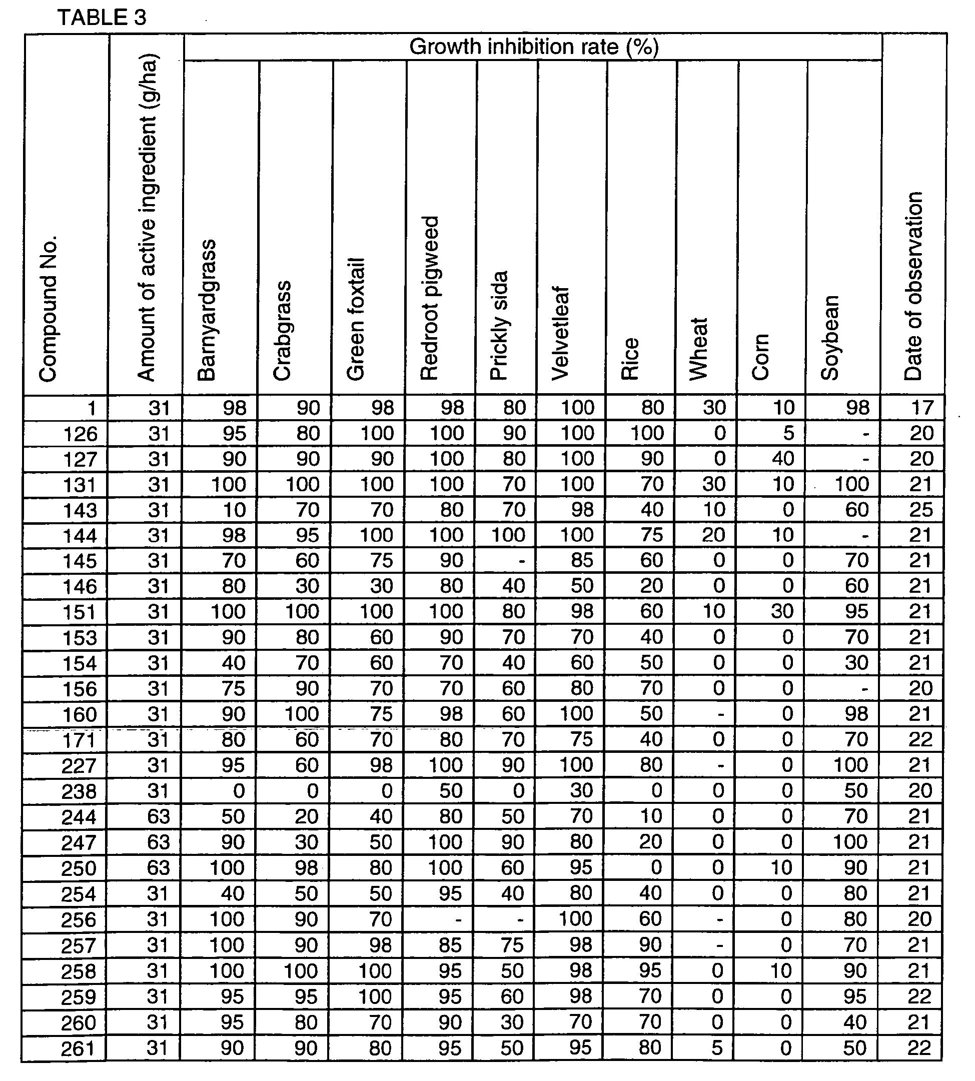

### TEST EXAMPLE 2

Upland field soil was put into a 1/170,000 hectare pot, and seeds of various plants barnyardgrass (Echinochloa crus-galli L.), crabgrass (Digitaria sanguinalis L.), green foxtail (Setaria viridis L.), redroot pigweed (Amaranthus retroflexus L.), prickly sida (Sida spinosa L.), velvetleaf (Abutilon theophrasti MEDIC.), rice (Oryza sativa L.), wheat (Triticum spp.), corn (Zea mays L.) and soybean (Glycine max Merr.)) were sown. On the day after sowing, wettable powders or emulsifiable concentrates of the compounds of the above formula (I) prepared in accordance with a conventional preparation method, were weighed so that the active ingredients became the prescribed amounts, and diluted with water in an amount corresponding to 500 liter per 1 hectare, followed by soil application with a small sprayer.

On the 19th to 28th day after the application, the state of growth of the respective plants was visually observed, and the herbicidal effect was evaluated by a growth inhibition rate (%) of from 0 (equivalent to the non-treated area) to 100% (complete kill). The results are shown in Table 4.

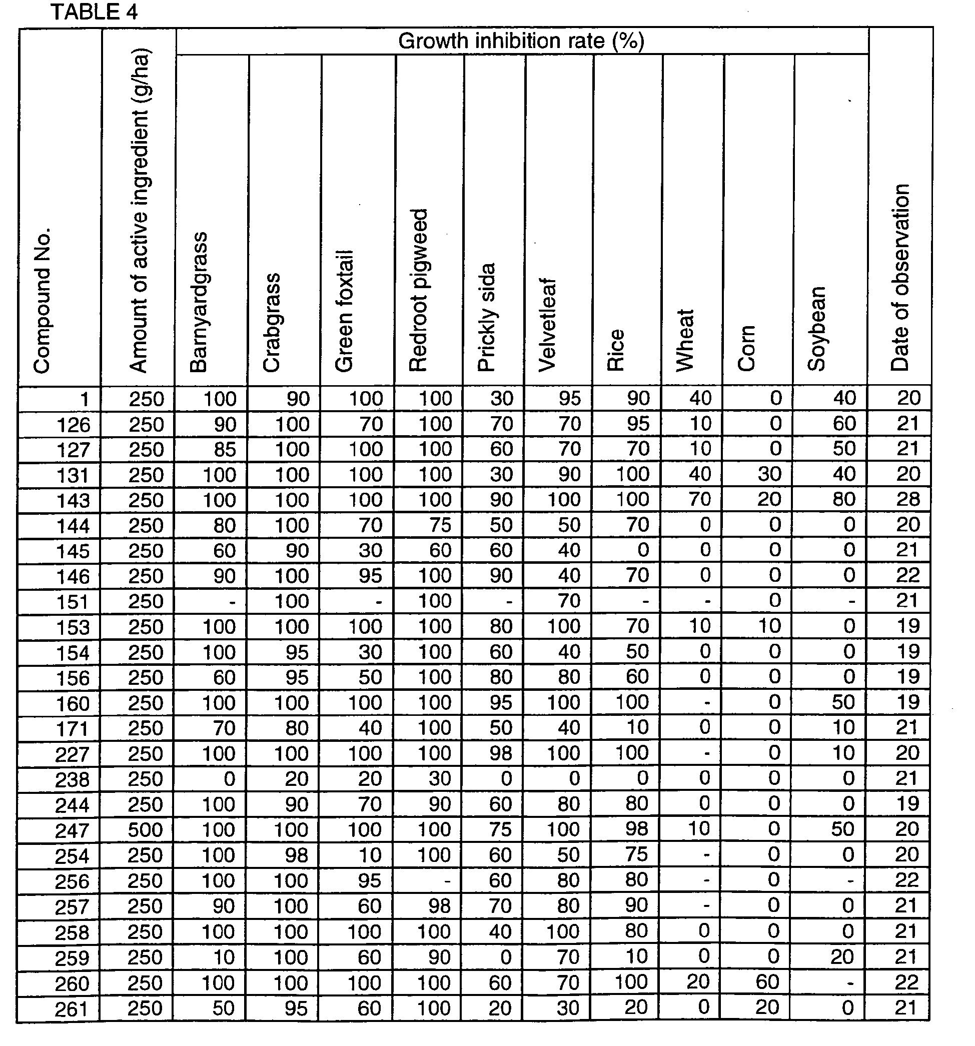

### TEST EXAMPLE 3

Upland field soil was put into a 1/1,000,000 hectare pot, and seeds of various plants were sown. When the respective plants reached predetermined leaf stage ((1) green foxtail (Setaria viridis L.): 4.0 to 4.5 leaf stage, (2) guineagrass (Panicum maximum Jacq.): 3.5 to 4.3 leaf stage, and (3) corn (Zea mays L.): 4.0 to 4.3 leaf stage, wettable powders or emulsifiable concentrates of compounds of the above formula (I), prepared in accordance with a conventional preparation method, were weighed so that the active ingredients became the prescribed amounts, and diluted with water in an amount corresponding to 300 or 500 liter per 1 hectare (containing of an agricultural spreader (MSO concentrate, manufactured by Cognis Corporation or "KUSARINOH", manufactured by NIHON NOHYAKU CO., LTD.). The spray solutions thus prepared were applied for foliar treatment by a small sprayer.

On the 18th to 24th day after application, the state of growth of the respective plants was visually observed, and the herbicidal effect was evaluated by a growth inhibition rate (%) of 0 (equivalent to the non-treated area) to 100% (complete kill). The results are shown in Tables 5 to 7.

**TABLE 5**

| Compound No. | Amount of active ingredient (g / ha) | Growth inhibition rate (%) (24th day after application) |
|---|---|---|
| | | Green foxtail |
| 151 | 15 | 100 |
| | 7 | 97 |
| 131 | 15 | 100 |
| | 7 | 98 |
| 258 | 15 | 100 |
| | 7 | 95 |

(Agricultural spreader: MSO Concentrate 0.5 vol%, amount of water spread: 300 Uha)

**TABLE 6**

| Compound No. | Amount of active ingredient (g / ha) | Growth inhibition rate (%) (18th day after application) |
|---|---|---|
| | | Guineagrass |
| 151 | 31 | 93 |
| 131 | 31 | 93 |

(Agricultural spreader: KUSARINOH Concentrate 0.1 vol%, amount of water spread: 500 L/ha)

**TABLE 7**

| Compound No. | Amount of active ingredient (g / ha) | Growth inhibition rate (%) (21st day after application) |
|---|---|---|
| | | Corn |
| 151 | 120 | 2 |
| 131 | 120 | 0 |

(Agricultural spreader: MSO Concentrate 0.5 vol%, amount of water spread: 300 Uha)

Now, Formulation Examples of the present invention will be described.

### FORMULATION EXAMPLE 1

| | | |
|---|---|---|
| (1) | The compound of the above formula (1) | 75 parts by weight |
| (2) | Geropon T-77 (tradename, manufactured by Rhone-Poulenc) | 14.5 parts by weight |
| (3) | NaCl | 10 parts by weight |
| (4) | Dextrin | 0.5 part by weight |

The above components are placed in a high-speed mixing granulator, admixed with 20 wt% of water, granulated, and dried to obtain water-dispersible granules.

### FORMULATION EXAMPLE 2

| | | |
|---|---|---|
| (1) | Kaolin | 78 parts by weight |
| (2) | Laveline FAN (tradename, manufactured by DAI-ICHI KOGYO SEIYAKU CO., LTD.) | 2 parts by weight |
| (3) | Sorpol 5039 (tradename, manufactured by TOHO Chemical Industry Co., Ltd.) | 5 parts by weight |
| (4) | Carplex (tradename, manufactured by | |
| | Shionogi & Company, Limited) | 15 parts by weight |

The mixture of the above components (1) to (4) and the compound of the above formula (I) are mixed in a weight ratio of 9:1 to obtain a wettable powder.

### FORMULATION EXAMPLE 3

| | | |
|---|---|---|
| (1) | Hi-Filler No. 10 (tradename, manufactured by Matsumura Sangyo Co., Ltd.) | 33 parts by weight |
| (2) | Sorpol 5050 (tradename, manufactured by TOHO Chemical Industry Co., Ltd.) | 3 parts by weight |
| (3) | Sorpol 5073 (tradename, manufactured by TOHO Chemical Industry Co., Ltd.) | 4 parts by weight |
| (4) | The compound of the above formula (1) | 60 parts by weight |

The above compounds (1) to (4) are mixed to obtain a wettable powder.

### FORMULATION EXAMPLE 4

| | | |
|---|---|---|
| (1) | The compound of the above formula (I) | 4 parts by weight |
| (2) | Bentonite | 30 parts by weight |
| (3) | Calcium carbonate | 61.5 parts by weight |
| (4) | Toxanon GR-31A (tradename, manufactured by Sanyo Chemical Industries Co., Ltd.) | 3 parts by weight |
| (5) | Calcium lignin sulfonate | 1.5 parts by weight |

Pulverized component (1) and components (2) and (3) are preliminarily mixed, and then components (4) and (5) and water are mixed thereto. The mixture is extruded and granulated, followed by drying and sieving to obtain granules.

### FORMULATION EXAMPLE 5

| | | |
|---|---|---|
| (1) | The compound of the above formula (I) | 30 parts by weight |
| (2) | Zieclite (tradename, manufactured by Zieclite Co., Ltd.) | 60 parts by weight |
| (3) | New Kalgen WG-1 (tradename, manufactured by TAKEMOTO OIL & FAT CO., LTD.) | 5 parts by weight |
| (4) | New Kalgen FS-7 (tradename, manufactured by TAKEMOTO OIL & FAT CO., LTD.) | 5 parts by weight |

Components (1), (2) and (3) are mixed and passed through a pulverizer, and then component (4) is added thereto. The mixture is kneaded and then extruded and granulated, followed by drying and sieving to obtain water dispersible granules.

### FORMULATION EXAMPLE 6

| | | |
|---|---|---|
| (1) | The compound of the above formula (I) | 28 parts by weight |
| (2) | Soprophor FL (tradename, manufactured by Rhone-Poulenc) | 2 parts by weight |
| (3) | Sorpol 335 (tradename, manufactured by TOHO Chemical Industry Co., Ltd.) | 1 part by weight |
| (4) | IP solvent 1620 (tradename, manufactured by Idemitsu Petrochemical Co., Ltd.) | 32 parts by weight |
| (5) | Ethylene glycol | 6 parts by weight |
| (6) | Water | 31 parts by weight |

The above components (1) to (6) are mixed and pulverized by a wet-grinding machine (Dyno-mill) to obtain a water-based suspension concentrate.

### INDUSTRIAL APPLICABILITY

The herbicidal composition of the present invention has excellent herbicidal effects, and its application range extends to various fields including agricultural fields such as paddy fields, crop plant fields, orchards and mulberry fields and non-agricultural fields such as forest land, farm roads, play grounds and factory sites. Further, the application method may be suitably selected from soil application, foliar application, water application, etc. Thus, its industrial applicability is very high.

The entire disclosures of Japanese Patent Application No. 2006-351617 filed on December 27, 2006, Japanese Patent Application No. 2007-155359 filed on June 12, 2007 and Japanese Patent Application No. 2007-195420 filed on July 27, 2007 including specifications, claims, drawings and summaries are incorporated herein by reference in their entireties.

## Claims

1. A herbicide containing a benzoylpyrazole compound represented by the formula (I) or its salt, as an active ingredient: 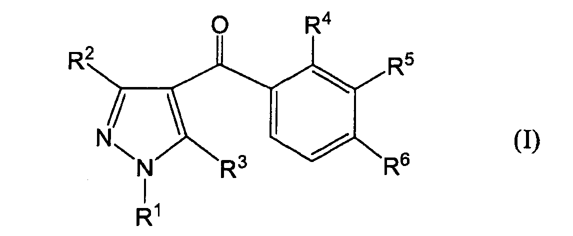 wherein R¹ is alkyl or cycloalkyl, R² is a hydrogen atom or alkyl, R³ is hydroxy, R⁴ is alkyl; haloalkyl; alkoxy; halogen; nitro; cyano; alkylthio; alkylsulfinyl; or alkylsulfonyl, R⁵ is halogen; cyano; cyanoalkyl; haloalkoxyalkyl; amino(thiocarbonyl)alkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷; thiocyanatoalkyl; haloalkoxy; alkoxyalkoxy; alkoxy substituted by at least 2 haloalkoxy; alkoxyhaloalkoxy; haloalkoxyhaloalkoxy; alkoxyalkyl substituted by at least 2 haloalkoxy; alkylthio substituted by at least 2 alkoxy; alkylthio substituted by at least 2 haloalkoxy; alkoxyhaloalkylthio; haloalkoxyhaloalkylthio; alkylthioalkylthio; haloalkylthioalkylthio; alkylthiohaloalkylthio; haloalkylthiohaloalkylthio; alkylthioalkoxy; alkylsulfonyl; alkylsulfonylalkyl; alkoxycarbonylalkyl; alkoxycarbonylalkoxy; heterocyclylalkyl; alkoxy substituted by at least 2 heterocyclyl; alkyl substituted by at least 2 heterocyclylalkoxy; -OC(O)SR⁷; or aminoalkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷, R⁶ is haloalkyl; halogen; nitro; cyano; alkylthio; alkylsulfinyl; or alkylsulfonyl, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, provided that when R⁴ is halogen, R⁵ is not cyanoalkyl; alkyl substituted by one haloalkoxy; or alkoxy substituted by one alkoxy.

2. The herbicide containing a benzoylpyrazole compound represented by the formula (I) or its salt, as an active ingredient, according to Claim 1, wherein R⁴ is alkyl, and R⁵ is cyano; cyanoalkyl; haloalkoxyalkyl; amino(thiocarbonyl)alkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷; thiocyanatoalkyl; haloalkoxy; alkoxyalkoxy; alkylsulfonyl; alkylsulfonylalkyl; alkoxycarbonylalkyl; alkoxycarbonylalkoxy; heterocyclylalkyl; or aminoalkyl which may be substituted by at least one substituent selected from the group consisting of alkyl, cyano, cyanoalkyl, (alkylthio)carbonylalkyl, alkyl(thiocarbonyl)alkyl, -C(O)OR⁷ and -C(O)SR⁷.

3. A herbicidal composition comprising the benzoylpyrazole compound or its salt as defined in Claim 1 and an agricultural adjuvant.

4. A method for controlling undesired plants or inhibiting their growth, which comprises applying a herbicidally effective amount of the benzoylpyrazole compound or its salt as defined in Claim 1, to the undesired plants or to a place where they grow.

5. The method according to Claim 4, wherein undesired plants are controlled in a cornfield.

6. The method according to Claim 5, wherein the corn is transformed one.

7. A process for producing a compound represented by the formula (1-a): 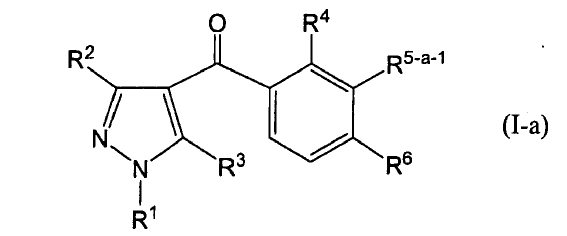 wherein R¹ is alkyl or cycloalkyl, R² is a hydrogen atom or alkyl, R³ is hydroxy, R⁴ is alkyl; haloalkyl; alkoxy; halogen; nitro; cyano; alkylthio; alkylsulfinyl; or alkylsulfonyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R⁶ is haloalkyl; halogen; nitro; cyano; alkylthio; alkylsulfinyl; or alkylsulfonyl, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, which comprises reacting a compound represented by the formula (I-b): 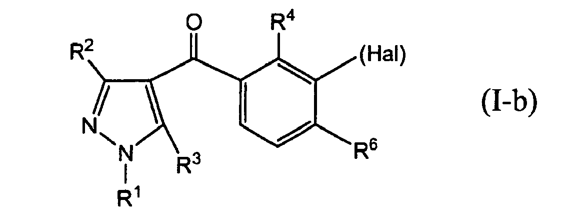 wherein R¹, R², R³, R⁴ and R⁶ are as defined above, and Hal is halogen, with a compound represented by the formula (VIII):
HO-R^{α} (VIII)
wherein R^{α} is alkyl; haloalkyl; alkoxyalkyl; haloalkoxyalkyl; alkoxyhaloalkyl; haloalkoxyhaloalkyl; heterocyclyl; heterocyclylalkyl; cycloalkyl; -C(O)SR⁷; -C(O)OR⁷; alkylthioalkyl; alkoxycarbonylalkyl; alkenyl; or alkynyl, and R⁷ is as defined above, and a base, or reacting the compound represented by the formula (I-b) with a metal salt of the compound represented by the formula (VIII).

8. A process for producing a compound represented by the formula (I-a-Hal-3): 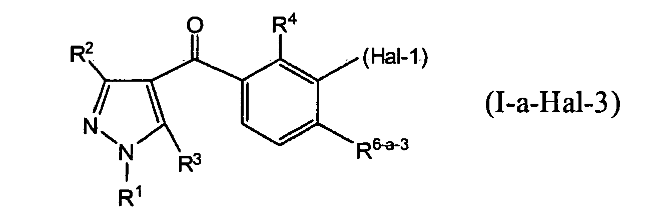 wherein R¹ is alkyl or cycloalkyl, R² is a hydrogen atom or alkyl, R³ is hydroxy, R⁴ is alkyl; haloalkyl; alkoxy; halogen; nitro; cyano; alkylthio; alkylsulfinyl; or alkylsulfonyl, R^{6-a-3} is alkylsulfonyl, and Hal-1 is halogen, which comprises reacting a compound represented by the formula (XXXIII): 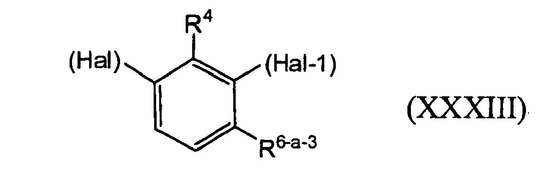 wherein R^{6-a-3} is as defined above, and each of Hal-1 and Hal, which may be the same or different from each other, is halogen, with a compound represented by the formula (III): 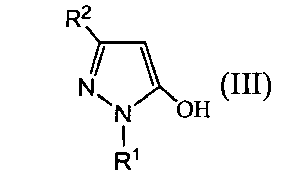 wherein R¹ and R² are as defined above, or its salt, and carbon monoxide or its equivalent, in the presence of a catalyst and a base.

9. A process for producing a compound represented by the formula (XXXIII): 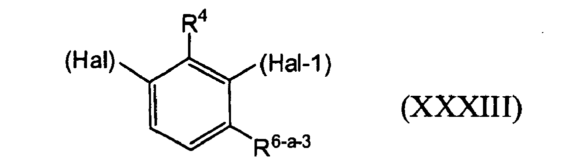 wherein R⁴ is alkyl; haloalkyl; alkoxy; halogen; nitro; cyano; alkylthio; alkylsulfinyl; or alkylsulfonyl, R^{6-a-3} is alkylsulfonyl, and each of Hal-1 and Hal which may be the same or different from each other, is halogen, which comprises reacting a compound represented by the formula (XXXIV): 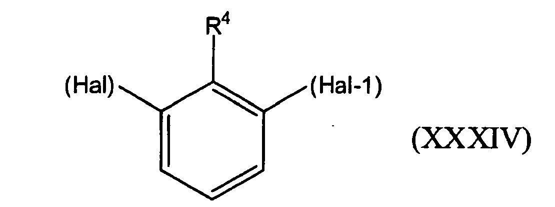 wherein R⁴, Hal-1 and Hal are as defined above, with a compound represented by the formula:
(R^{6-a-3})(Hal-3) or (R^{6-a-3})₂O
wherein R^{6-a-3} is as defined above, and Hal-3 is halogen, provided that Hal-1, Hal and Hal-3 may be the same or different from one another, in the presence of an acid.

10. A process for producing a compound represented by the formula (V-a-1-3): 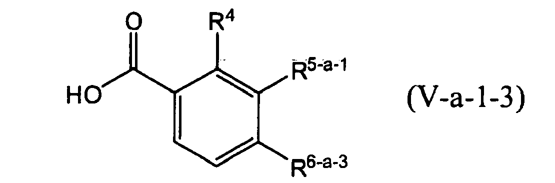 wherein R⁴ is alkyl; haloalkyl; alkoxy; halogen; nitro; cyano; alkylthio; alkylsulfinyl; or alkylsulfonyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R^{6-a-3} is alkylsulfonyl, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, which comprises reacting a compound represented by the formula (XXXII): 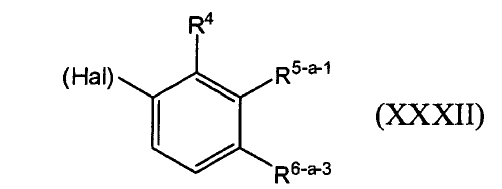 wherein R⁴, R^{5-a-1} and R^{6-a-3} are as defined above, and Hal is halogen, with carbon monoxide or its equivalent, and H₂O, in the presence of a catalyst and a base.

11. A process for producing a compound represented by the formula (XXXII): 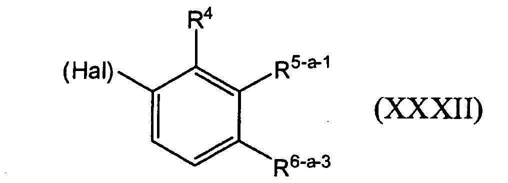 wherein R⁴ is alkyl; haloalkyl; alkoxy; halogen; nitro; cyano; alkylthio; alkylsulfinyl; or alkylsulfonyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R^{6-a-3} is alkylsulfonyl, Hal is halogen, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy, which comprises reacting a compound represented by the formula (XXXIII): 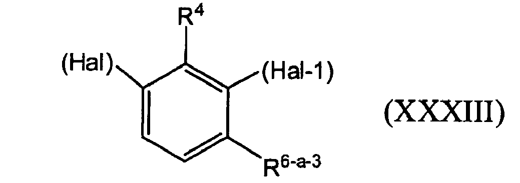 wherein R⁴ and R^{6-a-3} are as defined above, and each of Hal-1 and Hal, which may be the same or different from each other, is halogen, with a compound represented by the formula (VIII):
HO-R^{α} (VIII)
wherein R^{α} is alkyl; haloalkyl; alkoxyalkyl; haloalkoxyalkyl; alkoxyhaloalkyl; haloalkoxyhaloalkyl; heterocyclyl; heterocyclylalkyl; cycloalkyl; -C(O)SR⁷; -C(O)OR⁷; alkylthioalkyl; alkoxycarbonylalkyl; alkenyl; or alkynyl, and R⁷ is as defined above, in the presence of a base, or reacting the compound represented by the formula (XXXIII) with a metal salt of the compound represented by the formula (VIII).

12. A compound represented by the formula (XXXII-a): 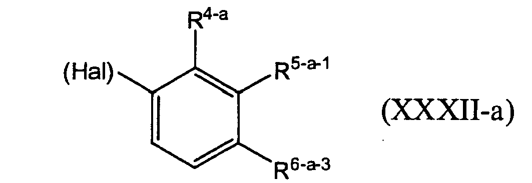 wherein R^{4-a} is alkyl, R^{5-a-1} is alkoxy; haloalkoxy; alkoxyalkoxy; haloalkoxyalkoxy; alkoxyhaloalkoxy; haloalkoxyhaloalkoxy; heterocyclyloxy; heterocyclylalkoxy; cycloalkyloxy; -OC(O)SR⁷; -OC(O)OR⁷; alkylthioalkoxy; alkoxycarbonylalkoxy; alkenyloxy; or alkynyloxy, R^{6-a-3} is alkylsulfonyl, Hal is halogen, R⁷ is alkyl; haloalkyl; alkoxyalkyl; alkenyl; haloalkenyl; alkynyl; or arylalkyl which may be substituted by R⁸, and R⁸ is halogen; alkyl; or alkoxy.
